(19) Europäisches Patentamt
European Patent Office
Office européen des brevets

(11) Publication number: **0 013 480**
**B1**

(12) **EUROPEAN PATENT SPECIFICATION**

(45) Date of publication of patent specification: **16.03.83**

(21) Application number: **79302769.9**

(22) Date of filing: **03.12.79**

(51) Int. Cl.$^3$: **A 01 N 47/36,**
C 07 D 401/12
//C07D213/71

(54) **Herbicidal pyridinesulfonamides, preparation and use thereof and compositions containing them.**

(30) Priority: **04.12.78 US 966258**
**22.10.79 US 83753**

(43) Date of publication of application:
**23.07.80 Bulletin 80/15**

(45) Publication of the grant of the patent:
**16.03.83 Bulletin 83/11**

(84) Designated Contracting States:
**BE DE FR GB IT LU NL**

(56) References cited:
**DE - A - 2 715 786**

(73) Proprietor: **E.I. DU PONT DE NEMOURS AND COMPANY**
**Legal Department 1007 Market Street**
**Wilmington Delaware 19898 (US)**

(72) Inventor: **Levitt, George**
**3218 Romilly Road**
**Cardiff Wilmington, Delaware 19810 (US)**

(74) Representative: **Hildyard, Edward Martin et al,**
**Frank B. Dehn & Co. Imperial House 15-19**
**Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 013 480 B1

Herbicidal pyridinesulfonamides, preparation and use thereof and compositions containing them

Background of the Invention

This invention relates to pyridinesulfonamides which are useful as agricultural chemicals.

French Patent No. 1,468,747 discloses the following *para*-substituted phenylsulfonamides, useful as anti-diabetic agents:

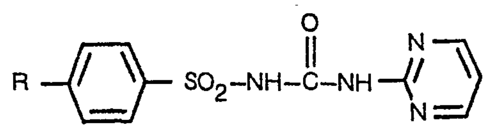

wherein R = H, halogen, $CF_3$ or alkyl.

Logemann et al. Chem. Ab., *53*, 18052 g (1959), disclose a number of sulfonamides, including uracil derivatives and those having the formula:

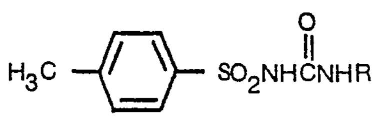 wherein R is butyl, phenyl or 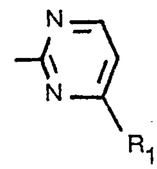

and $R_1$ is hydrogen or methyl. When tested for hypoglycemic effect in rats (oral doses of 25 mg/100 g), the compounds in which R is butyl and phenyl were most potent.

In our DE—A—2,715,786 we have disclosed a wide variety of herbicidal phenyl, furyl, thienyl and naphthyl-sulfonylureas but derivatives containing the strongly basic six-membered heterocyclic pyridylsulfonyl group were not contemplated.

Wojciechowski, J. Acta. Polon. Pharm. *19*, p. 125—5 (1962) [Chem. Ab., *59* 1633 e] describes the synthesis of N-[(2,6-dimethoxypyrimidin-4-yl)aminocarbonyl]-4-methylbenzenesulfonamide:

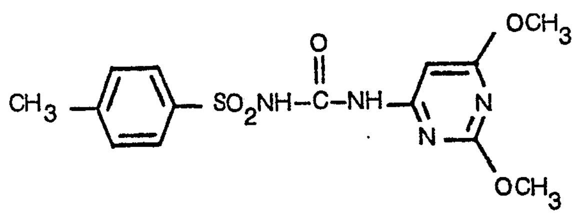

Based upon similarity to a known compound, the author predicted hypoglycemic activity for the foregoing compound.

Netherlands Patent 121,788, published Septemper 15, 1966, teaches the preparation of compounds of Formula (i), and their use as general or selective herbicides,

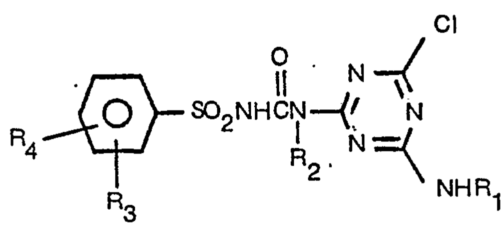

(i)

wherein

$R_1$ and $R_2$ may independently be alkyl of 1—4 carbon atoms; and

$R_3$ and $R_4$ may independently be hydrogen, chlorine or alkyl of 1—4 carbon atoms.

Compounds of Formula (ii), and their use as anti-diabetic agents, are reported in *J. Drug. Res. 6,* 123 (1974).

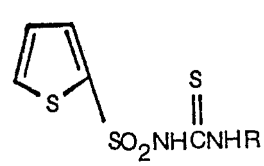

(ii)

wherein R is pyridyl.

United States Patent 3,689,549 F (Sept. 5, 1972) to R. P. Williams discloses "heterocyclic sulfonamides wherein the heteroatoms are inert can be used, e.g., compounds having the furan, thiophene or pyridine nucleus," in the production of sulfonyl isocyanates from sulfonamides in a sulfolane solvent.

B. G. Boggiano, V. Petrow, O. Stephenson and A. M. Wild, in *Journal of Pharmacy and Pharmacology 13,* 567—574 (1961) disclose the following compounds which were tested for hypoglycemic activity.

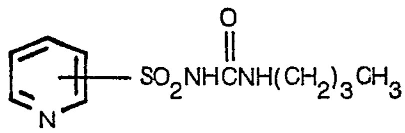 where $—SO_2NH\overset{\overset{O}{\|}}{C}NH(CH_2)_3CH_3$ is in 2 or 3 position.

J. Delarge in *Acta Pol. Pharm. 34,* 245—249 (1977) discloses N-alkylcarbamoylpyridinesulfonamides, as described in the structure below, as mild anti-inflammatory agents and strong diuretics.

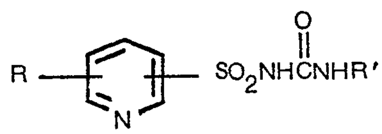

R = 3-, 4-, 5-, 6-Me, 2-, 4-, 6-Cl, 3-Br, 4-$ET_2N$, 4-$Me_2CHNH$, 4-(3-$ClC_6H_4$)NH, 4-(3-$CF_3C_6H_4$)NH
R′ = Et, Pr, $Me_2CH$, Bu

$SO_2NH\overset{\overset{O}{\|}}{C}NHR$ in 2, 3 and 4 position.

German Patent 2,516,025 (November 6, 1975) to J. E. Delarge, C. L. Lapiere and A. H. Georges discloses the following compounds as inflammation inhibitors and diuretics.

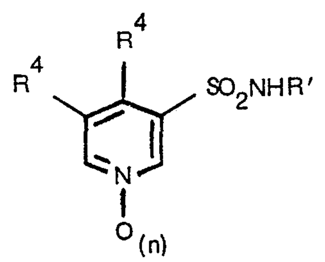

$R^4$ = XR

R = $C_6H_4R^3$($R^3$ = Cl, $CF_3$, Me, MeO, H, Br, F, $NO_2$, Et, $NH_2$), Et, iso-Pr, 4-methylfuryl, $C_6H_3Cl_2$-, $C_6H_3(CF_3)Cl$;
R′ = alkylcarbamoyl, cyclohexylcarbamoyl, arylcarbamoyl, $CSNHCH_2CH{=}CH_2$, $CONHC_6H_4Cl$-p, alkylthiocarbamoyl, H, COEt;
$R^2$ = H, Me;
X = NH, NMe, O, S, NEt; and
n = 0,1.

United States Patent 3,346,590 (October 10, 1967) (to K. Dickere and E. Kühle) discloses the following pyridinesulfonyl isothiocyanates as novel compounds.

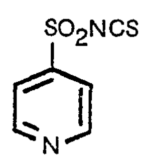 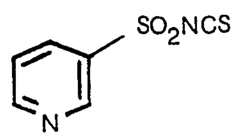

The presence of undesired vegetation causes substantial damage to useful crops, especially agricultural products that satisfy man's basic food and fiber needs, such as cotton, rice, corn, wheat, soybean and the like. The current population explosion and concomitant world food and fiber shortage demand improvements in the efficiency of producing these crops. Preventing or minimizing the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or control weeds without causing significant damage to useful crops.

Summary of the Invention

According to this invention, there are provided novel compounds of Formula I and their agriculturally suitable salts, suitable agricultural compositions containing them and methods of using them as pre-emergence and post-emergence herbicides and as plant growth regulants:

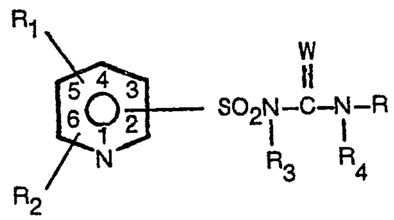

(I)

wherein

R is 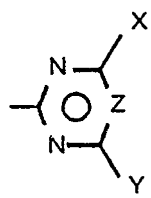 , 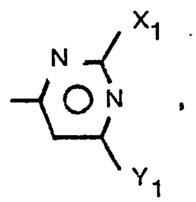 , or 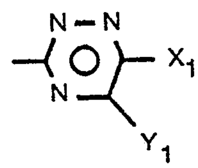

$R_1$ is H, Cl, Br, F, $C_1$—$C_4$ alkyl, $C_1$—$C_4$ alkoxy, $C_1$—$C_4$ alkylthio, $No_2$ or $\overset{O}{\overset{\|}{C}}OR_5$;
$R_2$ is H, Cl, Br or $CH_3$;
$R_3$ and $R_4$ are independently H or $CH_3$;
$R_5$ is $C_1$—$C_6$ alkyl, $C_3$—$C_6$ alkenyl, $CH_2CH_2OCH_3$, $CH_2CH_2OCH_2CH_3$, $CH_2CH_2CH_2OCH_3$ or $CH_2CH_2Cl$;
W is oxygen or sulfur;
X is $CH_3$, $CH_3O$— or $CH_3CH_2O$—;
Y is H, Cl, $CH_3$, $CF_3$, $NHCH_3$, —$N(CH_3)_2$, —$CH_2OR_{10}$, —$CH_2CH_2OR_{10}$, —$OCH_2CF_3$ or $VR_9$;
Z is CH or N;
V is oxygen or sulfur;
$R_9$ is $CH_3$, —$CH_2CH_3$, —$CH_2CH_2OR_{10}$, —$CH_2CO_2R_{10}$, $-\underset{CH_3}{\underset{|}{C}}HCO_2R_{10}$ or —$CH_2CH_2CO_2R_{10}$;
$R_{10}$ is $CH_3$ or $CH_3CH_2$;
$Y_1$ is H, $OCH_3$ or $CH_3$; and
$X_1$ is H, Cl, $OCH_3$, $OCH_2CH_3$ or $CH_3$;
and agricultural salts thereof;
providing that 1) both $X_1$ and $Y_1$ are not simultaneously H and 2) when

R is 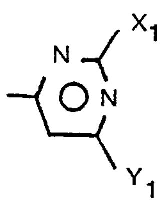 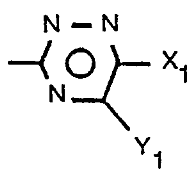 then $R_3$ and $R_4$ are both H.

Preferred in order of increasing activity and/or increasingly favorable cost are:
(1) Compounds of the generic scope wherein $R_3$ and $R_4$ are hydrogen and W is oxygen;
(2) Compounds of preferred (1) wherein $R_2$ is hydrogen;
(3) Compounds of preferred (2) wherein $R_1$ is chlorine;
(4) Compounds of preferred (3) that are 2- or 4-chloro-3-pyridyl sulfonyl compounds;
(5) Compounds of the generic scope or preferred 1—4 wherein

R is 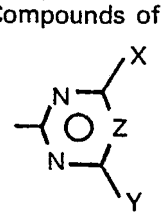 ;

(6) Compounds of preferred (5) wherein $R_1$ is chlorine and Z is CH;

(7) Compounds of preferred (5) wherein X is $CH_3$ or $—OCH_3$ and Y is $CH_3$, $—OCH_3$ or $—OCH_2CH_3$.

More preferred still for their higher activity and/or more favorable cost are those compounds of preferred (7) wherein $R_3$ and $R_4$ are hydrogen and W is oxygen.

Specifically preferred are:

2-Chloro-N-[(4-methoxy-6-methyl-1,3,5-triazin-2-yl)-aminocarbonyl]-3-pyridinesulfonamide; and

2-Chloro-N-[(4-methoxy-6-methylpyrimidin-2-yl)-aminocarbonyl]-3-pyridinesulfonamide;

and the 4,6-dimethyl and 4,6-dimethoxy analogues of these two compounds.

Synthesis

The synthesis of pyridinesulfonylisocyanates, the compounds of Formula II can be carried out by reacting a suitably substituted N-(alkylaminocarbonyl)pyridinesulfonamide with phosgene in a refluxing solvent such as xylene or chlorobenzene followed by filtration of the solution at room temperature and removal of the solvent. This reaction is shown in Equation 1.

Equation 1

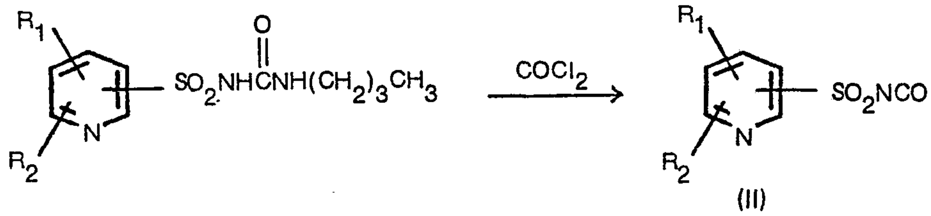


(II)

N-(alkylaminocarbonyl)pyridinesulfonamides may be conveniently prepared by the reaction of equivalent molar amounts of an appropriate pyridinesulfonamide, an alkyl isocyanate and an anhydrous base such as $K_2CO_3$ or $CaCO_3$ in an anhydrous solvent such as acetone or acetonitrile. Addition of water and adjustment of the pH to about 3 with HCl, followed by filtration yields the desired N-(alkylaminocarbonyl)pyridinesulfonamides.

Pyridinesulfonylisothiocyanates can be prepared according to the procedure taught by K.Dickere and E. Kuhle in United States Patent 3,346,590 (10/10/1967) whereby a suitable pyridinesulfonyliminodithiocarbonate is reacted with phosgene in the presence of a solvent such as toluene or xylene. The procedure is illustrated in Equation 2.

Equation 2

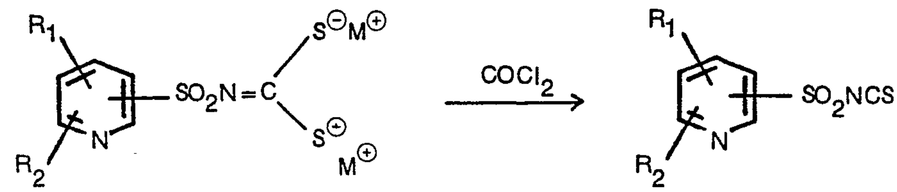


Compounds of Formula I are conveniently prepared by reacting an appropriately substituted pyridinesulfonylisocyanate or pyridinesulfonylisothiocyanate of Formula III with an appropriately substituted amino pyrimidine or amino triazine of Formula IV, according to the procedure of Equation 3.

Equation 3

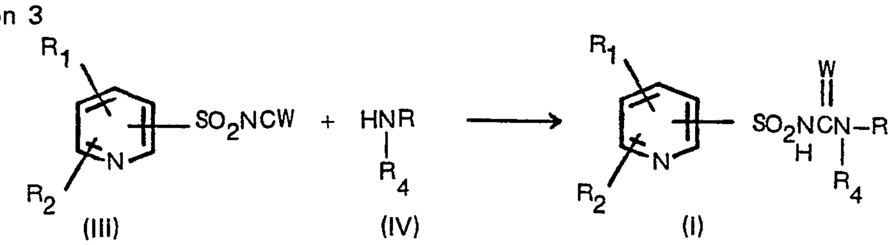

(III) (IV) (I)

The reaction of Equation 3 is best carried out in inert aprotic organic solvents such as methylene chloride, tetrahydrofuran or acetonitrile, at ambient pressure and temperature. The mode of addition is not critical; however, it is often convenient to add the sulfonyl isocyanate or isothiocyanate to a stirred suspension of amine IV. Since such isocyanates and isothiocyanates are liquids, low melting solids or are readily soluble in solvents such as those listed above, their addition can be easily controlled.

The reaction is generally exothermic. In some cases, the desired product is soluble in the warm reaction medium and on cooling crystallizes in pure form. Other products which are soluble in the

reaction mixture are isolated by evaporation of the solvent, trituration of the solid residue with solvents such as 1-chlorobutane or ethyl ether, and filtration.

As shown in Equation 4, compounds of Formula Ia, wherein $R_3$ is methyl and W is O, can be prepared by methylation of salts V wherein M is an alkali metal cation such as sodium (derived from compounds of Formula Ia, wherein $R_3$ is hydrogen).

Equation 4

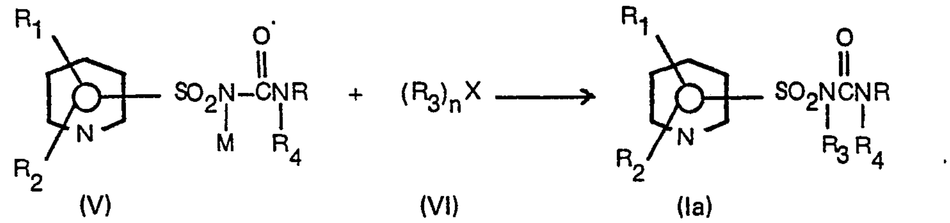

(V) (VI) (Ia)

X being an incipient anion and n being an integer corresponding to the valence of X.

The reaction of Equation 4 is best carried out in aprotic organic solvents such as tetrahydrofuran, N,N-dimethylformamide, or N,N-dimethylacetamide at ambient temperature. Methylating agents VI, such as dimethyl sulfate or methyl iodide, can be employed. The desired product can be isolated by pouring the reaction mixture into water and filtering off the precipitated solid.

As shown in Equation 5, compounds of Formula I wherein R, $R_1$, $R_2$, W and $R_4$ are as defined previously and $R_3$ is methyl, can also be prepared by the reaction of an appropriately substituted pyridyl sulfonyl-N-methylcarbamoyl chloride or sulfonyl-N-methylthiocarbamoyl chloride of Formula VII with an appropriate aminopyrimidine or aminotriazine of Formula IV.

Equation 5

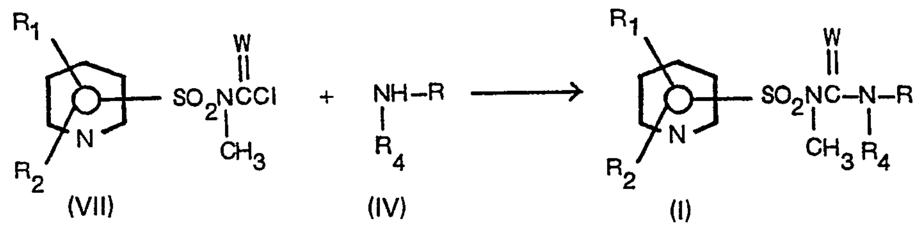

(VII) (IV) (I)

The preparation of ureas and thioureas, like those of Formula I, from amines and carbamoyl chlorides and thiocarbamoyl chlorides is well known to the art. The reaction can best be carried out by adding equivalent amounts of the chloride VII and amine IV to an inert organic solvent, such as tetrahydrofuran, xylene, or methylene chloride, in the presence of acid acceptor, such as triethylamine, pyridine, or sodium carbonate employing temperatures from 20° to 130°C. Soluble products can be isolated by filtering off precipitated salts and concentration of the filtrate. Insoluble products can be filtered off and washed free of salts with water.

The chlorides of Formula VII can be prepared by phosgenation or thiophosgenation of N-methylsulfonamide salts. The sulfonamide salt is added to an excess of phosgene or thiophosgene in an inert organic solvent, such as tetrahydrofuran, toluene, or xylene, whereupon, after removal of the excess phosgene, the chloride VII can be isolated or reacted *in situ* with the amine IV.

Compounds of Formula I, wherein R, $R_1$, $R_2$ and $R_4$ are defined previously, $R_3$ is hydrogen and W is oxygen can also be prepared by the reaction scheme shown in Equations 6, 7 and 8.

Equation 6

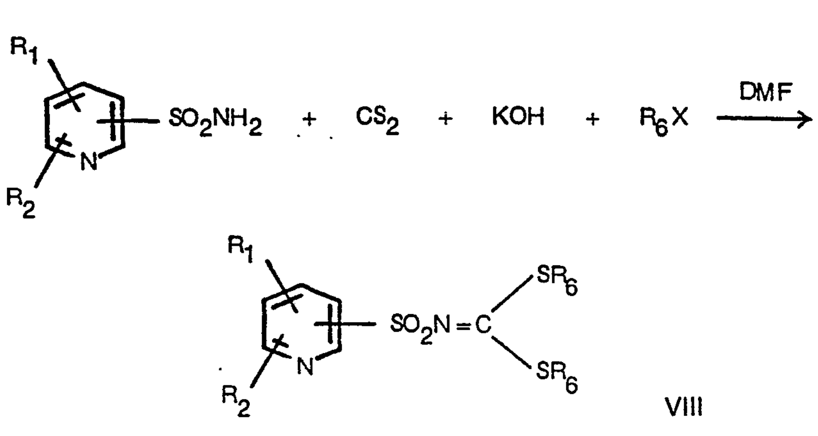


VIII

Dialkyl esters of N-(pyridinylsulfonyl)carbonimidothioic acids of Formula VIII wherein $R_6$ is $C_1$—$C_5$ alkyl, can be conveniently prepared by the reaction of an appropriate pyridinesulfonamide, carbon disulfide, an alkyl metal hydroxide and an alkyl halide in a suitable solvent, such as N,N-dimethylformamide or dimethyl sulfoxide at ambient temperature. Good yields can be obtained if alkali metal hydroxide and carbon disulfide are added in portions to the solution of pyridinesulfonamide. The reaction is generally exothermic. The desired product can be isolated by pouring the reaction mixture into cold water and filtering off the precipitated solid.

Equation 7

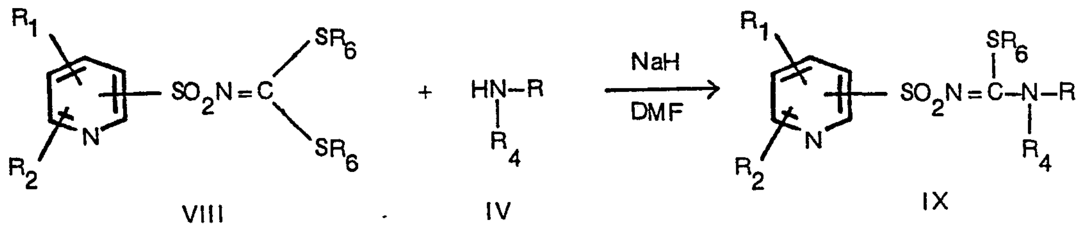


VIII IV IX

The reaction of Equation 7 is best carried out in inert aprotic solvents, such as tetrahydrofuran, N,N-dimethylformamide, or N,N-dimethylacetamide at ambient temperature. The anion of the aminopyrimidine or aminotriazine can be prepared by the reaction of an alkali metal hydride with an appropriate amine IV and a compound of Formula VIII is added to the stirring solution of the anion species at ambient temperature. Addition of water and adjustment to $pH_3$ by addition of HCl, followed by filtration yields the desired solid product.

Pyridinylsulfonylcarbonimidothioic acid esters of Formula IX, as prepared in Equation 7 can be converted to the corresponding N-(pyrimidinyl or triazinyl aminocarbonyl)pyridinesulfonamides according to Equation 8.

Equation 8

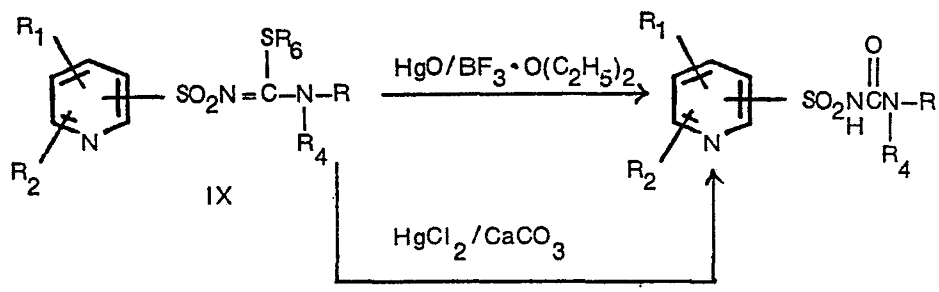


$CH_3CN/H_2O$

The reaction of Equation 8 is best carried out in aqueous tetrahydrofuran, or aqueous acetonitrile. Oxidizing agents, such as mercuric oxide/boron trifluoride etherate, or mercuric chloride/calcium carbonate may be employed at temperatures of 20° to 130°C. The desired product can be filtered off and washed free of salts with water.

As shown in Equation 9, compounds of Formula Ib, wherein R, $R_1$, $R_2$ and $R_3$ are as previously defined are alternatively prepared by the reaction of an appropriately substituted pyridylsulfonamide with the appropriate triazine or pyrimidine isothiocyanate of formula IIIa.

Equation 9

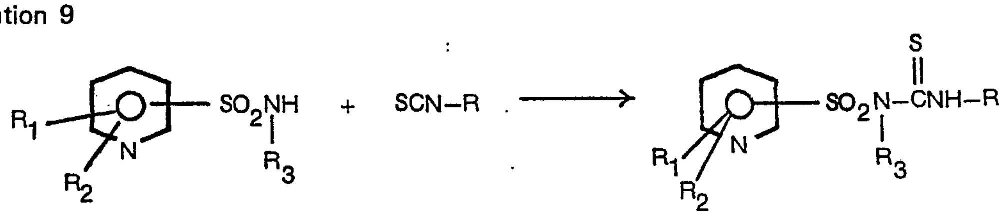

IIIa (Ib)

The reaction of Equation 9 is best carried out by dissolving or suspending the sulfonamide and isothiocyanate in a polar solvent such as acetone, acetonitrile, ethyl acetate methyl ethyl ketone, adding an equivalent of a base such as potassium carbonate and stirring the mixture at ambient temperature up to the reflux temperature for one to twenty-four hours. In some cases, the product

precipitates from the reaction mixture and can be removed by filtration. The product is stirred in dilute mineral acid, filtered and washed with cold water. If the product does not precipitate from the reaction mixture it can be isolated by evaporation of the solvent, trituration of the residue with dilute mineral acid and filtering off the insoluble product.

The heterocyclic isothiocyanates which are used in the procedure of Equation 9 are prepared, for example, according to the method of Japan Patent Application Pub: Kokai 51—143686, June 5, 1976, or that of W. Abraham and G. Barnikow, Tetrahedron *29,* 691—7 (1973).

N-(Triazinyl or pyrimidinylaminothioxomethyl)pyridinesulfonamides wherein R, $R_1$, $R_2$, $R_3$, and $R_4$ are as defined previously can be converted to the corresponding aminocarbonylpyridinesulfonamides as shown in Equation 9A.

Equation 9A

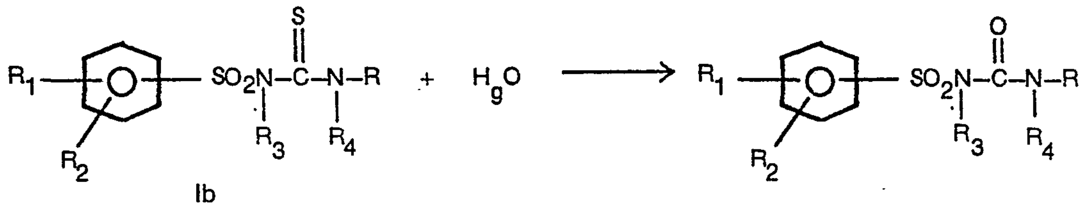

Ib

The reaction of Equation 9A is best carried out by suspending the appropriate compound of Formula Ib and mercuric oxide in a solvent such as acetone, acetonitrole or methyl ethyl ketone and stirring the mixture at ambient temperature for 10 to 72 hours. Filtration to remove the insoluble mercury compound and evaporation of the solvent yields a residue containing the desired product.

The procedure for the preparation of agriculturally suitable salts of the compounds of Formula I is described for alkylimetal salts, ammonium salts and acid addition salts in Equations 10, 11 and 12 respectively. Preparation of other salts would be obvious to one skilled in the art.

Equation 10

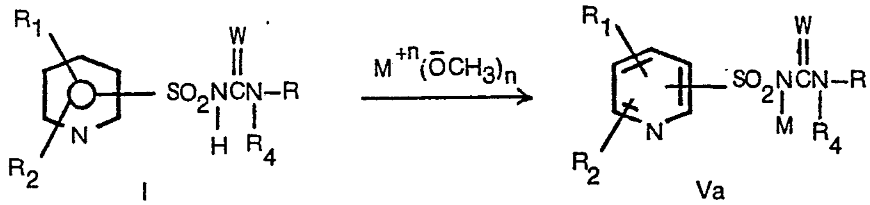


I Va

The reaction of Equation 10 is best carried out by adding to a suspension of one equivalent of the appropriate pyridinesulfonamide in a solvent such as methanol or ethanol one equivalent of an appropriate base such as a metal alkoxide, hydroxide, or carbonate in the same solvent. Following heating at 40—50°C the solvent is removed *in vacuo* to yield the desired salt which can be triturated with solvents such as ethyl acetate, ethyl ether or hexane to give the desired solid alkali metal salts.

Equation 11

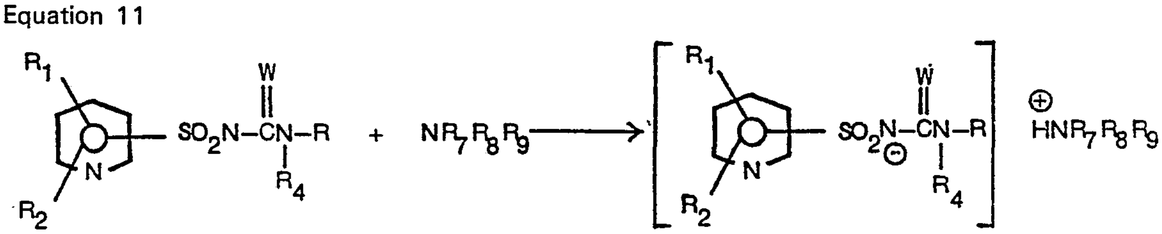

(I) (X) Vb

wherein

$R_7$ is $C_1$—$C_{12}$ alkyl, $CH_2CH_2OH$, $C_6H_5$ or H
$R_8$ is $C_1$—$C_{12}$ alkyl, $CH_2CH_2OH$, $C_6H_5$ or H
$R_9$ is $C_1$—$C_{12}$ alkyl, $CH_2CH_2OH$, $C_6H_5$ or H

or $R_7R_8R_9N$ = 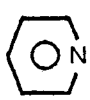

with the proviso that $R_7$, $R_8$ and $R_9$ cannot all be phenyl at the same time.

The ammonium salts of substituted pyridinesulfonamides can be prepared as described in Equation 11.

Generally, one equivalent of an appropriate amine such as ammonia, dimethylamine, diethanolamine or ethanolamine is added neat or in a solvent such as methylene chloride or methanol to a suspension of the appropriate benzenesulfonamide in the same solvent. The solvent is removed under reduced pressure to yield as product the desired salt.

Equation 12

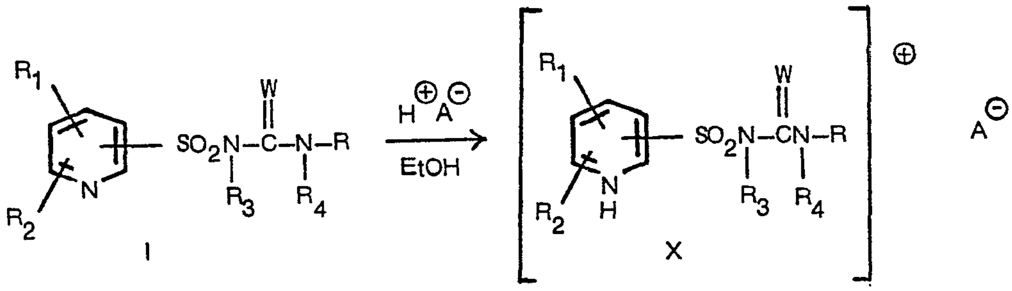


I

X

As shown in Equation 12, the acid addition salts of N-(heterocyclicaminocarbonyl)pyridinesulfonamides of Formula X can be prepared by heating at about 40—50°C a suspension of the aminocarbonylpyridinesulfonamide in a solvent such as ethanol or hexane containing an equivalent amount of an acid such as HCl, HBr, $H_2SO_4$, p-toluenesulfonic acid or trichloroacetic acid. In the case of volatile mineral acids such as HCl or organic acids such as trichloro acetic acid an excess may be employed. Removal of solvent *in vacuo* yields the desired salt.

The synthesis of sulfur compounds of pyridine has been reviewed in "The Chemistry of Heterocyclic Compounds", a series published by Interscience Publ., New York and London. Pyridinesulfonamides are described by H. L. Tale in "Pyridine and Its Derivatives" Supplement, Part 4 (1975).

The compounds of this invention and their preparation are further illustrated by the following examples wherein temperatures are given in degrees centrigrade.

Example 1

2-Chloro-3-pyridinesulfonylisocyanate

To 125 ml of dry xylene was added with stirring 20.7 g of 2-chloro-N-(butylcarbamoyl)-3-pyridinesulfonamide. This solution was heated to reflux, and phosgene added until no further uptake of this gas was observed. It was then cooled, filtered and the solvent was removed *in vacuo* to yield 2-chloro-3-pyridinesulfonylisocyanate as an oil Bp 108—110°C (0.7 mm Hg). This product showed a sharp absorption peak in the infrared region at 2220 $cm^{-1}$.

By using the procedure of Example 1 with the appropriate N-(alkylcarbamoyl)pyridinesulfonamide, the sulfonyl isocyanate of Table 1 can be prepared. The pyridinesulfonyliso-thiocyanates of Table 1 are prepared according to the method of K. Dickere and E. Kühle as described in Equation 2.

TABLE I

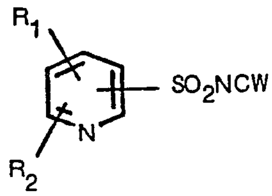

| $R_1$ | $R_2$ | W | Position $—SO_2NCW$ |
|---|---|---|---|
| 6-Cl | H | O | 3 |
| $2\text{-}CH_3$ | $6\text{-}CH_3$ | S | 3 |
| $5\text{-}CH_3$ | H | O | 3 |
| $6\text{-}CH_3$ | H | O | 3 |
| $5\text{-}C_2H_5$ | $2\text{-}CH_3$ | O | 3 |
| $5\text{-}n\text{-}C_4H_9$ | $2\text{-}CH_3$ | O | 3 |
| 5-Br | 6-Cl | O | 3 |
| $4\text{-}CO_2C_2H_5$ | H | O | 3 |
| $2\text{-}CO_2CH_3$ | H | O | 3 |
| $4\text{-}CO_s iC_3H_7$ | H | O | 3 |
| $4\text{-}OCH_3$ | H | O | 3 |
| $6\text{-}n\text{-}C_4H_9$ | H | O | 3 |
| $5\text{-}CH_3$ | 6-Cl | O | 3 |
| $5\text{-}NO_2$ | 6-Cl | O | 3 |
| 4-Cl | H | O | 3 |
| 2-F | H | O | 3 |
| 2-Br | H | O | 3 |
| $6\text{-}SC_2H_5$ | H | O | 3 |
| $4\text{-}SC_4H_9$ | H | O | 3 |
| $2\text{-}SC_2H_5$ | H | O | 3 |
| 4-F | H | O | 3 |
| 4-Br | H | O | 3 |
| $6\text{-}SCH_3$ | H | O | 3 |
| $5\text{-}NO_2$ | 6-Cl | S | 3 |
| 5-Br | H | S | 3 |
| $5\text{-}NO_2$ | H | S | 3 |

Table I (continued)

| $R_1$ | $R_2$ | W | Position $-SO_2NCW$ |
|---|---|---|---|
| 4-$n$-$C_4H_9$ | H | S | 3 |
| 6-$n$-$C_4H_9$ | H | O | 3 |
| 5-F | 6-Cl | O | 3 |
| 2-$NO_2$ | 6-Br | O | 3 |
| 2-$C_2H_5$ | H | O | 4 |
| 2-$CH_3$ | 6-$CH_3$ | O | 4 |
| 3-Cl | 5-Cl | O | 4 |
| 3-Br | 5-Br | S | 4 |
| H | 3-Cl | O | 4 |
| 3-Br | H | O | 4 |
| 3-$CH_3$ | H | O | 4 |
| 2-$CO_2$-$n$-$C_6H_{13}$ | H | O | 4 |
| 5-$NO_2$ | 2-Cl | O | 4 |
| H | H | S | 3 |
| H | H | O | 3 |
| 2-$NO_2$ | H | O | 3 |
| 2-$CO_2$-$i$-$C_3H_7$ | H | O | 3 |
| 2-$CO_2CH_3$ | H | O | 3 |
| 2-Cl | H | S | 3 |
| 2-$NO_2$ | H | S | 3 |
| 2-$CO_2$-$i$-$C_3H_7$ | H | S | 3 |
| 2-$CO_2CH_2$ | H | S | 3 |
| 2-$CH_3$ | H | O | 3 |
| 2-$CH_3$ | H | S | 3 |
| 4-Cl | H | S | 3 |
| 4-$NO_2$ | H | O | 3 |
| 4-$NO_2$ | H | S | 3 |
| 4-$CH_3$ | H | O | 3 |
| 4-$CH_3$ | H | S | 3 |

Table I (Continued)

| $R_1$ | $R_2$ | W | Position —$SO_2NCW$ |
|---|---|---|---|
| 4-$CO_2CH_3$ | H | O | 3 |
| 4-$CO_2CH_3$ | H | S | 3 |
| 4-$CO_2$-*i*-$C_3H_7$ | H | S | 3 |
| H | H | O | 4 |
| H | H | S | 4 |
| 3-Cl | H | S | 4 |
| 3-Cl | H | O | 4 |
| 3-$NO_2$ | H | O | 4 |
| 3-$NO_2$ | H | S | 4 |
| 3-$CH_3$ | H | S | 4 |
| 3-$CO_2CH_3$ | H | O | 4 |
| 3-$CO_2CH_3$ | H | S | 4 |
| 3-$CO_2$-*i*-$C_3H_7$ | H | S | 4 |
| 3-$CO_2$-*i*-$C_3H_7$ | H | O | 4 |

Example 2

2-Chloro-N-[(4-methoxy-6-methylpyrimidin-2-yl)aminocarbonyl]-3-pyridinesulfonamide

To a stirred suspension of 1.4 g of 2-amino-4-methoxy-6-methylpyrimidine in 20 ml of dry acetonitrile at room temperature was added 2.2 g of 2-chloropyridine-3-sulfonylisocyanate. The mixture was stirred for several hours and filtered. Evaporation of the filtrate yielded a solid residue which was stirred in approximately 30 ml of water while adjusting the pH of this mixture to 11 by the addition of 10% NaOH. This solution was filtered, the filtrate pH adjusted to pH 7 by adding 10% hydrochloric acid and the resulting neutral solution was again filtered. The desired product was then precipitated from this filtrate by adjusting the pH to 2, collected in a sintered funnel and dried in a vacuum oven at room temperature. It melted at 165—168°.

By using the procedure of Example 2 with an equivalent amount of a 2-aminopyrimidine and appropriately substituted sulfonylisocyanate or isothiocyanate, the compounds of Table II can be prepared.

TABLE II

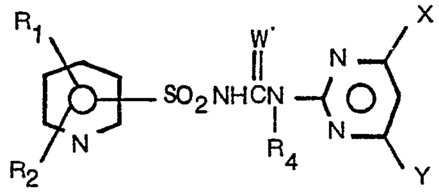

| $R_1$ | $R_2$ | $R_4$ | Position $-SO_2N<$ | W | X | Y | m.p. |
|---|---|---|---|---|---|---|---|
| 2-Cl | H | H | 3 | S | $CH_3$ | $SCH_3$ | |
| 2-Cl | H | $CH_3$ | 3 | O | $CH_3$ | $OCH_3$ | |
| 6-Cl | H | H | 3 | O | $CH_3$ | $OCH_3$ | 194—197° |
| 2-$CH_3$ | 6-$CH_3$ | H | 3 | O | $CH_3$ | $OCH_3$ | |
| 4-$CH_3$ | H | $CH_3$ | 3 | O | $CH_3$ | $OCH_2CH_2OCH_3$ | |
| 2-$CO_2CH_2CH{=}CH_2$ | H | H | 3 | O | $CH_3$ | $OCH_3$ | |
| 2-$CO_2CH—CH{=}CH—(CH_2)_2CH_3$ | H | H | 3 | O | $CH_3$ | $OCH_3$ | |
| 4-$CO_2CH_2CH_2OCH_3$ | H | H | 3 | O | $CH_3$ | $CH_3$ | |
| 4-$CO_2CH_2CH_2OCH_2CH_3$ | H | H | 3 | O | $CH_3$ | $OCH_3$ | |
| 6-Cl | H | H | 3 | O | $CH_3$ | $CH_3$ | 165—170° |
| 6-Cl | H | H | 3 | O | $—OCH_3$ | $—OCH_3$ | 211—214° |
| 2-$CO_2CH_2CH_2CH_2OCH_3$ | H | H | 3 | O | $CH_3$ | $OCH_3$ | |
| 2-$CO_2CH_2CH_2Cl$ | H | H | 3 | O | $OCH_3$ | $OCH_3$ | |
| 6-$CH_3$ | H | H | 3 | O | $CH_3$ | $OCH_2CO_2CH_3$ | |
| 5-$C_2H_5$ | 2-$CH_3$ | H | 3 | O | $CH_3$ | $OCH_2CO_2C_2H_5$ | |
| 5-*n*-$C_4H_9$ | 2-$CH_3$ | H | 3 | S | $CH_3$ | $OCH(CH_3)CO_2C_2H_5$ | |
| 5-Br | 6-Cl | H | 3 | O | $CH_3$ | $SCH_2CO_2CH_3$ | |
| 2-$CO_2CH_3$ | H | H | 3 | O | $CH_3$ | $S(CH_2)_2CO_2C_2H_5$ | |
| 4-$CO_2C_2H_5$ | H | H | 3 | O | $OCH_3$ | $OCH_3$ | |
| 5-$CO_2iC_3H_7$ | H | $CH_3$ | 3 | S | $CH_3$ | $OCH_3$ | |
| 6-$CO_2CH_3$ | H | H | 3 | O | $CH_3$ | $OCH_3$ | |
| 4-$OCH_3$ | H | H | 3 | O | $CH_3$ | $OCH_3$ | |
| 6-$OCH_3$ | H | H | 3 | O | $OC_2H_5$ | $OCH_3$ | |
| 5-$CH_3$ | 6-Cl | H | 3 | O | $CH_3$ | $CF_3$ | |
| 5-$NO_2$ | 6-Cl | H | 3 | O | $CH_3$ | $OCH_2CF_3$ | |
| 4-Cl | H | H | 3 | O | $CH_3$ | $N(CH_3)_2$ | |

TABLE II (continued)

| $R_1$ | $R_2$ | $R_4$ | Position $-SO_2N<$ | W | X | Y | m.p. |
|---|---|---|---|---|---|---|---|
| 6-F | H | H | 3 | S | $CH_3$ | Cl | |
| 6-Br | H | H | 3 | O | $CH_3$ | Cl | |
| 6-$OC_2H_5$ | H | H | 3 | O | $CH_3$ | $SC_2H_5$ | |
| 6-$SC_2H_5$ | H | $CH_3$ | 3 | S | $CH_3$ | $S(CH_2)_2OC_2H_5$ | |
| 4-S-*n*-$C_4H_9$ | H | H | 3 | O | $OCH_3$ | $OCH_3$ | |
| 2-$SC_2H_5$ | H | H | 3 | O | $CH_3$ | $OCH_3$ | |
| 4-F | H | H | 3 | O | $CH_3$ | $OCH_2CH_2CO_2C_2H_5$ | |
| 4-Br | H | $CH_3$ | 3 | S | $CH_3$ | $OCH_3$ | |
| 6-$SCH_3$ | H | H | 3 | O | $CH_3$ | $OCH_3$ | |
| 5-$NO_2$ | 6-Cl | $CH_3$ | 3 | O | $OCH_3$ | $OCH_3$ | |
| 5-Br | H | H | 3 | O | $OCH_3$ | $OCH_3$ | |
| 5-$NO_2$ | H | H | 3 | O | $CH_3$ | $CF_3$ | |
| 4-*n*-$C_4H_9$ | H | H | 3 | S | $CH_3$ | $SCH_2CO_2CH_3$ | |
| 6-*n*-$C_4H_9$ | H | H | 3 | O | $OCH_3$ | $OCH_3$ | |
| 5-F | 6-Cl | H | 3 | O | $OC_2H_5$ | $CH_2OCH_3$ | |
| 2-Cl | 6-Cl | H | 3 | O | $OCH_3$ | $OCH_3$ | 182—185° |
| 2-Cl | 6-Cl | H | 3 | O | $CH_3$ | $OCH_3$ | 180—183° |
| 2-$C_2H_5$ | H | $CH_3$ | 4 | O | $CH_3$ | $OCH_3$ | |
| 2-$C_2H_5$ | H | H | 4 | O | $CH_3$ | $OC_2H_5$ | |
| 2-$CH_3$ | 6-$CH_3$ | H | 4 | O | $CH_3$ | $CH_2CH_2OCH_3$ | |
| 3-Cl | 5-Cl | H | 4 | O | $CH_3$ | $OCH_2CH_2CO_2CH_3$ | |
| 3-Cl | 5-Cl | $CH_3$ | 4 | O | $OCH_3$ | $OCH_3$ | |
| 3-Br | 5-Br | H | 4 | S | $CH_3$ | $OCH_2CF_2$ | |
| 3-Cl | H | H | 4 | O | $CH_3$ | Cl | |
| 3-Br | H | H | 4 | O | $OCH_3$ | $OCH_3$ | |
| 3-$CH_3$ | H | H | 4 | O | $CH_3$ | $SC_2H_5$ | |
| 2-$CO_2$-*n*-$C_4H_9$ | H | H | 4 | O | $CH_3$ | $OCH(CH_3)CO_2CH_3$ | |
| 5-$NO_2$ | 2-Cl | H | 4 | O | $CH_3$ | $OCH_3$ | |
| 5-Cl | H | H | 4 | O | $CH_3$ | $OCH_3$ | |

TABLE II (continued)

| $R_1$ | $R_2$ | $R_4$ | Position $-SO_2N<$ | W | X | Y | m.p. |
|---|---|---|---|---|---|---|---|
| H | H | H | 3 | O | $CH_3$ | $CH_3$ | 152° dec. |
| H | H | H | 3 | S | $CH_3$ | $OCH_3$ | |
| H | H | H | 3 | O | $OCH_3$ | $OCH_3$ | 176° dec. |
| 2-Cl | H | H | 3 | O | $OCH_3$ | $OCH_3$ | 160—165° |
| 2-Cl | H | H | 3 | O | $CH_3$ | $-CH_2OCH_3$ | |
| 2-Cl | H | H | 3 | O | $CH_3$ | $CH_3$ | 140—145° |
| 2-$NO_2$ | H | H | 3 | O | $CH_3$ | $CH_3$ | |
| 2-$NO_2$ | H | H | 3 | O | $CH_3$ | $OCH_3$ | |
| 2-$NO_2$ | H | H | 3 | O | $OCH_3$ | $OCH_3$ | |
| 2-$CO_2CH_3$ | H | H | 3 | O | $OCH_3$ | OCH | |
| 2-$CO_2CH_3$ | H | H | 3 | O | $CH_3$ | $OCH_3$ | |
| 2-$CO_2CH_3$ | H | H | 3 | O | $CH_3$ | $CH_3$ | |
| 2-$CO_2$-*i*-$C_3H_7$ | H | H | 3 | O | $CH_3$ | $CH_3$ | |
| -$CO_2$-*i*-$C_3H_7$ | H | H | 3 | O | $CH_3$ | $OCH_3$ | |
| 2-$CO_2$-*i*-$C_3H_7$ | H | H | 3 | O | $OCH_3$ | $OCH_3$ | |
| 2-$CH_3$ | H | H | 3 | O | $OCH_3$ | $OCH_3$ | |
| 2-$CH_3$ | H | H | 3 | O | $CH_3$ | $OCH_3$ | |
| 2-$CH_3$ | H | H | 3 | O | $CH_3$ | $CH_3$ | |
| 4-Cl | H | H | 3 | O | $CH_3$ | $CH_3$ | |
| 4-Cl | H | H | 3 | S | $CH_3$ | $OCH_3$ | |
| 4-Cl | H | H | 3 | O | $OCH_3$ | $OCH_3$ | |
| 4-$NO_2$ | H | H | 3 | O | $OCH_3$ | $OCH_3$ | |
| 4-$NO_2$ | H | H | 3 | O | $CH_3$ | $OCH_3$ | |
| 4-$NO_2$ | H | H | 3 | O | $CH_3$ | $CH_3$ | |
| 4-$CO_2CH_3$ | H | H | 3 | O | $CH_3$ | $CH_3$ | |
| 4-$CO_2CH_3$ | H | H | 3 | O | $CH_3$ | $OCH_3$ | |
| 4-$CO_2CH_3$ | H | H | 3 | O | $OCH_3$ | $OCH_3$ | |
| 4-$CO_2$-*i*-$C_3H_7$ | H | H | 3 | O | $CH_3$ | $CH_3$ | |
| 4-$CO_2$-*i*-$C_3H_7$ | H | H | 3 | O | $OCH_3$ | $OCH_3$ | |
| 4-$CO_2$-*i*-$C_3H_7$ | H | H | 3 | O | $CH_3$ | $OCH_3$ | |

TABLE II (continued)

| $R_1$ | $R_2$ | $R_4$ | Position $-SO_2N<$ | W | X | Y |
|---|---|---|---|---|---|---|
| 4-$CH_3$ | H | H | 3 | S | $CH_3$ | $OCH_3$ |
| 4-$CH_3$ | H | H | 3 | O | $CH_3$ | $CH_3$ |
| 4-$CH_3$ | H | H | 3 | O | $OCH_3$ | $OCH_3$ |
| H | H | H | 4 | O | $OCH_3$ | $OCH_3$ |
| H | H | H | 4 | O | $CH_3$ | $OCH_3$ |
| H | H | H | 4 | O | $CH_3$ | $CH_3$ |
| 3-Cl | H | H | 4 | S | $OCH_3$ | $OCH_3$ |
| 3-Cl | H | H | 4 | O | $CH_3$ | $CH_3$ |
| 3-Cl | H | H | 4 | S | $CH_3$ | $OCH_3$ |
| 3-$NO_2$ | H | H | 4 | S | $CH_3$ | $CH_3$ |
| 3-$NO_2$ | H | H | 4 | O | $OCH_3$ | $OCH_3$ |
| 3-$NO_2$ | H | H | 4 | O | $CH_3$ | $OCH_3$ |
| 3-$CO_2CH_3$ | H | H | 4 | O | $OCH_3$ | $OCH_3$ |
| 3-$CO_2CH_3$ | H | H | 4 | S | $CH_3$ | $OCH_3$ |
| 3-$CO_2CH_3$ | H | H | 4 | O | $CH_3$ | $CH_3$ |
| 3-$CO_2$-*i*-$C_3H_7$ | H | H | 4 | O | $CH_3$ | $CH_3$ |
| 3-$CO_2$-*i*-$C_3H_7$ | H | H | 4 | O | $CH_3$ | $OCH_3$ |
| 3-$CO_2$-*i*-$C_3H_7$ | H | H | 4 | O | $OCH_3$ | $OCH_3$ |
| 3-$CH_3$ | H | H | 4 | O | $CH_3$ | $CH_3$ |
| 3-$CH_3$ | H | H | 4 | O | $CH_3$ | $OCH_3$ |
| 3-$CH_3$ | H | H | 4 | O | $OCH_3$ | $OCH_3$ |

## Example 3

2-Chloro-N-[(4-methoxy-6-methyl-1,3,5-triazin-2-yl)aminocarbonyl]-3-pyridinesulfonamide

To a stirred suspension of 1.4 g of 2-amino-4-methoxy-6-methyltriazine in 20 ml of dry acetonitrile at room temperature was added 2.2 g of 2-chloropyridine-3-sulfonylisocyanate. The mixture was stirred for several hours and then evaporated to dryness. The residue was mixed with 30 ml of water and enough 10% sodium hydroxide to adjust the pH to 11. This mixture was filtered and the filtrate adjusted to pH7 by the addition of 10% hydrochloric acid and the refiltered. Acidification of this filtrate to pH2 caused the desired compound to precipitate. The desired compound was filtered off and after drying in a vacuum oven was found to melt at 160—165°.

## Example 4

N-[(4,6-Dimethoxy-1,3,5-triazin-2-yl)aminothioxomethyl]-3-pyridinesulfonamide

A mixture of 1.58 g of 3-pyridinesulfonamide, 2.04 g of 4,6-dimethoxy-2-isothiocyanato-1,3,5-triazine and 1.5 g of anhydrous potassium carbonate was stirred in 75 ml of acetone for 60 hours at ambient temperature. The reaction mixture was then poured into 500 ml of cold water, acidified with

hydrochloric acid and the precipitated product was removed by filtration to yield 2.7 g of the desired compound, m.p. 158—160°.

### Example 5

N-[(4,6-Dimethoxy-1,3,5-triazin-2-yl)aminocarbonyl]-3-pyridinesulfonamide

A mixture containing 2.7 g of N-[(4,6-dimethoxy-1,3,5-triazin-2-yl)aminothioxomethyl]-3-pyridinesulfonamide, 2.98 g of mercuric oxide and 75 ml of acetone was stirred at ambient temperature for 60 hours and then filtered. Evaporation of the filtrate *in vacuo* yielded the desired product as a residual white solid of m.p. 182—185° dec.

By using the procedure of Example 3 with an equivalent amount of a 2-amino-1,3,5-triazine and appropriately substituted sulfonylisocyanate or isothiocyanate, the compounds of Table III can be prepared. Alternatively, N-(aminothoxomethyl)pyridinesulfonamides of Table III can be prepared according to the method of Example 4 by substituting equivalent amounts of an appropriately substituted pyridinesulfonamide and isothiocyanato-1,3,5-triazine. The aminothioxomethylsulfonamides obtained by the method of Example 4 or by Equations 3 or 5 wherein W = S can also be converted to the N-(aminocarbonyl)pyridinesulfonamides of Table III by reaction with mercuric oxide according to the procedure of Example 5.

TABLE III

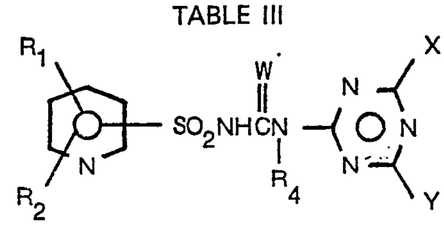

| $R_1$ | $R_2$ | $R_4$ | Position $-SO_2N<$ | W | X | Y | m.p. |
|---|---|---|---|---|---|---|---|
| 2-Cl | H | H | 3 | S | $CH_3$ | $SCH_3$ | |
| 2-Cl | H | $CH_3$ | 3 | O | $CH_3$ | $OCH_3$ | |
| 6-Cl | H | H | 3 | O | $CH_3$ | $OCH_3$ | 155—160° |
| 2-$CH_3$ | 6-$CH_3$ | H | 3 | O | $CH_3$ | $OCH_3$ | |
| 4-$CH_3$ | H | $CH_3$ | 3 | O | $CH_3$ | $OCH_2CH_2OCH_3$ | |
| 6-Cl | H | H | 3 | O | $OCH_3$ | $OCH_3$ | 151—155° |
| 2-Cl | H | $CH_3$ | 3 | O | $OCH_3$ | $OCH_3$ | 115—122° |
| 2-$CO_2CH_2CH_2Cl$ | H | H | 3 | O | $CH_3$ | $OCH_3$ | |
| 2-$CO_2CH_2$—$CH{=}CH_2$ | H | H | 3 | O | $CH_3$ | $OCH_3$ | |
| 2-$CO_2CH_2CH_2OCH_3$ | H | H | 3 | O | $OCH_3$ | $OCH_3$ | |
| 6-$CH_3$ | H | H | 3 | O | $CH_3$ | $OCH_2CO_2CH_3$ | |
| 5-$C_2H_5$ | 2-$CH_3$ | H | 3 | O | $CH_3$ | $OCH_2CO_2C_2H_5$ | |
| 5-n-$C_4H_9$ | 2-$CH_3$ | H | 3 | S | $CH_3$ | $OCH(CH_3)CO_2C_2H_5$ | |
| 5-Br | 6-Cl | H | 3 | O | $CH_3$ | $SCH_2CO_2CH_3$ | |
| 2-$CO_2CH_3$ | H | H | 3 | O | $CH_3$ | $S(CH_2(_2CO_2C_2H_5$ | |
| 4-$CO_2C_2H_5$ | H | H | 3 | O | $OCH_3$ | $OCH_3$ | |
| 5-$CO_2$-*i*-$C_3H_7$ | H | $CH_3$ | 3 | S | $CH_3$ | $OCH_3$ | |

TABLE III (continued)

| $R_1$ | $R_2$ | $R_4$ | Position $-SO_2N<$ | W | X | Y | m.p. |
|---|---|---|---|---|---|---|---|
| 6-$CO_2CH_3$ | H | H | 3 | O | $CH_3$ | $OCH_3$ | |
| 4-$OCH_3$ | H | H | 3 | O | $CH_3$ | $OCH_3$ | |
| 6-$OCH_3$ | H | H | 3 | O | $OC_2H_5$ | $OCH_3$ | |
| 5-$CH_3$ | 6-Cl | H | 3 | O | $CH_3$ | $CF_3$ | |
| 5-$NO_2$ | 6-Cl | H | 3 | O | $CH_3$ | $OCH_2CF_3$ | |
| 4-Cl | H | H | 3 | O | $CH_3$ | $N(CH_3)_2$ | |
| 6-F | H | H | 3 | S | $CH_3$ | Cl | |
| 6-Br | H | H | 3 | O | $CH_3$ | Cl | |
| 6-$OC_2H_5$ | H | $CH_3$ | 3 | O | $CH_3$ | $SC_2H_5$ | |
| 6-$SC_2H_5$ | H | $CH_3$ | 3 | S | $CH_3$ | $S(CH_2)_2OC_2H_5$ | |
| 4-S-n$C_4H_9$ | H | H | 3 | O | $OCH_3$ | $OCH_3$ | |
| 2-$SC_2H_5$ | H | H | 3 | O | $CH_3$ | $OCH_3$ | |
| 4-F | H | H | 3 | O | $CH_3$ | $OCH_2CH_2CO_2C_2H_5$ | |
| 4-Br | H | $CH_3$ | 3 | S | $CH_3$ | $OCH_3$ | |
| 6-$SCH_3$ | H | H | 3 | O | $CH_3$ | $OCH_3$ | |
| 5-$NO_2$ | 6-Cl | $CH_3$ | 3 | O | $OCH_3$ | $OCH_3$ | |
| 5-Br | H | H | 3 | O | $OCH_3$ | $OCH_3$ | |
| 5-$NO_2$ | H | H | 3 | O | $CH_3$ | $CF_3$ | |
| 4-*n*-$C_4H_9$ | H | H | 3 | S | $CH_3$ | $SCH_2CO_2CH_3$ | |
| 6-*n*-$C_4H_9$ | H | H | 3 | O | $OCH_3$ | $OCH_3$ | |
| 5-F | 6-Cl | H | 3 | O | $OC_2H_5$ | $CH_2OCH_3$ | |
| 2-Cl | 6-Cl | H | 3 | S | $OCH_3$ | $OCH_3$ | 143—145° |
| 2-Cl | 6-Cl | H | 3 | O | $CH_3$ | $OCH_3$ | 93— 96° |
| 2-Cl | 6-Cl | H | 3 | O | $OCH_3$ | $OCH_3$ | 172—177° |
| 2-$C_2H_5$ | H | $CH_3$ | 4 | O | $CH_3$ | $OCH_3$ | |
| 2-$C_2H_5$ | H | H | 4 | O | $CH_3$ | $OC_2H_5$ | |
| 2-$CH_3$ | 6-$CH_3$ | H | 4 | O | $CH_3$ | $OCH_2CH_2OCH_3$ | |
| 3-Cl | 5-Cl | H | 4 | O | $CH_3$ | $OCH_2CH_2CO_2CH_3$ | |
| 3-Cl | 5-Cl | $CH_3$ | 4 | O | $OCH_3$ | $OCH_3$ | |

TABLE III (continued)

| $R_1$ | $R_2$ | $R_4$ | Position $-SO_2N<$ | W | X | Y | m.p. |
|---|---|---|---|---|---|---|---|
| 3-Br | 5-Br | H | 4 | S | $CH_3$ | $OCH_2CF_3$ | |
| 3-Cl | H | H | 4 | O | $CH_3$ | Cl | |
| 3-Br | H | H | 4 | O | $OCH_3$ | $OCH_3$ | |
| 3-$CH_3$ | H | H | 4 | O | $CH_3$ | $SC_2H_5$ | |
| 2-$CO_2$-*n*-$C_4H_9$ | H | H | 4 | O | $CH_3$ | $OCH(CH_3)CO_2CH_3$ | |
| 5-$NO_2$ | 2-Cl | H | 4 | O | $CH_3$ | $OCH_3$ | |
| 5-Cl | H | H | 4 | O | $CH_3$ | $OCH_3$ | |
| H | H | H | 3 | O | $CH_3$ | $CH_3$ | |
| H | H | H | 3 | S | $CH_3$ | $OCH_3$ | |
| H | H | H | 3 | O | $OCH_3$ | $OCH_3$ | 230° dec. |
| 2-Cl | H | H | 3 | O | $OCH_3$ | $OCH_3$ | 144—150° |
| 2-Cl | H | H | 3 | O | $CH_3$ | $CH_2OCH_3$ | |
| 2-Cl | H | H | 3 | O | $CH_3$ | $CH_3$ | 155—158° |
| 2-$NO_2$ | H | H | 3 | O | $CH_3$ | $CH_3$ | |
| 2-$NO_2$ | H | H | 3 | O | $CH_3$ | $OCH_3$ | |
| 2-$NO_2$ | H | H | 3 | O | $OCH_3$ | $OCH_3$ | |
| 2-$CO_2CH_3$ | H | H | 3 | O | $OCH_3$ | OCH | |
| 2-$CO_2CH_3$ | H | H | 3 | O | $CH_3$ | $OCH_3$ | |
| 2-$CO_2CH_3$ | H | H | 3 | O | $CH_3$ | $CH_3$ | |
| 2-$CO_2$-*i*-$C_3H_7$ | H | H | 3 | O | $CH_3$ | $CH_3$ | |
| 2-$CO_2$-*i*-$C_3H_7$ | H | H | 3 | O | $CH_3$ | $OCH_3$ | |
| 2-$CO_2$-*i*-$C_3H_7$ | H | H | 3 | O | $OCH_3$ | $OCH_3$ | |
| 2-$CH_3$ | H | H | 3 | O | $OCH_3$ | $OCH_3$ | |
| 2-$CH_3$ | H | H | 3 | O | $CH_3$ | $OCH_3$ | |
| 2-$CH_3$ | H | H | 3 | O | $CH_3$ | $CH_3$ | |
| 4-Cl | H | H | 3 | O | $CH_3$ | $CH_3$ | |
| 4-Cl | H | H | 3 | S | $CH_3$ | $OCH_3$ | |
| 4-Cl | H | H | 3 | O | $OCH_3$ | $OCH_3$ | |
| 4-$NO_2$ | H | H | 3 | O | $OCH_3$ | $OCH_3$ | |

TABLE III (continued)

| $R_1$ | $R_2$ | $R_4$ | Position $-SO_2N<$ | W | X | Y | m.p. |
|---|---|---|---|---|---|---|---|
| 4-$NO_2$ | H | H | 3 | O | $CH_3$ | $OCH_3$ | |
| 4-$NO_2$ | H | H | 3 | O | $CH_3$ | $CH_3$ | |
| 4-$CO_2CH_3$ | H | H | 3 | O | $CH_3$ | $CH_3$ | |
| 4-$CO_2CH_3$ | H | H | 3 | O | $CH_3$ | $OCH_3$ | |
| 4-$CO_2CH_3$ | H | H | 3 | O | $OCH_3$ | $OCH_3$ | |
| 4-$CO_2$-*i*-$C_3H_7$ | H | H | 3 | O | $CH_3$ | $CH_3$ | |
| 4-$CO_2$-*i*-$C_3H_7$ | H | H | 3 | O | $OCH_3$ | $OCH_3$ | |
| 4-$CO_2$-*i*-$C_3H_7$ | H | H | 3 | O | $CH_3$ | $OCH_3$ | |
| 4-$CH_3$ | H | H | 3 | S | $CH_3$ | $OCH_3$ | |
| 4-$CH_3$ | H | H | 3 | O | $CH_3$ | $CH_3$ | |
| 4-$CH_3$ | H | H | 3 | O | $OCH_3$ | $OCH_3$ | |
| H | H | H | 4 | O | $OCH_3$ | $OCH_3$ | |
| H | H | H | 4 | O | $CH_3$ | $OCH_3$ | |
| H | H | H | 4 | O | $CH_3$ | $CH_3$ | |
| 3-Cl | H | H | 4 | S | $OCH_3$ | $OCH_3$ | |
| 3-Cl | H | H | 4 | O | $CH_3$ | $CH_3$ | |
| 3-CL | H | H | 4 | S | $CH_3$ | $OCH_3$ | |
| 3-$NO_2$ | H | H | 4 | S | $CH_3$ | $CH_3$ | |
| 3-$NO_2$ | H | H | 4 | O | $OCH_3$ | $OCH_3$ | |
| 3-$NO_2$ | H | H | 4 | O | $CH_3$ | $OCH_3$ | |
| 3-$CO_2CH_3$ | H | H | 4 | O | $OCH_3$ | $OCH_3$ | |
| 3-$CO_2CH_3$ | H | H | 4 | S | $CH_3$ | $OCH_3$ | |
| 3-$CO_2CH_3$ | H | H | 4 | O | $CH_3$ | $CH_3$ | |
| 3-$CO_2$-*i*-$C_3H_7$ | H | H | 4 | O | $CH_3$ | $CH_3$ | |
| 3-$CO_2$-*i*-$C_3H_7$ | H | H | 4 | O | $CH_3$ | $OCH_3$ | |
| 3-$CO_2$-*i*-$C_3H_7$ | H | H | 4 | O | $OCH_3$ | $OCH_3$ | |
| 3-$CH_3$ | H | H | 4 | O | $CH_3$ | $CH_3$ | |
| 3-$CH_3$ | H | H | 4 | O | $CH_3$ | $OCH_3$ | |
| 3-$CH_3$ | H | H | 4 | O | $OCH_3$ | $OCH_3$ | |

By using the procedure of Examples 2 or 3 with an equivalent amount of a 3-amino-1,2,4-triazine and appropriately substituted sulfonylisocyanate or isothiocyanate, the compounds of Table IV can be prepared.

TABLE IV

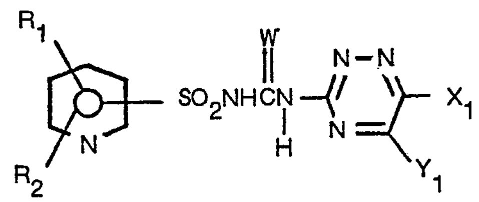

| $R_1$ | $R_2$ | Position —$SO_2NHR$ | W | $Y_1$ | $X_1$ |
|---|---|---|---|---|---|
| 2-Cl | H | 3 | O | $CH_3$ | $OCH_3$ |
| 2-Cl | H | 3 | O | $CH_3$ | $OCH_3$ |
| 2-Cl | H | 3 | S | $OCH_3$ | $OCH_3$ |
| 5-$C_2H_5$ | 2-$CH_3$ | 3 | O | $CH_3$ | $CH_3$ |
| 5-Br | 6-Cl | 3 | O | $CH_3$ | $OCH_3$ |
| 4-$CO_2C_2H_5$ | H | 3 | O | $CH_3$ | $CH_3$ |
| 6-$CO_2CH_3$ | H | 3 | O | $CH_3$ | $CH_3$ |
| 6-$OCH_3$ | H | 3 | O | $CH_3$ | $CH_3$ |
| 6-F | H | 3 | S | $CH_3$ | $CH_3$ |
| 6-Br | H | 3 | O | $OCH_3$ | $OCH_3$ |
| 6-$SC_2H_5$ | H | 3 | S | $OCH_3$ | $CH_3$ |
| 4-F | H | 3 | S | $OCH_3$ | $CH_3$ |
| 5-$NO_2$ | 6-Cl | 3 | O | $CH_3$ | $CH_3$ |
| 2-Cl | H | 3 | O | H | $CH_3$ |
| 2-Cl | H | 3 | O | $CH_3$ | $OCH_2CH_2$ |
| 2-$C_2H_5$ | H | 4 | O | $CH_3$ | $CH_3$ |
| 3-Cl | 5-Cl | 4 | O | $CH_3$ | $CH_3$ |
| 3-Br | H | 4 | S | $OCH_3$ | $OCH_3$ |

By using the procedure of Examples 2 or 3 with an equivalent amount of the appropriate 4-aminopyrimidines and appropriately substituted sulfonylisocyanate or isothiocyanate, the compounds of Table V can be prepared.

TABLE V

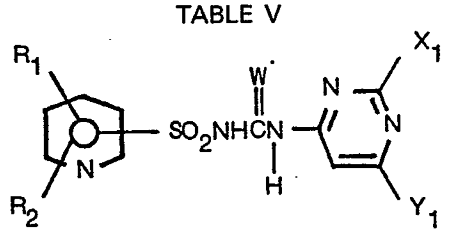

| $R_1$ | $R_2$ | Position of $—SO_2N$ | W | $Y_1$ | $X_1$ |
|---|---|---|---|---|---|
| 3-Br | H | 4 | O | $OCH_3$ | $CH_3$ |
| 3-Cl | 5-Cl | 4 | O | $CH_3$ | $OCH_3$ |
| $2-C_2H_5$ | H | 4 | O | $OCH_3$ | $OCH_3$ |
| $4-n-C_4H_9$ | H | 3 | S | $OCH_3$ | $CH_3$ |
| $5-NO_2$ | H | 3 | O | $OCH_3$ | $OCH_3$ |
| $5-NO_2$ | 6-Cl | 3 | O | $OCH_3$ | $OCH_3$ |
| 4-F | H | 3 | O | $OCH_3$ | $C_2H_5$ |
| $6-SC_2H_5$ | H | 3 | S | $OCH_3$ | $OCH_3$ |
| 2-Br | H | 3 | O | $CH_3$ | Cl |
| 6-F | H | 3 | O | $CH_3$ | $OCH_2CH_3$ |
| $6-OCH_3$ | H | 3 | O | $OCH_3$ | H |
| $2-CO_2CH_3$ | H | 3 | O | $OCH_3$ | $OCH_2CH_3$ |
| $4-CO_2CH_3$ | H | 3 | O | $OCH_3$ | Cl |
| $4-CO_2C_2H_5$ | H | 3 | O | $OCH_3$ | $OCH_3$ |
| 5-Br | 6-Cl | 3 | S | $OCH_3$ | H |
| $5-C_2H_5$ | $2-CH_3$ | 3 | O | $OCH_3$ | Cl |
| 2-Cl | H | 3 | O | $OCH_3$ | $OC_2H_5$ |
| 2-Cl | H | 3 | O | $OCH_3$ | $CH_3$ |
| 2-Cl | H | 3 | S | $OCH_3$ | Cl |

By using an appropriate N-[(triazinyl)aminocarbonyl]pyridinesulfonamide or N[(pyrimidinyl)-aminocarbonyl]pyridinesulfonamide, the compounds of Formula I (where $R_3 = CH_3$) set forth in Table VI can be prepared. For example, the compound of Example 2 can be converted to N - [(4 - methoxy - 6 - methyl - 1,3,5 - triazin - 2 - yl)aminocarbonyl] - 2 - chloro - N - methyl - 3 - pyridinesulfonamide by the methylation reaction set forth in Equation 3 as follows: An equivalent amount of sodium hydride (50% mineral oil dispersion) can be added to a solution of the compound of Example 2 in dimethylformamide under a nitrogen atmosphere. After hydrogen evolution has ceased, an equivalent amount of dimethyulsulfonate can be added. The resulting reaction mixture can be stirred for 2—18 hours and the reaction mixture can then be poured into a large volume of water to form a precipitate which can be filtered to yield the above-identified product.

TABLE VI

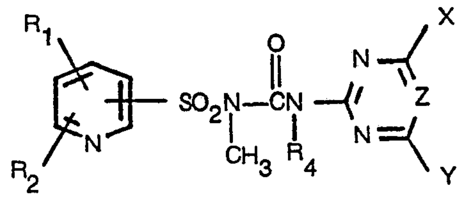

| $R_1$ | $R_2$ | $R_4$ | Position $-SO_2N<$ | X | Y | Z |
|---|---|---|---|---|---|---|
| 5-Br | 3-Br | H | 2 | $CH_3$ | $OCH_3$ | N |
| $3-CO_2CH_3$ | H | H | 2 | $CH_3$ | $OCH_3$ | N |
| $5-NO_2$ | H | H | 2 | $CH_3$ | $OCH_3$ | CH |
| $3-NO_2$ | H | $CH_3$ | 2 | $CH_3$ | $OCH_2CF_3$ | CH |
| $3-NO_2$ | H | $CH_3$ | 2 | $OCH_3$ | $OCH_3$ | N |
| 2-Cl | H | H | 3 | $OCH_3$ | $OCH_3$ | CH |
| 2-Cl | H | H | 3 | $CH_3$ | $OCH_3$ | N |
| 2-Cl | H | $CH_3$ | 3 | $CH_3$ | $OCH_3$ | CH |
| 2-Cl | H | H | 3 | $CH_3$ | $OCH_3$ | CH |
| $2-CO_2CH_3$ | H | H | 3 | $CH_3$ | $N(CH_3)_2$ | CH |
| $2-CO_2CH_3$ | H | H | 3 | $CH_3$ | $N(CH_3)_2$ | N |
| $5-CO_2C_2H_5$ | H | H | 3 | $CH_3$ | $OCH_2CH_2OCH_3$ | CH |
| $5-CO_2$-*n*-$C_4H_9$ | H | $CH_3$ | 3 | $CH_3$ | $CH_2OCH_3$ | N |
| $5-CH_3$ | 6-Cl | H | 3 | $CH_3$ | $N(CH_3)_2$ | N |
| $5-CH_3$ | 6-CL | $CH_3$ | 3 | $CH_3$ | $SC_2H_5$ | CH |
| $5-CH_3$ | 6-Cl | H | 3 | $CH_3$ | $OCH_3$ | N |
| $5-NO_2$ | 6-Cl | H | 3 | $OCH_3$ | $OCH_3$ | N |
| $5-NO_2$ | 6-Cl | H | 3 | $OCH_3$ | $OCH_3$ | CH |
| $5-NO_2$ | H | $CH_3$ | 3 | $CH_3$ | Cl | CH |
| $5-NO_2$ | H | H | 3 | $CH_3$ | $OCH_3$ | N |
| 6-Br | H | H | 3 | $CH_3$ | $OCH_2CO_2C_2H_5$ | CH |
| $5-NO_2$ | 6-Cl | $CH_3$ | 3 | $CH_3$ | $S(CH_2)_2OC_2H_5$ | CH |
| 5-F | 6-Cl | H | 3 | $CH_3$ | $CF_3$ | CH |
| 5-F | 6-Cl | H | 3 | $CH_3$ | $OC_2H_5$ | N |
| 3-Cl | 5-Cl | H | 4 | $CH_3$ | $CF_3$ | CH |

TABLE VI (continued)

| $R_1$ | $R_2$ | $R_4$ | Position $-SO_2N<$ | X | Y | Z |
|---|---|---|---|---|---|---|
| 3-Cl | 5-Cl | $CH_3$ | 4 | $CH_3$ | $OCH(CH_3)CO_2C_2H_5$ | N |
| 3-Cl | 5-Cl | H | 4 | $OCH_3$ | $OCH_3$ | N |
| 3-Br | H | H | 4 | $CH_3$ | $SC_2H_5$ | CH |
| 3-Br | H | $CH_3$ | 4 | $CH_3$ | Cl | CH |
| 3-Br | H | H | 4 | $CH_3$ | $OCH_2CH_2OCH_3$ | CH |
| 3-Br | H | $CH_3$ | 4 | $CH_3$ | $SCH_2CO_2CH_3$ | N |
| 2-$CO_2$-*n*-$C_6H_{13}$ | H | H | 4 | $OCH_3$ | $OCH_3$ | CH |
| 5-$NO_2$ | 2-Cl | H | 4 | $CH_3$ | $OC_2H_5$ | N |
| 5-$NO_2$ | 2-Cl | H | 4 | $CH_3$ | $CF_3$ | CH |
| 5-$NO_2$ | 2-Cl | $CH_3$ | 4 | $CH_3$ | $OCH_2CF_3$ | N |
| 5-Cl | H | H | 4 | $CH_3$ | $OCH_3$ | N |
| 5-Cl | H | H | 4 | $OCH_3$ | $OCH_3$ | CH |

Example 6

N-(3-Pyridinylsulfonyl)carbonimidodithioic acid dimethyl ester

To a stirred solution of 15.8 g of pyridine-3-sulfonamide in 70 ml of N,N-dimethylformamide was added 6.6 g of powdered potassium hydroxide. The mixture was stirred for 50 minutes at room temperature and 3 ml of carbon disulfide was added dropwise. The solution was then stirred at room temperature for 0.5 hr., then 3.3 g of powdered potassium hydroxide and 1.5 ml of carbon disulfide was added. The mixture was stirred for 0.5 hr., then 3.3 g of powdered potassium hydroxide and 1.5 ml of carbon disulfide was added. The mixture was stirred for 0.5 hr. again, and cooled to 0—5°, then 13 ml of iodomethane was added dropwise. The resulting mixture was stirred overnight and poured onto 250 g of ice-water. The precipitate was filtered and rinsed with water until neutral, then dried at reduced pressure to give 17 g of pale yellow solid, m.p. 115—118°

Using the method described in Example 6, the compounds of Table VII can be prepared from the appropriately substituted pyridinesulfonamide and the appropriate alkylating agent.

Table VII

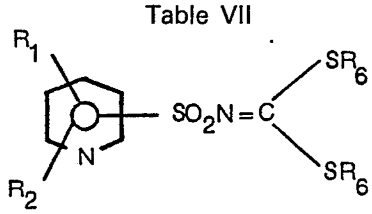

| $R_1$ | $R_2$ | Position 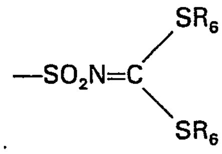 | $R_6$ |
|---|---|---|---|
| 5-Cl | H | 2 | $CH_3$ |
| 6-Cl | H | 2 | $CH_3$ |
| 6-Cl | 3-$CH_3$ | 2 | $CH_3$ |
| 6-Br | 3-$CH_3$ | 2 | $CH_3$ |
| 5-Br | H | 2 | $CH_3$ |
| 5-Br | 3-Br | 2 | $CH_3$ |
| 3-Br | H | 2 | $CH_3$ |
| 4-$OC_2H_5$ | 3-Br | 2 | $CH_3$ |
| 3-F | H | 2 | $CH_3$ |
| 4-$OCH_3$ | H | 2 | $CH_3$ |
| 5-F | H | 2 | $CH_3$ |
| 3-$COOCH_3$ | H | 2 | $CH_2CH_3$ |
| 4-COO-*n*-$C_6H_{13}$ | H | 2 | $CH_3$ |
| 3-$OCH_3$ | Cl | 2 | $CH_3$ |
| 6-F | H | 2 | $CH_3$ |
| 5-$NO_2$ | H | 2 | $CH_3$ |
| 3-$NO_2$ | H | 2 | $CH_3$ |
| 4-$CH_3$ | H | 2 | $CH_3$ |
| 4-$C_2H_5$ | H | 2 | $CH_3$ |
| 3-$CH_3$ | H | 2 | $CH_2CH_2CH_2CH_3$ |
| 6-Cl | H | 3 | $CH_3$ |
| 2-$CH_3$ | 6-$CH_3$ | 3 | $CH_3$ |
| 5-$CH_3$ | H | 3 | $CH_3$ |
| 6-$CH_3$ | H | 3 | $CH_3$ |
| 5-$C_2H_5$ | 2-$CH_3$ | 3 | $CH_3$ |
| 5-*n*-$C_4H_9$ | 2-$CH_3$ | 3 | $CH_3$ |

Table VII (continued)

| $R_1$ | $R_2$ | Position 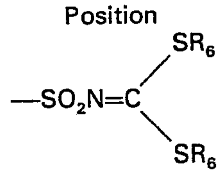 | $R_6$ |
|---|---|---|---|
| 2-Cl | 6-Cl | 3 | $CH_3$ |
| 4-$CO_2C_2H_5$ | H | 3 | $CH_3$ |
| 5-$CO_2CH_3$ | H | 3 | $CH_3$ |
| 2-$CO_2$-i-$C_3H_7$ | H | 3 | $CH_3$ |
| 4-$OCH_3$ | H | 3 | $CH_3$ |
| 6-*n*-$C_4H_9$ | H | 3 | $CH_3$ |
| 5-$CH_3$ | 6-Cl | 3 | $CH_2CH_2CH_3$ |
| 5-$NO_2$ | 6-Cl | 3 | $CH_2CH_3$ |
| 4-Cl | H | 3 | $CH_3$ |
| 6-F | H | 3 | $CH_3$ |
| 6-Br | H | 3 | $CH_3$ |
| 6-$SC_2H_5$ | H | 3 | $CH_3$ |
| 4-$SC_4H_9$ | H | 3 | $CH_3$ |
| 2-$SC_2H_5$ | H | 3 | $CH_3$ |
| 4-F | H | 3 | $CH_3$ |
| 4-Br | H | 3 | $CH_3$ |
| 6-$SCH_3$ | H | 3 | $CH_3$ |
| 5-$NO_2$ | 6-Cl | 3 | $CH_3$ |
| 5-Br | H | 3 | $CH(CH_3)_2$ |
| 5-$NO_2$ | H | 3 | $CH_3$ |
| 4-*n*-$C_4H_9$ | H | 3 | $CH_3$ |
| 6-*n*-$C_4H_9$ | H | 3 | $CH_3$ |
| 5-F | 6-Cl | 3 | $CH_3$ |
| 5-$NO_2$ | 6-Br | 3 | $CH_3$ |
| 2-$C_2H_5$ | H | 4 | $CH_3$ |
| 2-$CH_3$ | 6-$CH_3$ | 4 | $CH_3$ |
| 3-Cl | 5-Cl | 4 | $CH_3$ |
| 3-Br | 5-Br | 4 | $CH_3$ |
| H | 3-Cl | 4 | $CH_3$ |

Table VII (continued)

| $R_1$ | $R_2$ | Position 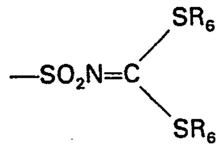 | $R_6$ |
|---|---|---|---|
| 3-Br | H | 4 | $CH_3$ |
| 3-$CH_3$ | H | 4 | $CH_3$ |
| 2-$CO_2$-*n*-$C_6H_{13}$ | H | 4 | $CH_3$ |
| 5-$NO_2$ | 2-Cl | 4 | $CH_3$ |
| H | H | 2 | $CH_3$ |
| 3-Cl | H | 2 | $CH_3$ |
| 3-$CO_2CH_3$ | H | 2 | $CH_3$ |
| 3-$CO_2$-*i*-$C_3H_7$ | H | 2 | $CH_3$ |
| 3-$CH_3$ | H | 2 | $CH_3$ |
| 3-$NO_2$ | H | 2 | $CH_3$ |
| 3-Cl | H | 2 | $CH_3$ |
| 3-$CO_2CH_3$ | H | 2 | $CH_3$ |
| 3-$CO_2$-*i*-$C_3H_7$ | H | 2 | $CH_3$ |
| 3-$CH_3$ | H | 2 | $CH_2CH_2CH_3$ |
| 3-$NO_2$ | H | 2 | $CH_2CH_2CH_3$ |
| H | H | 3 | $CH_2CH_2CH_3$ |
| 2-Cl | 4-Cl | 3 | $CH_2CH_2CH_3$ |
| 2-$NO_2$ | H | 3 | $CH_2CH_2CH_3$ |
| 2-$CO_2$-*i*-$C_3H_7$ | H | 3 | $CH_2CH_2CH_3$ |
| 2-$CO_2CH_3$ | H | 3 | $CH_2CH_2CH_3$ |
| H | H | 3 | $CH_2CH_2CH_3$ |
| 2-Cl | H | 3 | $CH_2CH_2CH_3$ |
| 2-$NO_2$ | H | 3 | $CH_2CH_2CH_3$ |
| 2-$CO_2$-*i*-$C_3H_7$ | H | 3 | $CH_2CH_2CH_3$ |
| 2-$CO_2CH_3$ | H | 3 | $CH_2CH_2CH_3$ |
| 2-$CH_3$ | H | 3 | $CH_2CH_2CH_3$ |
| 2-$CH_3$ | H | 3 | $CH_2CH_2CH_3$ |
| H | H | 3 | $CH_2CH_3$ |
| H | H | 3 | $CH_2CH_2CH_2CH_3$ |

TABLE VII (continued)

| $R_1$ | $R_2$ | Position 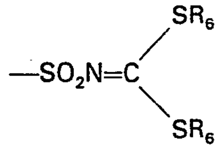 | $R_6$ |
|---|---|---|---|
| 4-Cl | H | 3 | $CH_3$ |
| 4-$NO_2$ | H | 3 | $CH_3$ |
| 4-$NO_2$ | H | 3 | $CH_3$ |
| 4-$CH_3$ | H | 3 | $CH_3$ |
| 4-$CH_3$ | H | 3 | $CH_3$ |
| 4-$CO_2CH_3$ | H | 3 | $CH_2CH_2CH_2CH_3$ |
| 4-$CO_2CH_3$ | H | 3 | $CH_3$ |
| 4-$CO_2$-*i*-$C_3H_7$ | H | 3 | $CH_3$ |
| 4-$CO_2$-*i*-$C_3H_7$ | H | 3 | $CH_3$ |
| H | H | 4 | $CH_3$ |
| H | H | 4 | $CH_3$ |
| 3-Cl | H | 4 | $CH_3$ |
| 3-Cl | H | 4 | $CH_3$ |
| 3-$NO_2$ | H | 4 | $CH_3$ |
| 3-$NO_2$ | H | 4 | $CH_3$ |
| 3-$CH_3$ | H | 4 | $CH_3$ |
| 3-$CH_3$ | H | 4 | $CH_3$ |
| 3-$CO_2CH_3$ | H | 4 | $CH_3$ |
| 3-$CO_2CH_3$ | H | 4 | $CH_3$ |
| 3-$CO_2$-*i*-$C_3H_7$ | H | 4 | $CH_3$ |
| 3-$CO_2$-*i*-$C_3H_7$ | H | 4 | $CH_2CH_2CH_3$ |

Example 7

N-(4-Methoxy-6-methyl-1,3,5-triazin-2-yl)-N'-(3-pyridinylsulfonyl)carbamimidothioic acid, methyl ester

To a suspension of 0.9 g of sodium hydride (50% oil dispersion) in 25 ml of dry N,N-dimethylformamide was added 2.3 g of 2-amino-4,6-dimethoxypyrimidine. The resulting yellow suspension was stirred at room temperature for 3 hrs., then added to 3.95 g of 3-pyridinylsulfonylcarbonimidodithioic acid, dimethyl ester. The reddish slurry was stirred at room temperature for 2 hours, poured into 250 g of ice-water and filtered to remove 1.6 g of unreacted, carbonimidodithioic acid, dimethyl ester. The filtrate was adjusted to pH 4 by adding 10% hydrochloric acid and the precipitate was filtered off, rinsed with water until neutral and then dried *in vacuo* to give 2.6 g of the desired compound, m.p. 146—149°.

By using the procedure of Example 7 with an equivalent amount of an appropriate aminopyrimidine, or aminotriazine and an appropriately substituted pyridinylsulfonylcarbonimidodithioic acid, dialkyl ester, the compounds of Tables VIII, IX, X and XI can be prepared.

TABLE VIII

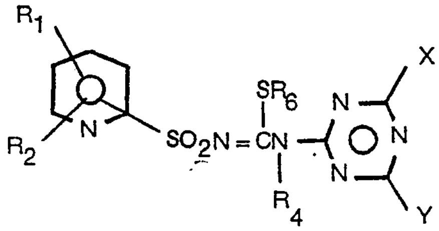

| $R_1$ | $R_2$ | $R_4$ | Position $-SO_2N$ | X | Y | $R_6$ |
|---|---|---|---|---|---|---|
| 5-Cl | H | H | 2 | $CH_3$ | H | $CH_3$ |
| 6-Cl | H | H | 2 | H | Cl | $CH_3$ |
| 6-Cl | 3-$CH_3$ | H | 2 | $OCH_3$ | $OCH_3$ | $CH_3$ |
| 6-Br | 3-$CH_3$ | H | 2 | $CH_3$ | $OCH_3$ | $CH_3$ |
| 5-Br | H | H | 2 | $CH_3$ | $OCH_3$ | $CH_3$ |
| 5-Br | 3-Br | H | 2 | $OCH_3$ | $OCH_3$ | $CH_3$ |
| 5-F | H | $CH_3$ | 2 | $CH_3$ | $CH_2OCH_3$ | $CH_3$ |
| 3-Br | H | $CH_3$ | 2 | $OCH_3$ | $OCH_3$ | $CH_3$ |
| 4-$OC_2H_5$ | 3-Br | H | 2 | $CH_3$ | $OC_2H_5$ | $CH_2CH_3$ |
| 3-F | H | H | 2 | $CH_3$ | $OCH_3$ | $CH_3$ |
| 3-$OCH_3$ | H | $CH_3$ | 2 | $CH_3$ | $CH_2OCH_3$ | $CH_3$ |
| 6-F | H | H | 2 | $CH_3$ | $OCH_2CF_3$ | $CH_3$ |
| 5-$NO_2$ | H | H | 2 | $CH_3$ | $N(CH_3)_2$ | $CH_3$ |
| 4-$CH_3$ | H | H | 2 | $CH_3$ | Cl | $CH_3$ |
| 4-$C_2H_5$ | H | H | 2 | $CH_3$ | $CF_3$ | $CH_3$ |
| 2-Cl | H | $CH_3$ | 3 | $CH_3$ | $OCH_3$ | $CH_2CH_2CH_3$ |
| 6-Cl | H | H | 3 | $CH_3$ | $OCH_3$ | $CH_3$ |
| 2-$CH_3$ | 6-$CH_3$ | H | 3 | $CH_3$ | $OCH_3$ | $CH_3$ |
| 4-$CH_3$ | H | $CH_3$ | 3 | $CH_3$ | $OCH_2CH_2OCH_3$ | $CH_3$ |
| 2-$CO_2CH_2CH_2Cl$ | H | H | 3 | $CH_3$ | $OCH_3$ | $CH_3$ |
| 2-$CO_2CH_2CH{=}CH_2$ | H | H | 3 | $CH_3$ | $OCH_3$ | $CH_3$ |
| 2-$CO_2CH_2CH_2OCH_3$ | H | H | 3 | $OCH_3$ | $OCH_3$ | $CH_3$ |
| 6-$CH_3$ | H | H | 3 | $CH_3$ | $OCH_2CO_2CH_3$ | $CH_3$ |
| 5-$C_2H_5$ | 2-$CH_3$ | H | 3 | $CH_3$ | $OCH_2CO_2C_2H_5$ | $CH_3$ |
| 5-Br | 6-Cl | H | 3 | $CH_3$ | $SCH_2CO_2CH_3$ | $CH_3$ |
| 2-$CO_2CH_3$ | H | H | 3 | $CH_3$ | $S(CH_2)_2CO_2C_2H_5$ | $CH_3$ |

TABLE VIII (continued)

| $R_1$ | $R_2$ | $R_4$ | Position —$SO_2N$ | X | Y | $R_6$ |
|---|---|---|---|---|---|---|
| 4-$CO_2C_2H_5$ | H | H | 3 | $OCH_3$ | $OCH_3$ | $CH_3$ |
| 6-$CO_2CH_3$ | H | H | 3 | $CH_3$ | $OCH_3$ | $CH(CH_3)_2$ |
| 4-$OCH_3$ | H | H | 3 | $CH_3$ | $OCH_3$ | $CH_3$ |
| 6-$OCH_3$ | H | H | 3 | $OC_2H_5$ | $OCH_3$ | $CH_3$ |
| 4-$CH_3$ | 2-Cl | H | 3 | $CH_3$ | $CF_3$ | $CH_3$ |
| 5-$NO_2$ | 6-Cl | H | 3 | $CH_3$ | $OCH_2CF_3$ | $CH_3$ |
| 4-Cl | H | H | 3 | $CH_3$ | $N(CH_3)_2$ | $CH_3$ |
| 6-Br | H | H | 3 | $CH_3$ | Cl | $CH_3$ |
| 6-$OC_2H_5$ | H | H | 3 | $CH_3$ | $SC_2H_5$ | $CH_3$ |
| 4-S-*n*-$C_4H_9$ | H | H | 3 | $OCH_3$ | $OCH_3$ | $CH_3$ |
| 2-$SC_2H_5$ | H | H | 3 | $CH_3$ | $OCH_3$ | $CH_3$ |
| 4-F | H | H | 3 | $CH_3$ | $OCH_2CH_2CO_2C_2H_5$ | $CH_3$ |
| 6-$SCH_3$ | H | H | 3 | $CH_3$ | $OCH_3$ | $CH_3$ |
| 5-$NO_2$ | 6-Cl | $CH_3$ | 3 | $OCH_3$ | $OCH_3$ | $CH_3$ |
| 5-Br | H | H | 3 | $OCH_3$ | $OCH_3$ | $CH_2CH_2CH_2CH_3$ |
| 5-$NO_2$ | H | H | 3 | $CH_3$ | $CF_3$ | $CH_3$ |
| 6-*n*-$C_4H_9$ | H | H | 3 | $OCH_3$ | $OCH_3$ | $CH_3$ |
| 5-F | 6-Cl | H | 3 | $OC_2H_5$ | $CH_2OCH_3$ | $CH_3$ |
| 2-$C_2H_5$ | H | $CH_3$ | 4 | $CH_3$ | $OCH_3$ | $CH_3$ |
| 2-$C_2H_5$ | H | H | 4 | $CH_3$ | $OC_2H_5$ | $CH_3$ |
| 2-$CH_3$ | 6-$CH_3$ | H | 4 | $CH_3$ | $OCH_2CH_2OCH_3$ | $CH_3$ |
| 3-Cl | 5-Cl | H | 4 | $CH_3$ | $OCH_2CH_2CO_2CH_3$ | $CH_3$ |
| 3-Cl | 5-Cl | $CH_3$ | 4 | $OCH_3$ | $OCH_3$ | $CH_3$ |
| 3-Cl | H | H | 4 | $CH_3$ | Cl | $CH_3$ |
| 3-Br | H | H | 4 | $OCH_3$ | $OCH_3$ | $CH_3$ |
| 3-$CH_3$ | H | H | 4 | $CH_3$ | $SC_2H_5$ | $CH_3$ |
| 2-$CO_2$-*n*-$C_4H_9$ | H | H | 4 | $CH_3$ | $OCH(CH_3)CO_2CH_3$ | $CH_3$ |
| 5-$NO_2$ | 2-Cl | H | 4 | $CH_3$ | $OCH_3$ | $CH_3$ |
| 5-Cl | H | H | 4 | $CH_3$ | $OCH_3$ | $CH_3$ |
| H | H | H | 2 | $CH_3$ | $CH_3$ | $CH_2CH_3$ |

TABLE VIII (continued)

| $R_1$ | $R_2$ | $R_4$ | Position —$SO_2N$ | X | Y | $R_6$ |
|---|---|---|---|---|---|---|
| H | H | H | 2 | $CH_3$ | $OCH_3$ | $CH_3$ |
| H | H | H | 2 | $OCH_3$ | $OCH_3$ | $CH_3$ |
| 3-Cl | H | H | 2 | $CH_3$ | $OCH_3$ | $CH_3$ |
| 3-Cl | H | H | 2 | $CH_3$ | $CH_3$ | $CH_3$ |
| 3-$NO_2$ | H | H | 2 | $CH_3$ | $CH_3$ | $CH_3$ |
| 3-$NO_2$ | H | H | 2 | $CH_3$ | $OCH_3$ | $CH_3$ |
| 3-$CO_2CH_3$ | H | H | 2 | $OCH_3$ | $OCH_3$ | $CH_3$ |
| 3-$CO_2CH_3$ | H | H | 2 | $CH_3$ | $OCH_3$ | $CH_2CH_2CH_3$ |
| 3-$CO_2CH_3$ | H | H | 2 | $CH_3$ | $CH_3$ | $CH_3$ |
| 3-$CO_2$-*i*-$C_3H_7$ | H | H | 2 | $CH_3$ | $CH_3$ | $CH_3$ |
| 3-$CH_3$ | H | H | 2 | $OCH_3$ | $OCH_3$ | $CH_3$ |
| 3-$CH_3$ | H | H | 2 | $CH_3$ | $OCH_3$ | $CH_3$ |
| 3-$CH_3$ | H | H | 2 | $CH_3$ | $CH_3$ | $CH_3$ |
| 3-H | H | H | 3 | $CH_3$ | $CH_3$ | $CH_3$ |
| 3-H | H | H | 3 | $OCH_3$ | $OCH_3$ | $CH_3$ |
| 2-Cl | H | H | 3 | $OCH_3$ | $OCH_3$ | $CH_3$ |
| 2-Cl | H | H | 3 | $CH_3$ | $CH_2OCH_3$ | $CH_3$ |
| 2-Cl | H | H | 3 | $CH_3$ | $CH_3$ | $CH_3$ |
| 2-$NO_2$ | H | H | 3 | $CH_3$ | $CH_3$ | $CH_3$ |
| 2-$NO_2$ | H | H | 3 | $CH_3$ | $OCH_3$ | $CH_3$ |
| 2-$NO_2$ | H | H | 3 | $OCH_3$ | $OCH_3$ | $CH_3$ |
| 2-$CO_2CH_3$ | H | H | 3 | $OCH_3$ | OCH | $CH_3$ |
| 2-$CO_2CH_3$ | H | H | 3 | $CH_3$ | $OCH_3$ | $CH_3$ |
| 2-$CO_2CH_3$ | H | H | 3 | $CH_3$ | $CH_3$ | $CH_3$ |
| 2-$CO_2$-*i*-$C_3H_7$ | H | H | 3 | $CH_3$ | $CH_3$ | $CH_3$ |
| 2-$CO_2$-*i*-$C_3H_7$ | H | H | 3 | $CH_3$ | $OCH_3$ | $CH_3$ |
| 2-$CO_2$-*i*-$C_3H_7$ | H | H | 3 | $OCH_3$ | $OCH_3$ | $CH_3$ |
| 2-$CH_3$ | H | H | 3 | $OCH_3$ | $OCH_3$ | $CH_3$ |
| 2-$CH_3$ | H | H | 3 | $CH_3$ | $OCH_3$ | $CH_3$ |
| 2-$CH_3$ | H | H | 3 | $CH_3$ | $CH_3$ | $CH_3$ |

TABLE VIII (continued)

| $R_1$ | $R_2$ | $R_4$ | Position $—SO_2N$ | X | Y | $R_6$ |
|---|---|---|---|---|---|---|
| 4-Cl | H | H | 3 | $CH_3$ | $CH_3$ | $CH_3$ |
| 4-Cl | H | H | 3 | $OCH_3$ | $OCH_3$ | $CH_3$ |
| 4-$NO_2$ | H | H | 3 | $OCH_3$ | $OCH_3$ | $CH_3$ |
| 4-$NO_2$ | H | H | 3 | $CH_3$ | $OCH_3$ | $CH_3$ |
| 4-$NO_2$ | H | H | 3 | $CH_3$ | $CH_3$ | $CH_3$ |
| 4-$CO_2CH_3$ | H | H | 3 | $CH_3$ | $CH_3$ | $CH_3$ |
| 4-$CO_2CH_3$ | H | H | 3 | $CH_3$ | $OCH_3$ | $CH_3$ |
| 4-$CO_2CH_3$ | H | H | 3 | $OCH_3$ | $OCH_3$ | $CH_3$ |
| 4-$CO_2$-*i*-$C_3H_7$ | H | H | 3 | $CH_3$ | $CH_3$ | $CH_3$ |
| 4-$CO_2$-*i*-$C_3H_7$ | H | H | 3 | $OCH_3$ | $OCH_3$ | $CH_3$ |
| 4-$CO_2$-*i*-$C_3H_7$ | H | H | 3 | $CH_3$ | $OCH_3$ | $CH_3$ |
| 4-$CH_3$ | H | H | 3 | $CH_3$ | $CH_3$ | $CH_3$ |
| 4-$CH_3$ | H | H | 3 | $OCH_3$ | $OCH_3$ | $CH_2CH_2CH_3$ |
| H | H | H | 4 | $OCH_3$ | $OCH_3$ | $CH_3$ |
| H | H | H | 4 | $CH_3$ | $OCH_3$ | $CH_3$ |
| H | H | H | 4 | $CH_3$ | $CH_3$ | $CH_3$ |
| 3-Cl | H | H | 4 | $CH_3$ | $CH_3$ | $CH_3$ |
| 3-$NO_2$ | H | H | 4 | $OCH_3$ | $OCH_3$ | $CH_3$ |
| 3-$NO_2$ | H | H | 4 | $CH_3$ | $OCH_3$ | $CH_3$ |
| 3-$CO_2CH_3$ | H | H | 4 | $OCH_3$ | $OCH_3$ | $CH_3$ |
| 3-$CO_2CH_3$ | H | H | 4 | $CH_3$ | $CH_3$ | $CH_3$ |
| 3-$CO_2$-*i*-$C_3H_7$ | H | H | 4 | $CH_3$ | $CH_3$ | $CH_3$ |
| 3-$CO_2$-*i*-$C_3H_7$ | H | H | 4 | $CH_3$ | $OCH_3$ | $CH_3$ |
| 3-$CO_2$-*i*-$C_3H_7$ | H | H | 4 | $OCH_3$ | $OCH_3$ | $CH_2CH_2CH_2CH_2CH_3$ |
| 3-$CH_3$ | H | H | 4 | $CH_3$ | $CH_3$ | $CH_3$ |
| 3-$CH_3$ | H | H | 4 | $CH_3$ | $OCH_3$ | $CH_3$ |
| 3-$CH_3$ | H | H | 4 | $OCH_3$ | $OCH_3$ | $CH_3$ |

TABLE IX

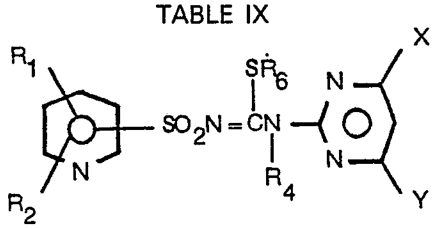

| $R_1$ | $R_2$ | $R_4$ | Position —$SO_2N$ | X | Y | $R_6$ |
|---|---|---|---|---|---|---|
| 5-Cl | H | H | 2 | $CH_3$ | H | $CH_3$ |
| 6-Cl | H | H | 2 | H | Cl | $CH_3$ |
| 6-Cl | 3-$CH_3$ | H | 2 | $OCH_3$ | $OCH_3$ | $CH_3$ |
| 6-Br | 3-$CH_3$ | H | 2 | $CH_3$ | $OCH_3$ | $CH_3$ |
| 5-Br | H | H | 2 | $CH_3$ | $OCH_3$ | $CH_3$ |
| 5-Br | 3-Br | H | 2 | $OCH_3$ | $OCH_3$ | $CH_3$ |
| 3-Br | H | $CH_3$ | 2 | $OCH_3$ | $OCH_3$ | $CH_3$ |
| 4-$OC_2H_5$ | 3-Br | H | 2 | $CH_3$ | $OC_2H_5$ | $CH_3$ |
| 3-F | H | H | 2 | $CH_3$ | $OCH_3$ | $CH_3$ |
| 5-F | H | $CH_3$ | 2 | $CH_3$ | $CH_2OCH_3$ | $CH_3$ |
| 3-$OCH_3$ | H | $CH_3$ | 2 | $CH_2$ | $CH_2OCH_3$ | $CH_3$ |
| 6-F | H | H | 2 | $CH_3$ | $OCH_2CF_3$ | $CH_2CH_3$ |
| 5-$NO_2$ | H | H | 2 | $CH_3$ | $N(CH_3)_2$ | $CH_3$ |
| 4-$CH_3$ | H | H | 2 | $CH_3$ | Cl | $CH_3$ |
| 4-$C_2H_5$ | H | H | 2 | $CH_3$ | $CF_3$ | $CH_3$ |
| 2-Cl | H | $CH_3$ | 3 | $CH_3$ | $OCH_3$ | $CH_3$ |
| 6-Cl | H | H | 3 | $CH_3$ | $OCH_3$ | $CH_3$ |
| 2-$CH_3$ | 6-$CH_3$ | H | 3 | $CH_3$ | $OCH_3$ | $CH_3$ |
| 4-$CH_3$ | H | $CH_3$ | 3 | $CH_3$ | $OCH_2CH_2OCH_3$ | $CH_3$ |
| 6-Cl | H | H | 3 | $CH_3$ | $CH_3$ | $CH_3$ |
| 6-Cl | H | H | 3 | $OCH_3$ | $OCH_3$ | $CH_3$ |
| 2-$CO_2CH_2CH{=}CH_2$ | H | H | 3 | $CH_3$ | $OCH_3$ | $CH_3$ |
| 2-$CO_2CH$-$CH{=}CH(CH_2)_2CH_3$ | H | H | 3 | $CH_3$ | $OCH_3$ | $CH_2CH_2CH_3$ |
| 2-$CO_2CH_2CH_2OCH_3$ | H | H | 3 | $CH_3$ | $CH_3$ | $CH_3$ |
| 2-$CO_2CH_2CH_2OCH_2CH_3$ | H | H | 3 | $CH_3$ | $OCH_3$ | $CH_3$ |
| 2-$CO_2CH_2CH_2CH_2OCH_3$ | H | H | 3 | $CH_3$ | $OCH_3$ | $CH_3$ |
| 2-$CO_2CH_2CH_2Cl$ | H | H | 3 | $OCH_3$ | $OCH_3$ | $CH_3$ |

TABLE IX (continued)

| $R_1$ | $R_2$ | $R_4$ | Position —$SO_2N$ | X | Y | $R_6$ |
|---|---|---|---|---|---|---|
| 6-$CH_3$ | H | H | 3 | $CH_3$ | $OCH_2CO_2CH_3$ | $CH_3$ |
| 5-$C_2H_5$ | 2-$CH_3$ | H | 3 | $CH_3$ | $OCH_2CO_2C_2H_5$ | $CH_3$ |
| 5-Br | 6-Cl | H | 3 | $CH_3$ | $SCH_2CO_2CH_3$ | $CH_3$ |
| 2-$CO_2CH_3$ | H | H | 3 | $CH_3$ | $S(CH_2)_2CO_2C_2H_5$ | $CH_3$ |
| 4-$CO_2C_2H_5$ | H | H | 3 | $OCH_3$ | $OCH_3$ | $CH_3$ |
| 6-$CO_2CH_3$ | H | H | 3 | $CH_3$ | $OCH_3$ | $CH_3$ |
| 4-$OCH_3$ | H | H | 3 | $CH_3$ | $OCH_3$ | $CH_3$ |
| 6-$OCH_3$ | H | H | 3 | $OC_2H$ | $OCH_3$ | $CH_3$ |
| 5-$CH_3$ | 6-Cl | H | 3 | $CH_3$ | $CF_3$ | $CH_3$ |
| 5-$NO_2$ | 6-Cl | H | 3 | $CH_3$ | $OCH_2CF_3$ | $CH_3$ |
| 4-Cl | H | H | 3 | $CH_3$ | $N(CH_3)_2$ | $CH_3$ |
| 6-Br | H | H | 3 | $CH_3$ | Cl | $CH_2CH_2CH_2CH_2CH_3$ |
| 6-$OC_2H_5$ | H | H | 3 | $CH_3$ | $SC_2H_5$ | $CH_3$ |
| 4-S-*n*-$C_4H_9$ | H | H | 3 | $OCH_3$ | $OCH_3$ | $CH_3$ |
| 2-$SC_2H_5$ | H | H | 3 | $CH_3$ | $OCH_3$ | $CH_3$ |
| 4-F | H | H | 3 | $CH_3$ | $OCH_2CH_2CO_2C_2H_5$ | $CH_3$ |
| 6-$SCH_3$ | H | H | 3 | $CH_3$ | $OCH_3$ | $CH_3$ |
| 5-$NO_2$ | 6-Cl | $CH_3$ | 3 | $OCH_3$ | $OCH_3$ | $CH_3$ |
| 5-Br | H | H | 3 | $OCH_3$ | $OCH_3$ | $CH_3$ |
| 5-$NO_2$ | H | H | 3 | $CH_3$ | $CF_3$ | $CH_3$ |
| 6-*n*-$C_4H_9$ | H | H | 3 | $OCH_3$ | $OCH_3$ | $CH_3$ |
| 5-F | 6-Cl | H | 3 | $OC_2H_5$ | $CH_2OCH_3$ | $CH_3$ |
| 2-Cl | 4-Cl | H | 3 | $OCH_3$ | $OCH_3$ | $CH_3$ |
| 2-Cl | 4-Cl | H | 3 | $CH_3$ | $OCH_3$ | $CH_3$ |
| 2-$C_2H_5$ | H | $CH_3$ | 4 | $CH_3$ | $OCH_3$ | $CH_3$ |
| 2-$C_2H_5$ | H | H | 4 | $CH_3$ | $OC_2H_5$ | $CH_3$ |
| 2-$CH_3$ | 6-$CH_3$ | H | 4 | $CH_3$ | $OCH_2CH_2OCH_3$ | $CH_3$ |
| 3-Cl | 5-Cl | H | 4 | $CH_3$ | $OCH_2CH_2CO_2CH_3$ | $CH_3$ |
| 3-Cl | 5-Cl | $CH_3$ | 4 | $OCH_3$ | $OCH_3$ | $CH_3$ |
| 3-Cl | H | H | 4 | $CH_3$ | Cl | $CH_3$ |

TABLE IX (continued)

| $R_1$ | $R_2$ | $R_4$ | Position —$SO_2N$ | X | Y | $R_6$ |
|---|---|---|---|---|---|---|
| 3-Br | H | H | 4 | $OCH_3$ | $OCH_3$ | $CH_3$ |
| 3-$CH_3$ | H | H | 4 | $CH_3$ | $SC_2H_5$ | $CH_3$ |
| 2-$CO_2$-*n*-$C_4H_9$ | H | H | 4 | $CH_3$ | $OCH(CH_3)CO_2CH_3$ | $CH_3$ |
| 5-$NO_2$ | 2-Cl | H | 4 | $CH_2$ | $OCH_3$ | $CH_3$ |
| 5-Cl | H | H | 4 | $CH_3$ | $OCH_3$ | $CH_3$ |
| H | H | H | 2 | $CH_3$ | $CH_3$ | $(CH(CH_3)_2$ |
| H | H | H | 2 | $CH_3$ | $OCH_3$ | $CH_3$ |
| H | H | H | 2 | $OCH_3$ | $OCH_3$ | $CH_3$ |
| 3-Cl | H | H | 2 | $CH_3$ | $OCH_3$ | $CH_3$ |
| 3-Cl | H | H | 2 | $CH_3$ | $CH_3$ | $CH_3$ |
| 3-$NO_2$ | H | H | 2 | $CH_3$ | $CH_3$ | $CH_3$ |
| 3-$NO_2$ | H | H | 2 | $CH_3$ | $OCH_3$ | $CH_3$ |
| 3-$CO_2CH_3$ | H | H | 2 | $OCH_3$ | $OCH_3$ | $CH_3$ |
| 3-$CO_2CH_3$ | H | H | 2 | $CH_3$ | $OCH_3$ | $CH_3$ |
| 3-$CO_2CH_3$ | H | H | 2 | $CH_3$ | $CH_3$ | $CH_3$ |
| 3-$CO_2$-*i*-$C_3H_7$ | H | H | 2 | $CH_2$ | $CH_3$ | $CH_3$ |
| 3-$CH_3$ | H | H | 2 | $OCH_3$ | $OCH_3$ | $CH_3$ |
| 3-$CH_3$ | H | H | 2 | $CH_3$ | $OCH_3$ | $CH_3$ |
| 3-$CH_3$ | H | H | 2 | $CH_3$ | $CH_3$ | $CH_3$ |
| H | H | H | 3 | $CH_3$ | $CH_3$ | $CH_3$ |
| H | H | H | 3 | $OCH_3$ | $OCH_3$ | $CH_3$ |
| 2-Cl | H | H | 3 | $OCH_3$ | $OCH_3$ | $CH_3$ |
| 2-Cl | H | H | 3 | $CH_3$ | —$CH_2OCH_3$ | $CH_3$ |
| 2-Cl | H | H | 3 | $CH_3$ | $CH_3$ | $CH_3$ |
| 2-$NO_2$ | H | H | 3 | $CH_3$ | $CH_3$ | $CH_3$ |
| 2-$NO_2$ | H | H | 3 | $CH_3$ | $OCH_3$ | $CH_3$ |
| 2-$NO_2$ | H | H | 3 | $OCH_3$ | $OCH_3$ | $CH_3$ |
| 2-$CO_2CH_3$ | H | H | 3 | $OCH_3$ | OCH | $CH_3$ |
| 2-$CO_2CH_3$ | H | H | 3 | $CH_3$ | $OCH_3$ | $CH_3$ |
| 2-$CO_2CH_3$ | H | H | 3 | $CH_3$ | $CH_3$ | $CH_3$ |

TABLE IX (continued)

| $R_1$ | $R_2$ | $R_4$ | Position $-SO_2N$ | X | Y | $R_6$ |
|---|---|---|---|---|---|---|
| 2-$CO_2$-*i*-$C_3H_7$ | H | H | 3 | $CH_3$ | $CH_3$ | $CH_3$ |
| 2-$CO_2$-*i*-$C_3H_7$ | H | H | 3 | $CH_3$ | $OCH_3$ | $CH_3$ |
| 2-$CO_2$-*i*-$C_3H_7$ | H | H | 3 | $OCH_3$ | $OCH_3$ | $CH_3$ |
| 2-$CH_3$ | H | H | 3 | $OCH_3$ | $OCH_3$ | $CH_2CH_2CH_2CH_3$ |
| 2-$CH_3$ | H | H | 3 | $CH_3$ | $OCH_3$ | $CH_3$ |
| 2-$CH_3$ | H | H | 3 | $CH_3$ | $CH_3$ | $CH_3$ |
| 4-Cl | H | H | 3 | $CH_3$ | $CH_3$ | $CH_3$ |
| 4-Cl | H | H | 3 | $OCH_3$ | $OCH_3$ | $CH_3$ |
| 4-$NO_2$ | H | H | 3 | $OCH_3$ | $OCH_3$ | $CH_3$ |
| 4-$NO_2$ | H | H | 3 | $CH_3$ | $OCH_3$ | $CH_3$ |
| 4-$NO_2$ | H | H | 3 | $CH_3$ | $CH_3$ | $CH_3$ |
| 4-$CO_2CH_3$ | H | H | 3 | $CH_3$ | $CH_3$ | $CH_3$ |
| 4-$CO_2CH_3$ | H | H | 3 | $CH_3$ | $OCH_3$ | $CH_3$ |
| 4-$CO_2CH_3$ | H | H | 3 | $OCH_3$ | $OCH_3$ | $CH_3$ |
| 4-$CO_2$-*i*-$C_3H_7$ | H | H | 3 | $CH_3$ | $CH_3$ | $CH_2CH_3$ |
| 4-$CO_2$-*i*-$C_3H_7$ | H | H | 3 | $OCH_3$ | $OCH_3$ | $CH_3$ |
| 4-$CO_2$-*i*-$C_3H_7$ | H | H | 3 | $CH_3$ | $OCH_3$ | $CH_3$ |
| 4-$CH_3$ | H | H | 3 | $CH_3$ | $CH_3$ | $CH_3$ |
| 4-$CH_3$ | H | H | 3 | $OCH_3$ | $OCH_3$ | $CH_3$ |
| H | H | H | 4 | $OCH_3$ | $OCH_3$ | $CH_3$ |
| H | H | H | 4 | $CH_3$ | $OCH_3$ | $CH_3$ |
| H | H | H | 4 | $CH_3$ | $CH_3$ | $CH_3$ |
| 3-Cl | H | H | 4 | $CH_3$ | $CH_3$ | $CH_3$ |
| 3-$NO_2$ | H | H | 4 | $OCH_3$ | $OCH_3$ | $CH_3$ |
| 3-$NO_2$ | H | H | 4 | $CH_3$ | $OCH_3$ | $CH_3$ |
| 3-$CO_2CH_3$ | H | H | 4 | $OCH_3$ | $OCH_3$ | $CH_3$ |
| 3-$CO_2CH_3$ | H | H | 4 | $CH_3$ | $CH_3$ | $CH_2CH_3$ |
| 3-$CO_2$-*i*-$C_3H_7$ | H | H | 4 | $CH_3$ | $CH_3$ | $CH_3$ |
| 3-$CO_2$-*i*-$C_3H_7$ | H | H | 4 | $CH_3$ | $OCH_3$ | $CH_3$ |
| 3-$CO_2$-*i*-$C_3H_7$ | H | H | 4 | $OCH_3$ | $OCH_3$ | $CH_3$ |
| 3-$CH_3$ | H | H | 4 | $CH_3$ | $CH_3$ | $CH_3$ |
| 3-$CH_3$ | H | H | 4 | $CH_3$ | $OCH_3$ | $CH_3$ |
| 3-$CH_3$ | H | H | 4 | $OCH_3$ | $OCH_3$ | $CH_3$ |

TABLE X

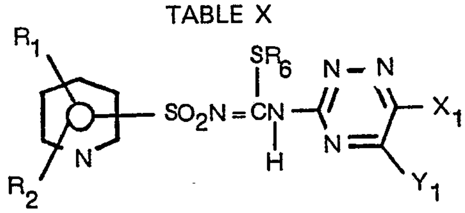

| $R_1$ | $R_2$ | Position —$SO_2NHR$ | $Y_1$ | $X_1$ | $R_6$ |
|---|---|---|---|---|---|
| 5-Cl | H | 2 | $CH_3$ | $CH_3$ | $CH_3$ |
| 6-Cl | 3-$CH_3$ | 2 | $CH_3$ | $CH_3$ | $CH_3$ |
| 5-Br | 3-Br | 2 | $OCH_3$ | $OCH_3$ | $CH_3$ |
| 4-$OC_2H_5$ | 3-Br | 2 | $OCH_3$ | $OCH_3$ | $CH_3$ |
| 4-F | H | 2 | $CH_3$ | $CH_3$ | $CH_3$ |
| 4-$C_2H_5$ | H | 2 | $CH_3$ | $OCH_3$ | $CH_3$ |
| 2-$C_2H_5$ | 2-$CH_3$ | 3 | $CH_3$ | $CH_3$ | $CH_3$ |
| 3-Br | 2-Cl | 5 | $CH_3$ | $OCH_3$ | $CH_3$ |
| 4-$CO_2C_2H_5$ | H | 3 | $CH_3$ | $CH_3$ | $CH_3$ |
| 2-$CO_2CH_3$ | H | 3 | $CH_3$ | $CH_3$ | $CH_3$ |
| 6-$OCH_3$ | H | 3 | $CH_3$ | $OCH_3$ | $CH_3$ |
| 6-Br | H | 3 | $OCH_3$ | $OCH_3$ | $CH_3$ |
| 5-$NO_2$ | 6-Cl | 3 | $CH_3$ | $CH_3$ | $CH_3$ |
| 2-$C_2H_5$ | H | 4 | $CH_3$ | $CH_3$ | $CH_2CH_2CH_3$ |
| 3-Cl | 5-Cl | 4 | $CH_3$ | $CH_3$ | $CH_3$ |
| 3-Br | H | 4 | $OCH_3$ | $OCH_3$ | $CH_3$ |

TABLE XI

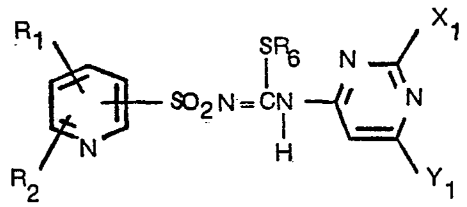

| $R_1$ | $R_2$ | Position of $—SO_2N$ | $Y_1$ | $X_1$ | $R_6$ |
|---|---|---|---|---|---|
| 3-Br | H | 4 | $OCH_3$ | $CH_3$ | $CH_3$ |
| 3-Cl | 5-Cl | 4 | $CH_3$ | $OCH_3$ | $CH_3$ |
| 2-$C_2H_5$ | H | 4 | $OCH_3$ | $OCH_3$ | $CH_3$ |
| 2-F | 5-$NO_2$ | 3 | $OCH_3$ | $OCH_3$ | $CH_3$ |
| 6-*n*-$C_4H_9$ | H | 3 | $OCH_3$ | $CH_3$ | $CH_3$ |
| 5-$NO_2$ | H | 3 | $OCH_3$ | $OCH_3$ | $CH_3$ |
| 5-$NO_2$ | 6-Cl | 3 | $OCH_3$ | $OCH_3$ | $CH_3$ |
| 6-$SC_2H_5$ | H | 3 | $OCH_3$ | $OCH_3$ | $CH_2CH_2CH_2CH_2CH_3$ |
| 6-$CO_2CH_3$ | H | 3 | $OCH_3$ | $OCH_2CH_3$ | $CH_3$ |
| 4-$CO_2CH_3$ | H | 3 | $OCH_3$ | Cl | $CH_3$ |
| 2-Cl | H | 3 | $OCH_3$ | $OCH_3$ | $CH_3$ |
| 4-$C_2H_5$ | H | 2 | $OCH_3$ | $OCH_3$ | $CH_2CH_3$ |
| 5-$NO_2$ | H | 2 | $OCH_3$ | $OCH_3$ | $CH_3$ |
| 4-$CO_2CH_3$ | H | 2 | $OCH_3$ | $OCH_3$ | $CH_3$ |
| 5-Cl | H | 2 | $OCH_3$ | $OCH_3$ | $CH_3$ |

### Example 8

N-[(4-methoxy-6-methyl-1,3,5-triazin-2-yl)aminocarbonyl]-3-pyridinesulfonamide

To 4 ml of 15% aqueous tetrahydrofuran was added with stirring 0.4 g of mercuric oxide and 0.28 ml of boron-trifluoride etherate. The mixture was stirred for 20 minutes, and then 0.4 g of N-(4-methoxy-6-methyl-1,3,5-triazin-2-yl)-N′-(3-pyridinylsulfonyl)carbamimidothioic acid, methyl ester was added. The slurry was stirred at room temperature for 1 hour, filtered and the solid was washed with 50 ml of water, followed by 40 ml of dichloromethane, and dissolved with 25 ml of 0.1 N sodium hydroxide. The basic solution was filtered and the filtrate acidified with dilute hydrochloric acid to yield the desired product as a precipitate. The solid was filtered off, washed with water, and dried to give 0.2 g of pale yellow solid, m.p. 185—190°.

### Example 9

N-[(4-methoxy-6-methyl-1,3,5-triazin-2-yl)aminocarbonyl]-3-pyridinesulfonamide

To 25 ml of 80% aqueous acetonitrile was added with stirring 0.6 g of mercuric chloride and then 0.34 g of N-(4-methoxy-6-methyl-1,3,5-triazin-2-yl)-N′-(3-pyridinylsulfonyl)carbamimidothioic acid, methyl ester. To this mixture, 0.22 g of calcium carbonate was added to adjust the pH to 7. The resulting mixture was heated to reflux for 2 hours, then cooled to room temperature and filtered. The solid was rinsed with 25 ml of dichloromethane three times, 25 ml of water three times, and then dissolved in 30 ml of 0.1N of sodium hydroxide. Acidification of the basic solution with dilute hydrochloric acid yielded the desired product as a precipitate which was filtered off, rinsed with water, and dried at reduced pressure to give 0.2 g of pale yellow solid, m.p. 185—190°.

By using the procedures of Examples 8 or 9, the compounds of Table XII can be prepared from the appropriately substituted pyridinesulfonyl carbamimidothioic acid esters.

TABLE XII

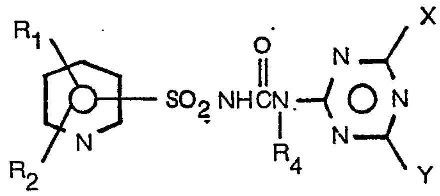

| $R_1$ | $R_2$ | $R_4$ | Position —$SO_2N$ | X | Y |
|---|---|---|---|---|---|
| 3-Cl | H | H | 2 | $CH_3$ | H |
| 4-Cl | H | H | 2 | H | Cl |
| 6-Cl | 3-$CH_3$ | H | 2 | $OCH_3$ | $OCH_3$ |
| 6-Br | 3-$CH_3$ | H | 2 | $CH_3$ | $OCH_3$ |
| 3-Br | H | H | 2 | $CH_3$ | $OCH_3$ |
| 5-Br | 3-Br | H | 2 | $OCH_3$ | $OCH_3$ |
| 5-F | H | $CH_3$ | 2 | $CH_3$ | $CH_2OCH_3$ |
| 3-Br | H | $CH_3$ | 2 | $OCH_3$ | $OCH_3$ |
| 4-$OC_2H_5$ | 3-Br | H | 2 | $CH_3$ | $OC_2H_5$ |
| 3-F | H | H | 2 | $CH_3$ | $OCH_3$ |
| 3-$OCH_3$ | H | $CH_3$ | 2 | $CH_3$ | $CH_2OCH_3$ |
| 6-F | H | H | 2 | $CH_3$ | $OCH_2CF_3$ |
| 4-$NO_2$ | H | H | 2 | $CH_3$ | $N(CH_3)_2$ |
| 4-$CH_3$ | H | H | 2 | $CH_3$ | Cl |
| 4-$C_2H_5$ | H | H | 2 | $CH_3$ | $CF_3$ |
| 2-Cl | H | $CH_3$ | 3 | $CH_3$ | $OCH_3$ |
| 6-Cl | H | H | 3 | $CH_3$ | $OCH_3$ |
| 2-$CH_3$ | 6-$CH_3$ | H | 3 | $CH_3$ | $OCH_3$ |
| 4-$CH_3$ | H | $CH_3$ | 3 | $CH_3$ | $OCH_2CH_2OCH_3$ |
| 2-$CO_2CH_2CH_2Cl$ | H | H | 3 | $CH_3$ | $OCH_3$ |
| 2-$CO_2CH_2CH{=}CH_2$ | H | H | 3 | $CH_3$ | $OCH_3$ |
| 2-$CO_2CH_2CH_2OCH_3$ | H | H | 3 | $OCH_3$ | $OCH_3$ |

TABLE XII (continued)

| $R_1$ | $R_2$ | $R_4$ | Position $-SO_2N$ | X | Y |
|---|---|---|---|---|---|
| 6-$CH_3$ | H | H | 3 | $CH_3$ | $OCH_2CO_2CH_3$ |
| 5-$C_2H_5$ | 2-$CH_3$ | H | 3 | $CH_3$ | $OCH_2CO_2C_2H_5$ |
| 5-Br | 6-Cl | H | 3 | $CH_3$ | $SCH_2CO_2CH_3$ |
| 2-$CO_2CH_3$ | H | H | 3 | $CH_3$ | $S(CH_2)_2CO_2C_2H_5$ |
| 4-$CO_2C_2H_5$ | H | H | 3 | $OCH_3$ | $OCH_3$ |
| 2-$CO_2CH_3$ | H | H | 3 | $CH_3$ | $OCH_3$ |
| 4-$OCH_3$ | H | H | 3 | $CH_3$ | $OCH_3$ |
| 6-$OCH_3$ | H | H | 3 | $OC_2H_5$ | $OCH_3$ |
| 5-$CH_3$ | 6-Cl | H | 3 | $CH_3$ | $CF_3$ |
| 5-$NO_2$ | 6-Cl | H | 3 | $CH_3$ | $OCH_2CF_3$ |
| 4-Cl | H | H | 3 | $CH_3$ | $N(CH_3)_2$ |
| 2-Br | H | H | 3 | $CH_3$ | Cl |
| 2-$OC_2H_5$ | H | H | 3 | $CH_3$ | $SC_2H_5$ |
| 4-S-*n*-$C_4H_9$ | H | H | 3 | $OCH_3$ | $OCH_3$ |
| 2-$SC_2H_5$ | H | H | 3 | $CH_3$ | $OCH_3$ |
| 4-F | H | H | 3 | $CH_3$ | $OCH_2CH_2CO_2C_2H_5$ |
| 6-$SCH_3$ | H | H | 3 | $CH_3$ | $OCH_3$ |
| 5-$NO_2$ | 6-Cl | $CH_3$ | 3 | $OCH_3$ | $OCH_3$ |
| 5-Br | H | H | 3 | $OCH_3$ | $OCH_3$ |
| 5-$NO_2$ | H | H | 3 | $CH_3$ | $CF_3$ |
| 6-*n*-$C_4H_9$ | H | H | 3 | $OCH_3$ | $OCH_3$ |
| 5-F | 6-Cl | H | 3 | $OC_2H_5$ | $CH_2OCH_3$ |
| 2-$C_2H_5$ | H | $CH_3$ | 4 | $CH_3$ | $OCH_3$ |
| 2-$C_2H_5$ | H | H | 4 | $CH_3$ | $OC_2H_5$ |
| 2-$CH_3$ | 6-$CH_3$ | H | 4 | $CH_3$ | $OCH_2CH_2OCH_3$ |
| 3-Cl | 5-Cl | H | 4 | $CH_3$ | $OCH_2CH_2CO_2CH_3$ |
| 3-Cl | 5-Ci | $CH_3$ | 4 | $OCH_3$ | $OCH_3$ |
| 3-Cl | H | H | 4 | $CH_3$ | Cl |

TABLE XII (continued)

| $R_1$ | $R_2$ | $R_4$ | Position $-SO_2N$ | X | Y |
|---|---|---|---|---|---|
| 3-Br | H | H | 4 | $OCH_3$ | $OCH_3$ |
| $3-CH_3$ | H | H | 4 | $CH_3$ | $SC_2H_5$ |
| $2-CO_2-n-C_4H_9$ | H | H | 4 | $CH_3$ | $OCH(CH_3)CO_2CH_3$ |
| $5-NO_2$ | 2-Cl | H | 4 | $CH_3$ | $OCH_3$ |
| 5-Cl | H | H | 4 | $CH_3$ | $OCH_3$ |
| H | H | H | 2 | $CH_3$ | $CH_3$ |
| H | H | H | 2 | $CH_3$ | $OCH_3$ |
| H | H | H | 2 | $OCH_3$ | $OCH_3$ |
| 3-Cl | H | H | 2 | $CH_3$ | $OCH_3$ |
| 3-Cl | H | H | 2 | $CH_3$ | $CH_3$ |
| $3-NO_2$ | H | H | 2 | $CH_3$ | $CH_3$ |
| $3-NO_2$ | H | H | 2 | $CH_3$ | $OCH_3$ |
| $3-CO_2CH_3$ | H | H | 2 | $OCH_3$ | $OCH_3$ |
| $3-CO_2CH_3$ | H | H | 2 | $CH_3$ | $OCH_3$ |
| $3-CO_2CH_3$ | H | H | 2 | $CH_3$ | $CH_3$ |
| $3-CO_2-i-C_3H_7$ | H | H | 2 | $CH_3$ | $CH_3$ |
| $3-CH_3$ | H | H | 2 | $OCH_3$ | $OCH_3$ |
| $3-CH_3$ | H | H | 2 | $CH_3$ | $OCH_3$ |
| $3-CH_3$ | H | H | 2 | $CH_3$ | $CH_3$ |
| H | H | H | 3 | $CH_3$ | $CH_3$ |
| H | H | H | 3 | $OCH_3$ | $OCH_3$ |
| 2-Cl | H | H | 3 | $OCH_3$ | $OCH_3$ |
| 2-Cl | H | H | 3 | $CH_3$ | $CH_2OCH_3$ |
| 2-Cl | H | H | 3 | $CH_3$ | $CH_3$ |
| $2-NO_2$ | H | H | 3 | $CH_3$ | $CH_3$ |
| $2-NO_2$ | H | H | 3 | $CH_3$ | $OCH_3$ |
| $2-NO_2$ | H | H | 3 | $OCH_3$ | $OCH_3$ |
| $2-CO_2CH_3$ | H | H | 3 | $OCH_3$ | OCH |
| $2-CO_2CH_3$ | H | H | 3 | $CH_3$ | $OCH_3$ |
| $2-CO_2CH_3$ | H | H | 3 | $CH_3$ | $CH_3$ |

TABLE XII (continued)

| $R_1$ | $R_2$ | $R_4$ | Position —$SO_2N$ | X | Y |
|---|---|---|---|---|---|
| 2-$CO_2$-*i*-$C_3H_7$ | H | H | 3 | $CH_3$ | $CH_3$ |
| 2-$CO_2$-*i*-$C_3H_7$ | H | H | 3 | $CH_3$ | $OCH_3$ |
| 2-$CO_2$-*i*-$C_3H_7$ | H | H | 3 | $OCH_3$ | $OCH_3$ |
| 2-$CH_3$ | H | H | 3 | $OCH_3$ | $OCH_3$ |
| 2-$CH_3$ | H | H | 3 | $CH_3$ | $OCH_3$ |
| 2-$CH_3$ | H | H | 3 | $CH_3$ | $CH_3$ |
| 4-Cl | H | H | 3 | $CH_3$ | $CH_3$ |
| 4-Cl | H | H | 3 | $OCH_3$ | $OCH_3$ |
| 4-$NO_2$ | H | H | 3 | $OCH_3$ | $OCH_3$ |
| 4-$NO_2$ | H | H | 3 | $CH_3$ | $OCH_3$ |
| 4-$NO_2$ | H | H | 3 | $CH_3$ | $CH_3$ |
| 4-$CO_2CH_3$ | H | H | 3 | $CH_3$ | $CH_3$ |
| 4-$CO_2CH_3$ | H | H | 3 | $CH_3$ | $OCH_3$ |
| 4-$CO_2CH_3$ | H | H | 3 | $OCH_3$ | $OCH_3$ |
| 4-$CO_2$-*i*-$C_3H_7$ | H | H | 3 | $CH_3$ | $CH_3$ |
| 4-$CO_2$-*i*-$C_3H_7$ | H | H | 3 | $OCH_3$ | $OCH_3$ |
| 4-$CO_2$-*i*-$C_3H_7$ | H | H | 3 | $CH_3$ | $OCH_3$ |
| 4-$CH_3$ | H | H | 3 | $CH_3$ | $CH_3$ |
| 4-$CH_3$ | H | H | 3 | $OCH_3$ | $OCH_3$ |
| H | H | H | 4 | $OCH_3$ | $OCH_3$ |
| H | H | H | 4 | $CH_3$ | $OCH_3$ |
| H | H | H | 4 | $CH_3$ | $CH_3$ |
| 3-Cl | H | H | 4 | $CH_3$ | $CH_3$ |
| 3-$NO_2$ | H | H | 4 | $OCH_3$ | $OCH_3$ |
| 3-$NO_2$ | H | H | 4 | $CH_3$ | $OCH_3$ |
| 3-$CO_2CH_3$ | H | H | 4 | $OCH_3$ | $OCH_3$ |
| 3-$CO_2CH_3$ | H | H | 4 | $CH_3$ | $CH_3$ |
| 3-$CO_2$-*i*-$C_3H_7$ | H | H | 4 | $CH_3$ | $CH_3$ |
| 3-$CO_2$-*i*-$C_3H_7$ | H | H | 4 | $CH_3$ | $OCH_3$ |
| 3-$CO_2$-*i*-$C_3H_7$ | H | H | 4 | $OCH_3$ | $OCH_3$ |
| 3-$CH_3$ | H | H | 4 | $CH_3$ | $CH_3$ |
| 3-$CH_3$ | H | H | 4 | $CH_3$ | $OCH_3$ |
| 3-$CH_3$ | H | H | 4 | $OCH_3$ | $OCH_3$ |

Example 10

N-[(4,6-dimethoxypyrimidin-2-yl)aminocarbonyl]-3-pyridinesulfonamide

To 8 ml of 15% aqueous tetrahydrofuran was added with stirring 0.8 g of mercuric acid oxide and 0.56 ml of boron trifluoride etherate. The mixture was stirred for 20 minutes, and then 0,8 g of N-(4,6-dimethoxypyrimidin-2-yl)-N'-(3-pyridinylsulfonyl)carbamimidothioic acid, methyl ester was added. The slurry was stirred at room temperature for 1 hour, filtered, and the solid was rinsed with 50 ml of water, followed by 40 ml of dichloromethane, and dried to give 0.3 g of the desired product, m.p. 176° dec.

By using the procedure of Example 10, the compounds of Tables XIII, XIV and XV can be prepared from the appropriately substituted pyridinesulfonylcarbamimidothioic acid esters.

TABLE XIII

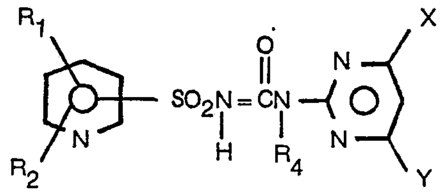

| $R_1$ | $R_2$ | $R_4$ | Position $-SO_2N$ | X | Y |
|---|---|---|---|---|---|
| 3-Cl | H | H | 2 | $CH_3$ | H |
| 6-Cl | H | H | 2 | H | Cl |
| 6-Cl | 3-$CH_3$ | H | 2 | $OCH_3$ | $OCH_3$ |
| 6-Br | 3-$CH_3$ | H | 2 | $CH_3$ | $OCH_3$ |
| 5-Br | H | H | 2 | $CH_3$ | $OCH_3$ |
| 5-Br | 3-Br | H | 2 | $OCH_3$ | $OCH_3$ |
| 3-Br | H | $CH_3$ | 2 | $OCH_3$ | $OCH_3$ |
| 4-$OC_2H_5$ | 3-Br | H | 2 | $CH_3$ | $OC_2H_5$ |
| 3-F | H | H | 2 | $CH_3$ | $OCH_3$ |
| 5-F | H | $CH_3$ | 2 | $CH_3$ | $CH_2OCH_3$ |
| 3-$OCH_3$ | H | $CH_3$ | 2 | $CH_3$ | $CH_2OCH_3$ |
| 6-F | H | H | 2 | $CH_3$ | $OCH_2CF_3$ |
| 5-$NO_2$ | H | H | 2 | $CH_3$ | $N(CH_3)_2$ |
| 4-$CH_3$ | H | H | 2 | $CH_3$ | Cl |
| 4-$C_2H_5$ | H | H | 2 | $CH_3$ | $CF_3$ |
| 2-Cl | H | $CH_3$ | 3 | $CH_3$ | $OCH_3$ |
| 4-Cl | H | H | 3 | $CH_3$ | $OCH_3$ |
| 2-$CH_3$ | 6-$CH_3$ | H | 3 | $CH_3$ | $OCH_3$ |
| 4-$CH_3$ | H | $CH_3$ | 3 | $CH_3$ | $OCH_2CH_2OCH_3$ |
| 6-Cl | H | H | 3 | $CH_3$ | $CH_3$ |
| 6-Cl | H | H | 3 | $OCH_3$ | $OCH_3$ |
| 2-$CO_2CH_2CH{=}CH_2$ | H | H | 3 | $CH_3$ | $OCH_3$ |

TABLE XIII (continued)

| $R_1$ | $R_2$ | $R_4$ | Position $—SO_2N$ | X | Y |
|---|---|---|---|---|---|
| $2\text{-}CO_2CH—CH{=}CH(CH_2)_2CH_3$ | H | H | 3 | $CH_3$ | $OCH_3$ |
| $2\text{-}CO_2CH_2CH_2OCH_3$ | H | H | 3 | $CH_3$ | $CH_3$ |
| $2\text{-}CO_2CH_2CH_2OCH_2CH_3$ | H | H | 3 | $CH_3$ | $OCH_3$ |
| $2\text{-}CO_2CH_2CH_2CH_2OCH_3$ | H | H | 3 | $CH_3$ | $OCH_3$ |
| $2\text{-}CO_2CH_2CH_2Cl$ | H | H | 3 | $OCH_3$ | $OCH_3$ |
| $6\text{-}CH_3$ | H | H | 3 | $CH_3$ | $OCH_2CO_2CH_3$ |
| $5\text{-}C_2H_5$ | $2\text{-}CH_3$ | H | 3 | $CH_3$ | $OCH_2CO_2C_2H_5$ |
| 5-Br | 6-Cl | H | 3 | $CH_3$ | $SCH_2CO_2CH_3$ |
| $2\text{-}CO_2CH_3$ | H | H | 3 | $CH_3$ | $S(CH_2)_2CO_2C_2H_5$ |
| $4\text{-}CO_2C_2H_5$ | H | H | 3 | $OCH_3$ | $OCH_3$ |
| $6\text{-}CO_2CH_3$ | H | H | 3 | $CH_3$ | $OCH_3$ |
| $4\text{-}OCH_3$ | H | H | 3 | $CH_3$ | $OCH_3$ |
| $6\text{-}OCH_3$ | H | H | 3 | $OC_2H$ | $OCH_3$ |
| $5\text{-}CH_3$ | 6-Cl | H | 3 | $CH_3$ | $CF_3$ |
| $5\text{-}NO_2$ | 6-Cl | H | 3 | $CH_3$ | $OCH_2CF_3$ |
| 4-Cl | H | H | 3 | $CH_3$ | $N(CH_3)_2$ |
| 6-Br | H | H | 3 | $CH_3$ | Cl |
| $6\text{-}OC_2H_5$ | H | H | 3 | $CH_3$ | $SC_2H_5$ |
| $4\text{-}S\text{-}n\text{-}C_4H_9$ | H | H | 3 | $OCH_3$ | $OCH_3$ |
| $2\text{-}SC_2H_5$ | H | H | 3 | $CH_3$ | $OCH_3$ |
| 4-F | H | H | 3 | $CH_3$ | $OCH_2CH_2CO_2C_2H_5$ |
| $6\text{-}SCH_3$ | H | H | 3 | $CH_3$ | $OCH_3$ |
| $5\text{-}NO_2$ | 6-Cl | $CH_3$ | 3 | $OCH_3$ | $OCH_3$ |
| 5-Br | H | H | 3 | $OCH_3$ | $OCH_3$ |
| $5\text{-}NO_2$ | H | H | 3 | $CH_3$ | $CF_3$ |
| $6\text{-}n\text{-}C_4H_9$ | H | H | 3 | $OCH_3$ | $OCH_3$ |
| 5-F | 6-Cl | H | 3 | $OC_2H_5$ | $CH_2OCH_3$ |
| 2-Cl | 4-Cl | H | 3 | $OCH_3$ | $OCH_3$ |
| 2-Cl | 4-Cl | H | 3 | $CH_3$ | $OCH_3$ |
| $2\text{-}C_2H_5$ | H | $CH_3$ | 4 | $CH_3$ | $OCH_3$ |

TABLE XIII (continued)

| $R_1$ | $R_2$ | $R_4$ | Position $—SO_2N$ | X | Y |
|---|---|---|---|---|---|
| $2-C_2H_5$ | H | H | 4 | $CH_3$ | $OC_2H_5$ |
| $2-CH_3$ | $6-CH_3$ | H | 4 | $CH_3$ | $OCH_2CH_2OCH_3$ |
| 3-Cl | 5-Cl | H | 4 | $CH_3$ | $OCH_2CH_2CO_2CH_3$ |
| 3-Cl | 5-Cl | $CH_3$ | 4 | $OCH_3$ | $OCH_3$ |
| 3-Cl | H | H | 4 | $CH_3$ | Cl |
| 3-Br | H | H | 4 | $OCH_3$ | $OCH_3$ |
| $3-CH_3$ | H | H | 4 | $CH_3$ | $SC_2H_5$ |
| $2-CO_2$-*n*-$C_4H_9$ | H | H | 4 | $CH_3$ | $OCH(CH_3)CO_2CH_3$ |
| $5-NO_2$ | 2-Cl | H | 4 | $CH_3$ | $OCH_3$ |
| 5-Cl | H | H | 4 | $CH_3$ | $OCH_3$ |
| H | H | H | 2 | $CH_3$ | $CH_3$ |
| H | H | H | 2 | $CH_3$ | $OCH_3$ |
| H | H | H | 2 | $OCH_3$ | $OCH_3$ |
| 3-Cl | H | H | 2 | $CH_3$ | $OCH_3$ |
| 3-Cl | H | H | 2 | $CH_3$ | $CH_3$ |
| $3-NO_2$ | H | H | 2 | $CH_3$ | $CH_3$ |
| $3-NO_2$ | H | H | 2 | $CH_3$ | $OCH_3$ |
| $3-CO_2CH_3$ | H | H | 2 | $OCH_3$ | $OCH_3$ |
| $3-CO_2CH_3$ | H | H | 2 | $CH_3$ | $OCH_3$ |
| $3-CO_2CH_3$ | H | H | 2 | $CH_3$ | $CH_3$ |
| $3-CO_2$-*i*-$C_3H_7$ | H | H | 2 | $CH_3$ | $CH_3$ |
| $3-CH_3$ | H | H | 2 | $OCH_3$ | $OCH_3$ |
| $3-CH_3$ | H | H | 2 | $CH_3$ | $OCH_3$ |
| $3-CH_3$ | H | H | 2 | $CH_3$ | $CH_3$ |
| H | H | H | 3 | $CH_3$ | $CH_3$ |
| H | H | H | 3 | $OCH_3$ | $OCH_3$ |
| 2-Cl | H | H | 3 | $OCH_3$ | $OCH_3$ |
| 2-Cl | H | H | 3 | $CH_3$ | $—CH_2OCH_3$ |
| 2-Cl | H | H | 3 | $CH_3$ | $CH_3$ |
| $2-NO_2$ | H | H | 3 | $CH_3$ | $CH_3$ |

TABLE XIII (continued)

| $R_1$ | $R_2$ | $R_4$ | Position —$SO_2N$ | X | Y |
|---|---|---|---|---|---|
| 2-$NO_2$ | H | H | 3 | $CH_3$ | $OCH_3$ |
| 2-$NO_2$ | H | H | 3 | $OCH_3$ | $OCH_3$ |
| 2-$CO_2CH_3$ | H | H | 3 | $OCH_3$ | OCH |
| 2-$CO_2CH_3$ | H | H | 3 | $CH_3$ | $OCH_3$ |
| 2-$CO_2CH_3$ | H | H | 3 | $CH_3$ | $CH_3$ |
| 2-$CO_2$-*i*-$C_3H_7$ | H | H | 3 | $CH_3$ | $CH_3$ |
| 2-$CO_2$-*i*-$C_3H_7$ | H | H | 3 | $CH_3$ | $OCH_3$ |
| 2-$CO_2$-*i*-$C_3H_7$ | H | H | 3 | $OCH_3$ | $OCH_3$ |
| 2-$CH_3$ | H | H | 3 | $OCH_3$ | $OCH_3$ |
| 2-$CH_3$ | H | H | 3 | $CH_3$ | $OCH_3$ |
| 2-$CH_3$ | H | H | 3 | $CH_3$ | $CH_3$ |
| 4-Cl | H | H | 3 | $CH_3$ | $CH_3$ |
| 4-Cl | H | H | 3 | $OCH_3$ | $OCH_3$ |
| 4-$NO_2$ | H | H | 3 | $OCH_3$ | $OCH_3$ |
| 4-$NO_2$ | H | H | 3 | $CH_3$ | $OCH_3$ |
| 4-$NO_2$ | H | H | 3 | $CH_3$ | $CH_3$ |
| 4-$CO_2CH_3$ | H | H | 3 | $CH_3$ | $CH_3$ |
| 4-$CO_2CH_3$ | H | H | 3 | $CH_3$ | $OCH_3$ |
| 4-$CO_2CH_3$ | H | H | 3 | $OCH_3$ | $OCH_3$ |
| 4-$CO_2$-*i*-$C_3H_7$ | H | H | 3 | $CH_3$ | $CH_3$ |
| 4-$CO_2$-*i*-$C_3H_7$ | H | H | 3 | $OCH_3$ | $OCH_3$ |
| 4-$CO_2$-*i*-$C_3H_7$ | H | H | 3 | $CH_3$ | $OCH_3$ |
| 4-$CH_3$ | H | H | 3 | $CH_3$ | $CH_3$ |
| 4-$CH_3$ | H | H | 3 | $OCH_3$ | $OCH_3$ |
| H | H | H | 4 | $OCH_3$ | $OCH_3$ |
| H | H | H | 4 | $CH_3$ | $OCH_3$ |
| H | H | H | 4 | $CH_3$ | $CH_3$ |
| 3-Cl | H | H | 4 | $CH_3$ | $CH_3$ |
| 3-$NO_2$ | H | H | 4 | $OCH_3$ | $OCH_3$ |
| 3-$NO_2$ | H | H | 4 | $CH_3$ | $OCH_3$ |

TABLE XIII (continued)

| $R_1$ | $R_2$ | $R_4$ | Position —$SO_2N$ | X | Y |
|---|---|---|---|---|---|
| 3-$CO_2CH_3$ | H | H | 4 | $OCH_3$ | $OCH_3$ |
| 3-$CO_2CH_3$ | H | H | 4 | $CH_3$ | $CH_3$ |
| 3-$CO_2$-*i*-$C_3H_7$ | H | H | 4 | $CH_3$ | $CH_3$ |
| 3-$CO_2$-*i*-$C_3H_7$ | H | H | 4 | $CH_3$ | $OCH_3$ |
| 3-$CO_2$-*i*-$C_3H_7$ | H | H | 4 | $OCH_3$ | $OCH_3$ |
| 3-$CH_3$ | H | H | 4 | $CH_3$ | $CH_3$ |
| 3-$CH_3$ | H | H | 4 | $CH_3$ | $OCH_3$ |
| 3-$CH_3$ | H | H | 4 | $OCH_3$ | $OCH_3$ |

TABLE XIV

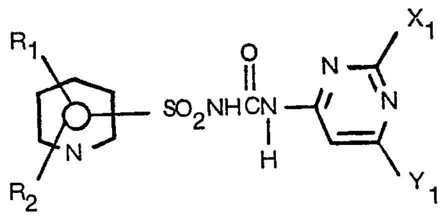

| $R_1$ | $R_2$ | Position —$SO_2NHR$ | $Y_1$ | $X_1$ |
|---|---|---|---|---|
| 4-Cl | H | 2 | $CH_3$ | $CH_3$ |
| 6-Cl | 3-$CH_3$ | 2 | $CH_3$ | $CH_3$ |
| 5-Br | 3-Br | 2 | $OCH_3$ | $OCH_3$ |
| 4-$OC_2H_5$ | 3-Br | 2 | $OCH_3$ | $OCH_3$ |
| 4-F | H | 2 | $CH_3$ | $CH_3$ |
| 4-$C_2H_5$ | H | 2 | $CH_3$ | OCH |
| 2-Cl | H | 3 | $CH_3$ | $OCH_3$ |
| 5-$C_2H_5$ | 2-$CH_3$ | 3 | $CH_3$ | $CH_3$ |
| 5-Br | 6-Cl | 3 | $CH_3$ | $OCH_3$ |
| 4-$CO_2C_2H_5$ | H | 3 | $CH_3$ | $CH_3$ |
| 6-$CO_2CH_3$ | H | 3 | $CH_3$ | $CH_3$ |
| 6-$OCH_3$ | H | 3 | $CH_3$ | $OCH_3$ |
| 5-Br | H | 3 | $OCH_3$ | $OCH_3$ |
| 5-$NO_2$ | 6-Cl | 3 | $CH_3$ | $CH_3$ |
| 2-$C_2H_5$ | H | 4 | $CH_3$ | $CH_3$ |
| 3-Cl | 5-Cl | 4 | $CH_3$ | $CH_3$ |
| 3-Br | H | 4 | $OCH_3$ | $OCH_3$ |

TABLE XV

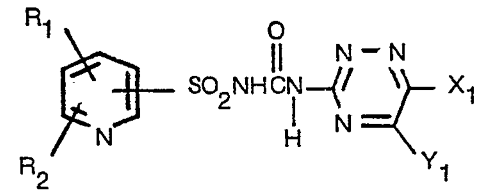

| $R_1$ | $R_2$ | Position of —$SO_2N$ | $Y_1$ | $X_1$ |
|---|---|---|---|---|
| 3-Br | H | 4 | $OCH_3$ | $CH_3$ |
| 3-Cl | 5-Cl | 4 | $CH_3$ | $OCH_3$ |
| 2-$C_2H_5$ | H | 4 | $OCH_3$ | $OCH_3$ |
| 6-F | 5-$NO_2$ | 3 | $OCH_3$ | $OCH_3$ |
| 6-n-$C_4H_9$ | H | 3 | $OCH_3$ | $CH_3$ |
| 5-$NO_2$ | H | 3 | $OCH_3$ | $OCH_3$ |
| 5-$NO_2$ | 6-Cl | 3 | $OCH_3$ | $OCH_3$ |
| 4-F | H | 3 | $OCH_3$ | $OC_2H_5$ |
| 6-$SC_2H_5$ | H | 3 | $OCH_3$ | $OCH_3$ |
| 6-$CO_2CH_3$ | H | 3 | $OCH_3$ | $OCH_2CH_2$ |
| 2-Cl | H | 3 | $OCH_3$ | $OCH_3$ |
| 4-$C_2H_5$ | H | 2 | $OCH_3$ | $OCH_3$ |
| 5-$NO_2$ | H | 2 | $OCH_3$ | $OCH_3$ |
| 4-$CO_2CH_3$ | H | 2 | $OCH_3$ | $OCH_3$ |
| 5-Cl | H | 2 | $OCH_3$ | $OCH_3$ |

Agriculturally useful acid addition salts of appropriate N-[(triazinyl)aminocarbonyl]pyridinesulfonamides or N-[(pyrimidinyl)aminocarbonyl]pyridinesulfonamides can be prepared as described in Equation 12 by treating the appropriate N-aminocarbonylpyridinesulfonamide with an equivalent amount of a suitable mineral or organic acid in a solvent such as methanol or ethanol and removing the solvent to yield the desired acid salt. By this method, acid addition salts can be prepared with anions such as: $Cl^-$, $Br^-$, $NO_3^-$, $HSO_4^-$, $SO_4^=$, $H_2PO_4^-$, $HPO_4^=$, $CCl_3COO^-$, $CH_3SO_3^-$, p-$CH_3C_6H_4SO_3$, and $C_{12}H_{25}SO_3^-$.

Agriculturally useful alkali metal salts can be prepared as described in Equation 10 by the reaction of a suitable N-[(triazinyl)-aminocarbonyl]pyridinesulfonamide or a N-(pyrimidinylaminocarbonyl)-pyridinesulfonamide with an equivalent of a suitable alkali metal alkoxide in an alcohol solvent (for example, sodium methoxide in methanol) removal of solvent and trituration usually performed with a solvent such as ethylacetate. In this manner, a variety of alkali metal salts can be made with cations such as: $Li^+$, $Na^+$, $K^+$, $Ca^{++}$.

It should be noted that there is no intent to be limited to the salts which are mentioned above.

Example 11

2-Chloro-N-[(4-methoxy-6-methyl-1,3,5-triazin-2-yl)aminocarbonyl]-3-pyridinesulfonamide, sodium salt.

To a stirred suspension of 1.0 g of 2-chloro-N-[(4-methoxy-6-methyl-1,3,5-triazin-2-yl)aminocarbonyl]-3-pyridinesulfonamide in 25 ml of methanol is added 0.15 g of sodium methoxide. The resulting solution is heated on a steam bath for 5 minutes, cooled and the solvent removed *in vacuo* to

yield the desired salt Trituration with ethylacetate yields the sodium salt of 2-chloro-N-[(4-methoxy-6-methyl-1,3,5-triazin-2-yl)aminocarbonyl]-3-pyridinesulfonamide as a colorless solid.

As described in Equation 11 and further illustrated in Example 12, reaction of a suitable N-(triazinylaminocarbonyl)pyridinesulfonamide or a N-(pyrimidinylaminocarbonyl)pyridinesulfonamide with an equivalent of an appropriate amine in a solvent such as methylene chloride or methanol followed by removal of this solvent yields agriculturally useful ammonium salts of N-(aminocarbonyl)-pyridinesulfonamides. Amines such as ammonia, ethylamine, diethylamine, triethylamine, trimethylamine, diethanolamine, triethanolamine, isopropylamine, pyridine, dimethylaniline, laurylamine or di-n-butylamine may be employed.

Example 12

2-Chloro-N-[(4-methoxy-6-methylpyrimidin-2-yl)aminocarbonyl]-3-pyridinesulfonamide, triethanolamine salt

To a stirring suspension of 1.0 g of 2-chloro-N-[(4-methoxy-6-methylpyrimidin-2-yl)aminocarbonyl]-3-pyridinesulfonamide in 25 ml of methylene chloride is added 0.42 g of triethanolamine. The solvent is removed *in vacuo* to yield as product the desired triethanolamine salt.

By using an appropriately substitued sulfonyl-N-alkylpyridinecarbamyl chloride or thiocarbamylchloride and an appropriate aminopyrimidine or aminotriazine, the compounds of Formula I set forth in Table XVI can be prepared by the procedure of Equation 5. For example, 2-chloro-N-[N-(4-methoxy-6-methylpyrimidin-2-yl]-N-methylaminocarbonyl]-N-methyl-3-pyridinesulfonamide can be prepared by reacting 2.1 g of N-[(2-chloro-3-pyridine)sulfonyl]-N'-methyl-carbamoyl chloride in 50 ml of tetrahydrofuran containing 1.0 g of triethylamine to 1.5 g of 2-methylamino-4-methoxy-6-methylpyrimidine. That mixture can be stirred at reflux for several hours, the precipitated salts can be filtered off and the filtrate can be concentrated to yield the desired product.

TABLE XVI

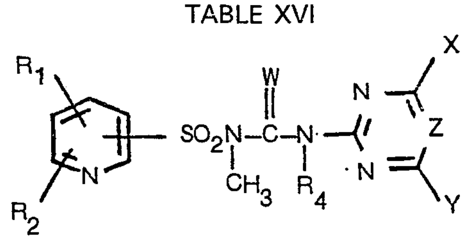

| $R_1$ | $R_2$ | $R_4$ | Position $-SO_2N$ | W | X | Y | Z |
|---|---|---|---|---|---|---|---|
| 5-Br | 3-Br | H | 2 | O | $CH_3$ | $OCH_3$ | N |
| 3-$CO_2CH_3$ | H | H | 2 | O | $CH_3$ | $OCH_3$ | N |
| 5-$NO_2$ | H | H | 2 | O | $CH_3$ | $OCH_3$ | CH |
| 3-$NO_2$ | H | $CH_3$ | 2 | S | $CH_3$ | $OCH_2CF_3$ | CH |
| 3-$NO_2$ | H | $CH_3$ | 2 | O | $OCH_3$ | $OCH_3$ | N |
| 2-Cl | H | H | 3 | O | $OCH_3$ | $OCH_3$ | CH |
| 2-Cl | H | H | 3 | O | $CH_3$ | $OCH_3$ | N |
| 2-Cl | H | $CH_3$ | 3 | S | $CH_3$ | $OCH_3$ | CH |
| 2-Cl | H | H | 3 | O | $CH_3$ | $OCH_3$ | CH |
| 2-$CO_2CH_3$ | H | H | 3 | O | $CH_3$ | $N(CH_3)_2$ | CH |
| 2-$CO_2CH_3$ | H | H | 3 | O | $CH_3$ | $N(CH_3)_2$ | N |
| 5-$CO_2C_2H_5$ | H | H | 3 | O | $CH_3$ | $OCH_2CH_2OCH_3$ | CH |

TABLE XVI (continued)

| $R_1$ | $R_2$ | $R_4$ | Position $-SO_2N<$ | W | X | Y | Z |
|---|---|---|---|---|---|---|---|
| 5-$CO_2nC_6H_{13}$ | H | $CH_3$ | 3 | O | $CH_3$ | $CH_2OCH_3$ | N |
| 5-$CH_3$ | 6-Cl | H | 3 | S | $CH_3$ | $N(CH_3)_2$ | N |
| 5-$CH_3$ | 6-Cl | $CH_3$ | 3 | O | $CH_3$ | $SC_2H_5$ | CH |
| 5-$CH_3$ | 6-Cl | H | 3 | O | $CH_3$ | $OCH_3$ | N |
| 5-$NO_2$ | 6-Cl | H | 3 | O | $OCH_3$ | $OCH_3$ | N |
| 5-$NO_2$ | 6-Cl | H | 3 | S | $OCH_3$ | $OCH_3$ | CH |
| 5-$NO_2$ | H | $CH_3$ | 3 | O | $CH_3$ | Cl | CH |
| 5-$NO_2$ | H | H | 3 | O | $CH_3$ | $OCH_3$ | N |
| 6-Br | H | H | 3 | O | $CH_3$ | $OCH_2CO_2C_2H_5$ | CH |
| 5-$NO_2$ | 6-Cl | $CH_3$ | 3 | S | $CH_3$ | $S(CH_2)_2OC_2H_5$ | CH |
| 5-F | 6-Cl | H | 3 | S | $CH_3$ | $CF_3$ | CH |
| 5-F | 6-Cl | H | 3 | O | $CH_3$ | $OC_2H_5$ | N |
| 3-Cl | 5-Cl | H | 4 | S | $CH_3$ | $CF_3$ | CH |
| 3-Cl | 5-Cl | $CH_3$ | 4 | O | $CH_3$ | $OCH(CH_3)CO_2C_2H_5$ | N |
| 3-Cl | 5-Cl | H | 4 | O | $OCH_3$ | $OCH_3$ | N |
| 3-Br | H | H | 4 | O | $CH_3$ | $SC_2H_5$ | CH |
| 3-Br | H | $CH_3$ | 4 | O | $CH_3$ | Cl | CH |
| 3-Br | H | H | 4 | O | $CH_3$ | $OCH_2CH_2OCH_3$ | CH |
| 3-Br | H | $CH_3$ | 4 | O | $CH_3$ | $SCH_2CO_2CH_3$ | N |
| 2-$CO_2$-n-$C_4H_9$ | H | H | 4 | O | $OCH_3$ | $OCH_3$ | CH |
| 5-$NO_2$ | 2-Cl | H | 4 | S | $CH_3$ | $OC_2H_5$ | N |
| 5-$NO_2$ | 2-Cl | H | 4 | O | $CH_3$ | $CF_3$ | CH |
| 5-$NO_2$ | 2-Cl | $CH_3$ | 4 | O | $CH_3$ | $OCH_2CF_3$ | N |
| 5-Cl | H | H | 4 | O | $CH_3$ | $OCH_3$ | N |
| 5-Cl | H | H | 4 | O | $OCH_3$ | $OCH_3$ | CH |

## Formulations

Useful formulations of the compounds of Formula I can be prepared in conventional ways. They include dusts, granules, pellets, suspensions, emulsions, wettable powders, emulsifiable concentrates and the like. Many of them can be applied directly. Sprayable formulations can be extended in suitable media and used at spray volumes of from a few liters to several hundred liters per hectare. The formulations, broadly contain about 0.1% to 99% by weight of active ingredient(s) and at least one of a)

about 0.1% to 20% surfactant(s) and b) about 1% to 99.9% solid or liquid diluent(s). More specifically, they will contain these ingredients in the approximate proportions set forth in Table XVII.

TABLE XVII

| | Weight Percent* | | |
|---|---|---|---|
| | Active Ingredient | Diluent(s) | Surfactant(s) |
| Wettable Powders | 20—90 | 0—74 | 1—10 |
| Oil suspensions, Emulsions (including Emulsifiable Concentrates) | 3—50 | 40—95 | 0—15 |
| Aqueous Suspensions | 10—50 | 40—84 | 1—20 |
| Dusts | 1—25 | 70—99 | 0—5 |
| Granules and Pellets | 0.1—95 | 5—99.9 | 0—15 |

*Active ingredients plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation, or by tank mixing.

Some typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates, solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending, and usually, grinding as in a hammer or fluid energy mill. Suspensions are prepared by wet milling (see, for example, Littler, U.S. Patent 3,060,084). Granules and pellets may be made by spraying the active material on preformed granular carriers or by agglomeration techniques. See J. E. Browning, "Agglomeration", *Chemical Engineering,* Dec. 4, 1967, pp. 147ff. and "Perry's Chemical Engineer's Handbook", 4th Ed., McGraw-Hill, New York, 1963, pp. 8—59ff.

For further information regarding the art of formulation, see for example:

H. M. Loux, U.S. Patent 3,235,361, Col. 6, line 16 through Col. 7, line 19 and Examples 10 through 41.

R. W. Luckenbaugh, U.S. Patent 3,309,192, Col. 5, line 43 through Col. 7, line 62 and Examples 8, 12, 15, 39, 41, 52, 53, 58, 132, 138—140, 162—164, 166, 167, and 169—182.

H. Gysin and E. Knusli, U.S. Patent 2,891,855, Col. 3, line 66 through Col. 5, line 17 and Examples 1—4.

G. C. Klingman, "Weed Control as a Science", John Wiley & Sons, Inc., New York, 1961, pp. 81—96.

J. D. Fryer and S. A. Evans, "Weed Control Handbook", 5th Ed., Blackwell Scientific Publications, Oxford, 1968, pp. 101—103.

Unless indicated otherwise, all parts are by weight in the following examples.

Example 13

Wettable Powder

| | |
|---|---|
| 2-Chloro-N-[(4-methoxy-6-methylpyrimidin-2-yl)amino-carbonyl]-3-pyridinesulfonamide | 95% |
| dioctyl sodium sulfosuccinate | 0.1% |
| sodium ligninsulfonate | 1% |
| synthetic fine silica | 3.9% |

The ingredients are blended and ground in a hammer-mill to produce particles almost all of which are below 100 microns in size. That material is sifted through a U.S.S. No. 50 (0.3 mm openings) screen and packaged.

Example 14

Granule

| | |
|---|---|
| wettable powder of Example 13 | 10% |
| attapulgite granules | 90% |
| (U.S.S. #20—40; 0.84—0.42 mm) | |

A slurry of wettable powder containing 50% solids is sprayed onto the surface of attapulgite granules in a double-cone blender. The granules are dried and packaged.

Example 15

Wettable Powder

| | |
|---|---|
| 2-Chloro-N-[(4-methoxy-6-methyl-1,3,5-triazin-2-yl)-aminocarbonyl]-3-pyridinesulfonamide | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended and passed through an air mill to produce an average particle size under 15 microns, reblended, and sifted through a U.S.S. No. 50 sieve (0.3 mm openings) before packaging.

Example 16

Granule

| | |
|---|---|
| wettable powder of Example 15 | 25% |
| gypsum | 64% |
| potassium sulfate | 11% |

The ingredients are blended in a rotating mixer, and water is sprayed onto that blend so as to effect granulation. When most of the granules have reached 1.0 to 0.42 m (U.S.S. #18 to 40 sieves) in size, they are removed, dried, and screened. Oversize material is crushed to produce additional material in the desired range. The resulting granules contain 10% of the active ingredient.

Example 17

Wettable Powder

| | |
|---|---|
| 2-Chloro-N-[(4-methoxy-6-methyl-1,3,5-triazin-2-yl)-aminocarbonyl]-3-pyridinesulfonamide | 65% |
| dodecylphenol polyethylene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying on the solid ingredients in a blender. After grinding in a hammer-mill to produce particles almost all of which are below 100 microns in size, the material is reblended, sifted through a U.S.S. #50 sieve (0.3 mm opening) and packaged.

Example 18

Oil Suspension

| | |
|---|---|
| 2-Chloro-N-[(4-methoxy-6-methyl-1,3,5-triazin-2-yl)-aminocarbonyl]-3-pyridinesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting suspension may be applied directly, but preferably after being extended further with oils or emulsified in water.

Example 19

Extruded Pellet

| | |
|---|---|
| 2-Chloro-N-[(4-methoxy-6-methylpyrimidin-2-yl)-aminocarbonyl]-3-pyridinesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer milled and then moistened with about 12% water. The mixture is extruded in the form of cylinders about 3 mm in diameter which are cut to produce pellets about 3 mm long. The pellets may be used directly, after drying, or dried pellets may be crushed to pass a U.S.S. No. 20 sieve (0.84 mm openings). The granules held on a U.S.S. No. 40 sieve (0.42 mm openings) may be packaged for use and the fines recycled.

Example 20

Solution

| | |
|---|---|
| 2-Chloro-N-[(4-methoxy-6-methyl-1,3,5-triazin-2-yl)-aminocarbonyl]-3-pyridinesulfonamide | 5% |
| dimethylformamide | 95% |

The ingredients are combined and stirred to produce a solution, which can be used for low volume applications.

Example 21

Wettable Powder

| | |
|---|---|
| 2-Chloro-N-[(4-methoxy-6-methylpyrimidin-2-yl)-aminocarbonyl]-3-pyridinesulfonamide | 80% |
| sodium alkylnaphthalenesulfonate | 2% |
| sodium ligninsulfonate | 2% |
| synthetic amorphous silica | 3% |
| kaolinite | 13% |

The ingredients are thoroughly blended. After grinding in a hammer mill to produce particles essentially all of which are under 100 microns in size, the material is reblended, sifted through a U.S.S. No. 50 sieve and packaged.

Utility

The compounds of the present invention are highly active herbicides. They have utility for broadspectrum pre and/or post-emergence weed control in areas where complete control of all vegetation is desired, such as around fuel storage tanks, ammunition depots, industrial storage areas, oil well sites, drive-in theaters, around billboards, highway and railroad structures. By properly selecting rate and time of application, compounds of this invention may be used also to modify plant growth beneficially.

The precise amount of the compound of Formula I to be used in any given situation will vary according to the particular end result desired, the amount of foliage present, the weeds to be controlled, the soil type, the formulation and mode of application, weather conditions, etc. Since so many variables play a role, it is not possible to state a rate of application suitable for all situations. Broadly speaking, the compounds of this invention are used at levels of about 0.005 to 20 kg/ha with a preferred range of 0.125 to 10 kg/ha. In general, the higher rates of application from within this range will be selected for adverse conditions or where extended persistance in soil is desired.

Certain compounds of this invention are useful as selective herbicides in cucumber, wheat, oats, squash, flax, and tomato. The rates employed for selective herbicidal use will vary with the crop, weeds present, soil type, climate, etc. and no one rate can be selected. However, for most uses they will be in the range of .03 to 5 kg/ha.

The compounds of Formula I may be combined with other herbicides and are particularly useful in combination with 3-(3,4-dichlorophenyl)-1,1-dimethyl urea; the triazines such as 2-chloro-4-(ethylamino)-6-(isopropylamino)-*s*-triazine: the uracils such as 5-bromo-3-*sec*-butyl-6-methyluracil; N-(phosphonomethyl)glycine; 3-cyclohexyl-1-methyl-6-dimethylamino-*s*-triazine-2,4-(1H,3H)-dione; N,N-dimethyl-2,2-diphenylacetamide; 2,4-dichlorophenoxyacetic acid (and closely related compounds); 4-chloro-2-butynyl-3-chlorophenylcarbamate; diisopropylthiolcarbamic acid, ester with 2,3-dichloroallyl alcohol; diisopropylthiolcarbamic acid, S-(2,3,3-trichloroallyl)ester; ethyl-N-(benzoyl-N-(3,4-dichlorophenyl)-2-aminopropionate; 1,2-dimethyl-3,5-diphenylpyrazolium methylsulfate; methyl 2-[4-(2,4-dichlorophenoxy)-phenoxy]propanoate; 4-amino-6-*tert*-butyl-3-(methylthio)-1,2,4-triazin-5(4H)-one; 3-(3,4-dichlorophenyl)-1-methoxy-1-methylurea; 3-isopropyl-1H-2,1,3-benzothiodiazin-(4)3H-one-2,2-dioxide; $\alpha,\alpha,\alpha$-trifluoro-2,6-dinitro-N,N-dipropyl-*p*-toluidine; 1,1′-dimethyl-4,4′-bipyridinium ion; monosodium methanearsonate; 2-chloro-2′,6′-diethyl(methoxymethyl)acetanilide; and 1,1-dimethyl-3-($\alpha,\alpha,\alpha$-trifluoro-*m*-tolyl)urea.

The activity of the compounds was discovered in greenhouse tests. The tests are described and the data resulting from them are shown below.

Test Procedure A

Seeds of crabgrass (*Digitaria* spp.), barnyard-grass (*Echinochloa crusgalli*), wild oats (*Avena fatua*), cassia (*Cassia tora*), morningglory (*Ipomoea* spp.), cocklebur (*Xanthium* spp.), sorghum, corn, soybean, rice, wheat and nutsedge tubers (*Cyperus rotundus*) were planted in a growth medium and treated preemergence with a nonphytotoxic solvent solution of the compounds of Table XVIII. At the same time, cotton having five leaves (including cotyledonary ones), bush beans with the third trifoliate leaf expanding, crabgrass with two leaves, barnyardgrass with two leaves, wild oats with two leaves, cassia with three leaves (including cotyledonary ones), morningglory with four leaves (including the cotyledonary ones), cocklebur with four leaves (including the cotyledonary ones), sorghum with four leaves, corn with four leaves, soybean with two cotyledonary leaves, rice with three leaves, wheat with one leaf, and nutsedge with three-five leaves were sprayed with a nonphytotoxic solvent solution of the compounds of Table XVIII. Other containers of the above mentioned weeds and crops were

treated pre- and post-emergence with the same nonphytotoxic solvent so as to provide a solvent control. A set of untreated control plants was also included for comparison. Preemergence and postemergence treated plants and controls were maintained in a greenhouse for sixteen days, then all treated plants were compared with their respective controls and rated visually for response to treatment using the following rating system.

0 = no effect
10 = maximum effect
C = chlorosis or necrosis
D = defoliation
E = emergence inhibition
G = growth retardation
H = formative effects
U = unusual pigmentation
F = flowering response
Y = flowering response
S = albanism

The data in Table XVIII shows that the compounds of this invention are very effective herbicides.

TABLE XVIII

| | | 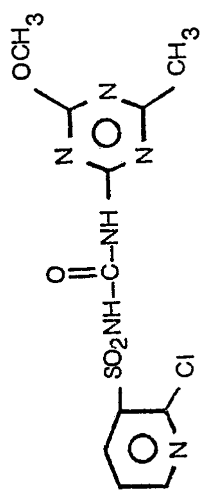 | 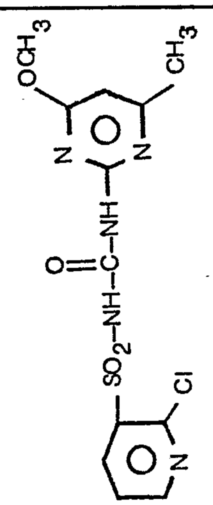 |
|---|---|---|---|
| | *Rate kg/ha* | 0.4 | 0.4 |
| POSTEMERGENCE | BUSH BEAN | 9C | 9C |
| | COTTON | 9C | 9C |
| | MORNINGGLORY | 10C | 10C |
| | COCKLEBUR | 10C | 10C |
| | CASSIA | 9C | 9C |
| | NUTSEDGE | 10C | 10C |
| | CRABGRASS | 9C | 9C |
| | BARNYARDGRASS | 10C | 10C |
| | WILD OATS | 2C 5G | 5C 9G |
| | WHEAT | 5C 7G | 5C 8G |
| | CORN | 10C | 10C |
| | SOYBEAN | 9C | 9C |
| | RICE | 9C | 9C |
| | SORGHUM | 9C | 10C |
| | | | |
| PREEMERGENCE | MORNINGGLORY | 9C | 9G |
| | COCKLEBUR | 9H | 9H |
| | CASSIA | 3C 9G | 5C 9G |
| | NUTSEDGE | 8G | 10E |
| | CRABGRASS | 4C 9G | 5C 9G |
| | BARNYARDGRASS | 10C | 10H |
| | WILD OATS | 9H | 5C 9G |
| | WHEAT | 9H | 10E |
| | CORN | 10E | 10E |
| | SOYBEAN | 9H | 9H |
| | RICE | 10E | 10E |
| | SORGHUM | 10H | 10H |

TABLE XVIII (Continued)

| | | | |
|---|---|---|---|
| | | 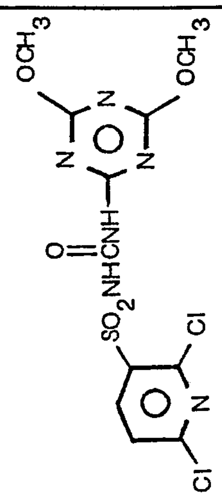 | 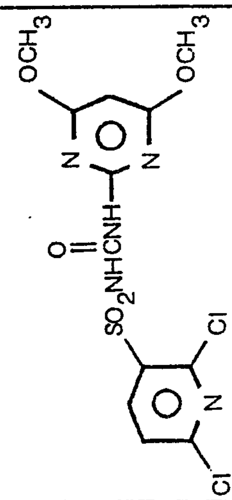 |
| | *Rate kg/ha* | 0.4 | 0.4 |
| POSTEMERGENCE | BUSH BEAN | 9D 2C 9G | 5C 8G 6Y |
| | COTTON | 5C 8G | 4C 8G |
| | MORNINGGLORY | 3C 7G | 1C 6G |
| | COCKLEBUR | 3C | 3C 8G |
| | CASSIA | 3C | 3C 8G |
| | NUTSEDGE | 1C | 9G |
| | CRABGRASS | 2G | 1C 4G |
| | BARNYARDGRASS | 2C 5H | 2C 8H |
| | WILD OATS | 0 | 1C |
| | WHEAT | 0 | 1C |
| | CORN | 7H | 2U 8H |
| | SOYBEAN | 2C 5G | 3C 7G |
| | RICE | 1C 6G | 3C 7G |
| | SORGHAM | 3C | 2C 7G |
| | | | |
| PREEMERGENCE | MORNINGGLORY | 2G | 8G |
| | COCKLEBUR | 1C | 8G |
| | CASSIA | 3C | 8G |
| | NUTSEDGE | 1C 4G | 10E |
| | CRABGRASS | 2G | 1C 5G |
| | BARNYARDGRASS | 1C 6G | 3C 9H |
| | WILD OATS | 0 | 1C 5G |
| | WHEAT | 0 | 7G |
| | CORN | 2C 3G | 9G |
| | SOYBEAN | 2C 3G | 9H |
| | RICE | 3C | 10E |
| | SORGHUM | 3C 6G | 1C 9G |

TABLE XVIII (Continued)

| | | 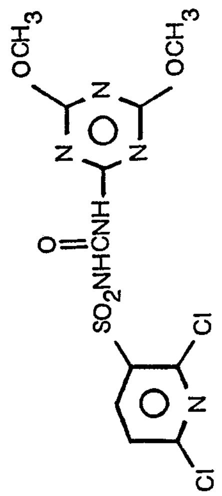 | 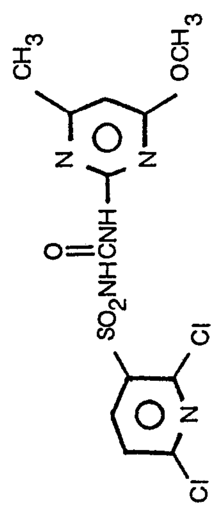 |
|---|---|---|---|
| | *Rate kg/ha* | 0.4 | 0.4 |
| POSTEMERGENCE | BUSH BEAN | 4C 8G 6Y | 9D 9G 6Y |
| | COTTON | 4C 8G | 5C 9G |
| | MORNINGGLORY | 1C | 10C |
| | COCKLEBUR | 1C | 9C |
| | CASSIA | 3C | 3C 7G |
| | NUTSEDGE | 0 | 2C 8G |
| | CRABGRASS | 0 | 1C |
| | BARNYARDGRASS | 2C | 2C 8H |
| | WILD OATS | 0 | 1C 2G |
| | WHEAT | 0 | 3G |
| | CORN | 1C 3H | 2U 8G |
| | SOYBEAN | 2C 5G | 5C 8G |
| | RICE | 1C 6G | 5C 7G |
| | SORGHUM | 1C 3G | 5C 8G |
| | | | |
| PREEMERGENCE | MORNINGGLORY | 2C | 9G |
| | COCKLEBUR | 0 | 8G |
| | CASSIA | 2C | 8G |
| | NUTSEDGE | 1C 5G | 10E |
| | CRABGRASS | 2G | 2G |
| | BARNYARDGRASS | 1C | 2C 8H |
| | WILD OATS | 0 | 1C 5G |
| | WHEAT | 0 | 1C 5G |
| | CORN | 1C 2G | 1C 9G |
| | SOYBEAN | 1C | 2C 5H |
| | RICE | 1C 5G | 9H |
| | SORGHUM | 1C 2G | 1C 9G |

TABLE XVIII (Continued)

| | | | |
|---|---|---|---|
| | | 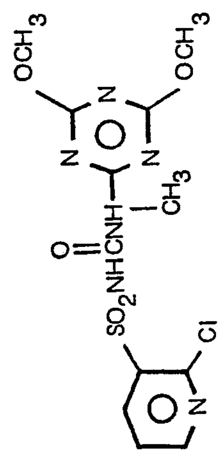 | 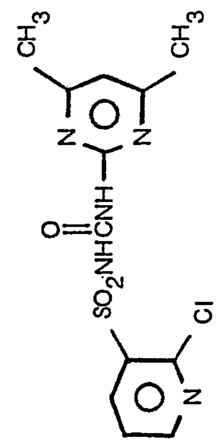 |
| | Rate kg/ha | 0.4 | 2 |
| POSTEMERGENCE | BUSH BEAN | 3S 7G 6Y | 5S 9G 6Y |
| | COTTON | 5C 8G | 2C |
| | MORNINGGLORY | 9C | 2C |
| | COCKLEBUR | 9C | 2C 2H 6F |
| | CASSIA | 2C 8G | 2C 5G |
| | NUTSEDGE | 7G | 0 |
| | CRABGRASS | 2G | 0 |
| | BARNYARDGRASS | 2C 9H | 0 |
| | WILD OATS | 0 | 0 |
| | WHEAT | 0 | 0 |
| | CORN | 5C 9G | 0 |
| | SOYBEAN | 2C 7G | 2G |
| | RICE | 2C 6G | 0 |
| | SORGHUM | 3G | 0 |
| | | | |
| PREEMERGENCE | MORNINGGLORY | 9G | 5H |
| | COCKLEBUR | 3G | 0 |
| | CASSIA | 1C 4G | – |
| | NUTSEDGE | 0 | 0 |
| | CRABGRASS | 0 | 0 |
| | BARNYARDGRASS | 4H | 0 |
| | WILD OATS | 0 | 0 |
| | WHEAT | 0 | 0 |
| | CORN | 1C 3G | 0 |
| | SOYBEAN | 2C | 2G |
| | RICE | 1C 3H | 1C |
| | SORGHUM | 2G | 0 |

TABLE XVIII (Continued)

| | | 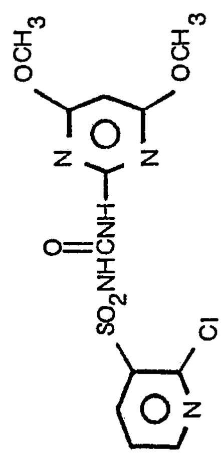 | 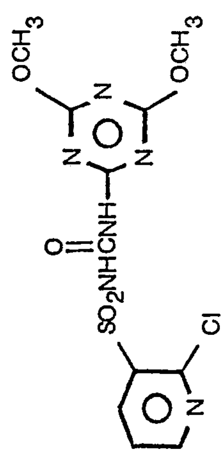 |
|---|---|---|---|
| | *Rate kg/ha* | 0.4 | 0.4 |
| POSTEMERGENCE | BUSH BEAN | 9C | 6C 9G 6Y |
| | COTTON | 9C | 6C 8G |
| | MORNINGGLORY | 9C | 10C |
| | COCKLEBUR | 10C | 10C |
| | CASSIA | 10C | 5C 9G |
| | NUTSEDGE | 9C | 2C 7G |
| | CRABGRASS | 9C | 6G |
| | BARNYARDGRASS | 9C | 5C 9H |
| | WILD OATS | 3C 7G | 2C |
| | WHEAT | 3C 7G | 3C |
| | CORN | 6U 9G | 9U 9C |
| | SOYBEAN | 5C 9G | 5C 9G |
| | RICE | 5C 9G | 6C 9G |
| | SORGHUM | 9C | 2C 8G |
| | | | |
| PREEMERGENCE | MORNINGGLORY | 9G | 9G |
| | COCKLEBUR | 9G | 9G |
| | CASSIA | 9G | 9G |
| | NUTSEDGE | 10E | 4G |
| | CRABGRASS | 9H | 4G |
| | BARNYARDGRASS | 5C 9H | 9H |
| | WILD OATS | 2C 7G | 4G |
| | WHEAT | 9H | 2C 9H |
| | CORN | 10E | 10E |
| | SOYBEAN | 9H | 9H |
| | RICE | 10E | 10E |
| | SORGHUM | 9H | 5C 9H |

TABLE XVIII (Continued)

| | | | |
|---|---|---|---|
| | | 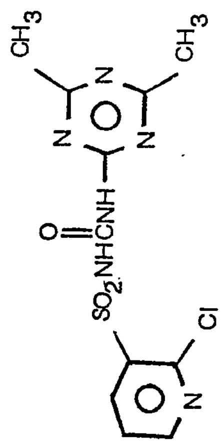 | 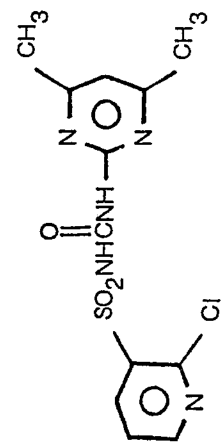 |
| | Rate kg/ha | 0.4 | 0.4 |
| POSTEMERGENCE | BUSH BEAN | 4C 9G 6Y | 5C 9G 6Y |
| | COTTON | 3C 6G | 6C 9G |
| | MORNINGGLORY | 3C 8G | 6C 9G |
| | COCKLEBUR | 2C 8H | 6C 9G |
| | CASSIA | 2C 5G | 5C 8G |
| | NUTSEDGE | 2C 8G | 9C |
| | CRABGRASS | 9C | 3C 8G |
| | BARNYARDGRASS | 4C 9H | 5C 9H |
| | WILD OATS | 2C | 3C 7G |
| | WHEAT | 2C | 8C |
| | CORN | 9C | 6U 9G |
| | SOYBEAN | 4C | 3C 9G |
| | RICE | 4C | 4C 9G |
| | SORGHUM | 4C | 6C 9G |
| | | | |
| PREEMERGENCE | MORNINGGLORY | 6G | 9G |
| | COCKLEBUR | 0 | 9G |
| | CASSIA | 2C 5G | 9G |
| | NUTSEDGE | 0 | 10E |
| | CRABGRASS | 0 | 2C 8G |
| | BARNYARDGRASS | 2C 9H | 5C 9H |
| | WILD OATS | 0 | 2C 6G |
| | WHEAT | 8H | 9H |
| | CORN | 3C 9G | 2C 9G |
| | SOYBEAN | 1C | 8H |
| | RICE | 9H | 10E |
| | SORGHUM | 2C 9G | 4C 9H |

TABLE XVIII (Continued)

| | | | |
|---|---|---|---|
| | | 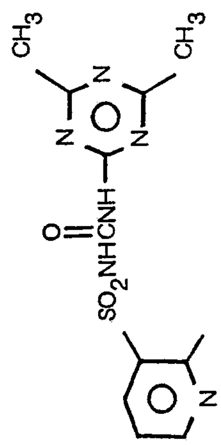 | |
| | Rate kg/ha | 0.4 | |
| POSTEMERGENCE | BUSH BEAN | 3S 5G 6Y | |
| | COTTON | 2C | |
| | MORNINGGLORY | 2C 5G | |
| | COCKLEBUR | 1C | |
| | CASSIA | 1C | |
| | NUTSEDGE | 0 | |
| | CRABGRASS | 3G | |
| | BARNYARDGRASS | 0 | |
| | WILD OATS | 0 | |
| | WHEAT | 0 | |
| | CORN | 0 | |
| | SOYBEAN | 0 | |
| | RICE | 0 | |
| | SORGHUM | 0 | |
| | | | |
| PREEMERGENCE | MORNINGGLORY | 3G | |
| | COCKLEBUR | 0 | |
| | CASSIA | 0 | |
| | NUTSEDGE | 0 | |
| | CRABGRASS | 0 | |
| | BARNYARDGRASS | 0 | |
| | WILD OATS | 0 | |
| | WHEAT | 0 | |
| | CORN | 0 | |
| | SOYBEAN | 0 | |
| | RICE | 0 | |
| | SORGHUM | 0 | |

TABLE XVIII (Continued)

| | | 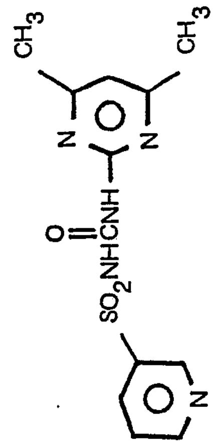 | 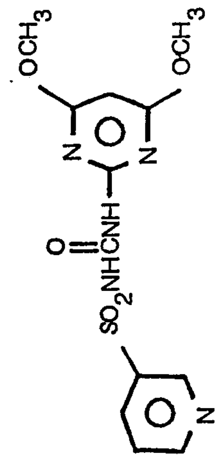 |
|---|---|---|---|
| | *Rate kg/ha* | 2 | 2 |
| POSTEMERGENCE | BUSH BEAN | 1C 3G | 5C 8G 6Y |
| | COTTON | 3C 4G | 5C 9G |
| | MORNINGGLORY | 1C | 9C |
| | COCKLEBUR | 3C 9G | 10C |
| | CASSIA | 2C | 3C 8G |
| | NUTSEDGE | 0 | 5C 9G |
| | CRABGRASS | 0 | 1C 5G |
| | BARNYARDGRASS | 0 | 1C 3G |
| | WILD OATS | 0 | 4G |
| | WHEAT | 0 | 0 |
| | CORN | 1G | 1C 3G |
| | SOYBEAN | 1C | 2C 9G |
| | RICE | 7G | 2C 9G |
| | SORGHUM | 3G | 4G |
| | | | |
| PREEMERGENCE | MORNINGGLORY | 6G | 9G |
| | COCKLEBUR | 8G | 10E |
| | CASSIA | 1C 8G | 1C 9G |
| | NUTSEDGE | 0 | 10E |
| | CRABGRASS | 0 | 5G |
| | BARNYARDGRASS | 0 | 3C 8H |
| | WILD OATS | 4G | 8G |
| | WHEAT | 7G | 8G |
| | CORN | 0 | 9G |
| | SOYBEAN | 2H | 9H |
| | RICE | 2G | 10E |
| | SORGHUM | 0 | 2C 9H |

TABLE XVIII (Continued)

| | | 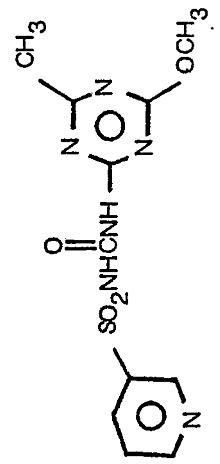 | 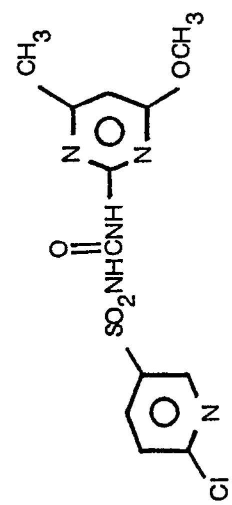 |
|---|---|---|---|
| | *Rate kg/ha* | 2 | 2 |
| POSTEMERGENCE | BUSH BEAN | 4C 7G 6Y | 4C 6G 6Y |
| | COTTON | 2C 4H | 5C 9G |
| | MORNINGGLORY | 0 | 5C 9G |
| | COCKLEBUR | 3C 9G | 3C 9G |
| | CASSIA | 1C | 0 |
| | NUTSEDGE | 1C 5G | 0 |
| | CRABGRASS | 0 | 0 |
| | BARNYARDGRASS | 0 | 0 |
| | WILD OATS | 0 | 0 |
| | WHEAT | 0 | 0 |
| | CORN | 2G | 0 |
| | SOYBEAN | 3C 7G | 2C 3H |
| | RICE | – | 0 |
| | SORGHUM | 2G | 0 |
| | | | |
| PREEMERGENCE | MORNINGGLORY | 8G | 7G |
| | COCKLEBUR | 1C | 7G |
| | CASSIA | 2C 7G | 2C |
| | NUTSEDGE | 0 | 4G |
| | CRABGRASS | 0 | 2G |
| | BARNYARDGRASS | 1C | 2G |
| | WILD OATS | 0 | 0 |
| | WHEAT | 0 | 0 |
| | CORN | 2C 7G | 5G |
| | SOYBEAN | 2C | 3C |
| | RICE | 1C | 8G |
| | SORGHUM | 1C | 0 |

TABLE XVIII (Continued)

| | | 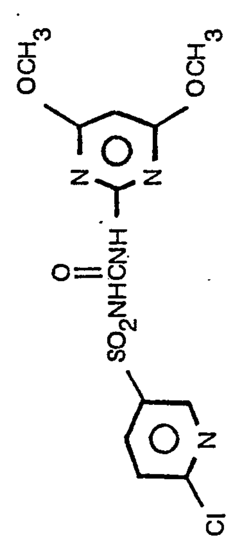 | 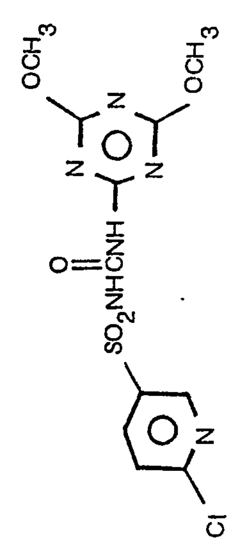 |
|---|---|---|---|
| | *Rate kg/ha* | 2 | 2 |
| POSTEMERGENCE | BUSH BEAN | 3C 7G 6Y | 2G |
| | COTTON | 4C 9G | 0 |
| | MORNINGGLORY | 3C 7H | 0 |
| | COCKLEBUR | 2C 8G | 0 |
| | CASSIA | 2C | 0 |
| | NUTSEDGE | 7G | 0 |
| | CRABGRASS | 0 | 0 |
| | BARNYARDGRASS | 0 | 0 |
| | WILD OATS | 0 | 0 |
| | WHEAT | 0 | 0 |
| | CORN | 0 | 0 |
| | SOYBEAN | 3C 7G | 0 |
| | RICE | 7G | 0 |
| | SORGHUM | 0 | 0 |
| | | | |
| PREEMERGENCE | MORNINGGLORY | 9G | 0 |
| | COCKLEBUR | 9H | 0 |
| | CASSIA | 2C 5G | 0 |
| | NUTSEDGE | 10E | 0 |
| | CRABGRASS | 0 | 0 |
| | BARNYARDGRASS | 2C 4H | 0 |
| | WILD OATS | 1C | 0 |
| | WHEAT | 1C | 0 |
| | CORN | 1C 8G | 0 |
| | SOYBEAN | 1C 2G | 0 |
| | RICE | 9H | 0 |
| | SORGHUM | 2G | 0 |

TABLE XVIII (Continued)

| | | 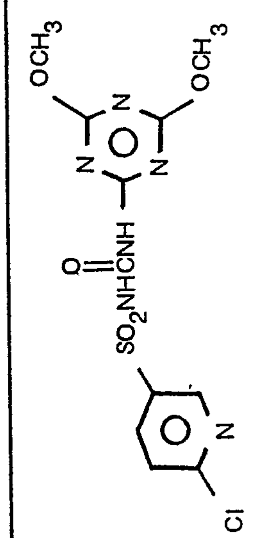 | 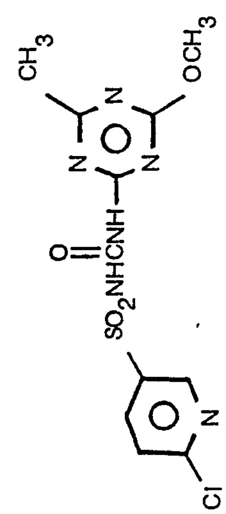 |
|---|---|---|---|
| | *Rate kg/ha* | 2 | 2 |
| POSTEMERGENCE | BUSH BEAN | 2C | 2C 3H |
| | COTTON | 2C 2H | 2C 3H |
| | MORNINGGLORY | 0 | 2C 6H |
| | COCKLEBUR | 0 | 7H |
| | CASSIA | 0 | 1C |
| | NUTSEDGE | 0 | 0 |
| | CRABGRASS | 0 | 0 |
| | BARNYARDGRASS | 0 | 0 |
| | WILD OATS | 0 | 0 |
| | WHEAT | 0 | 0 |
| | CORN | 0 | 0 |
| | SOYBEAN | 0 | 0 |
| | RICE | 0 | 0 |
| | SORGHUM | 0 | 0 |
| | | | |
| PREEMERGENCE | MORNINGGLORY | 0 | 6H |
| | COCKLEBUR | 0 | 2H |
| | CASSIA | 0 | 0 |
| | NUTSEDGE | 0 | 0 |
| | CRABGRASS | 0 | 0 |
| | BARNYARDGRASS | 0 | 0 |
| | WILD OATS | 0 | 0 |
| | WHEAT | 0 | 0 |
| | CORN | 0 | 2C 2G |
| | SOYBEAN | 0 | 1C |
| | RICE | 0 | 5G |
| | SORGHUM | 0 | 0 |

Test B

Two bulb pans were filled with fertilized and limed Fallsington silt loam soil.One pan was planted with seeds of corn, sorghum, Kentucky bluegrass and several grassy weeds. The other pan was planted with seeds of soybeans, purple nutsedge tubers (*Cyperus rotundus*), and seeds of several broadleaf weeds. Seeds of the following grassy and broadleaf weeds were planted: crabgrass (*Digitaria sanguinalis*), barnyardgrass (*Echinochloa crusgalli*), wild oats (*Avena fatua*), johnsongrass (*Sorghum halepense*), giant foxtail (*Setaria faberii*), dallisgrass (*Paspalum dilatatum*), cheatgrass (*Bromus secalinus*), mustard (*Brassica arvensis*), cocklebur (*Xanthium pennsylvanicum*), pigweed (*Amaranthus retroflexus*) morningglory (*Ipomoea hederacea*), cassia (*Cassia tora*), teaweed (*Sida spinosa*), velvetleaf (*Abutilon theophrasti*), and jimsonweed (*Datura stramonium*). A smaller pot was also filled with prepared soil and planted with rice and wheat seeds. Another small pot was planted with seeds of sugarbeets. The above four containers were treated preemergence with nonphytotoxic solvent solutions of the compound shown (i.e., solutions of said compound were sprayed on the soil surface before seed germination). Duplicates of the above-described seeded containers were prepared without treatment and used as controls.

Twenty-eight days after treatment, the treated and control plants were evaluated and the data recorded as set forth in Table XIX.

TABLE XIX

SECONDARY PRE-EMERGENCE
ON FALLSINGTON SILT LOAM

| Compound | 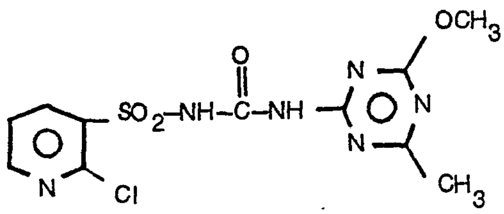 | |
|---|---|---|
| *Rate, kg/ha* | 0.06 | 0.25 |
| CRABGRASS | 7G | 10C |
| BARNYARDGRASS | 9G, 9C | 10C |
| SORGHUM | 10C | 10E |
| WILD OATS | 0 | 4G |
| JOHNSONGRASS | 9G, 9C | 10C |
| DALLISGRASS | 5G | 7G |
| GIANT FOXTAIL | 10C | 10C |
| KY. BLUEGRASS | 10E | 10E |
| CHEATGRASS | 8G, 9C | 10C |
| SUGARBEETS | 10C | 10C |
| CORN | 10C | 10E |
| MUSTARD | 10C | 10C |
| COCKLEBUR | 8G, 5H | 8G, 9H |
| PIGWEED | 10E | 10E |
| NUTSEDGE | 5G | 7G |
| COTTON | 8G | 8G |
| MORNINGGLORY | 8G, 7C | 10C |
| CASSIA | 8G, 8C | 8G, 9C |
| TEAWEED | 10C | 10C |
| VELVETLEAF | 8G, 8C | 10C |
| JIMSONWEED | 7G | 8G, 9C |
| SOYBEAN | 10E | 10E |
| RICE | 4G | 7G, 3C |
| WHEAT | | |

TABLE XIX (Continued)

SECONDARY PRE-EMERGENCE
ON FALLSINGTON SILT LOAM

| Compound | 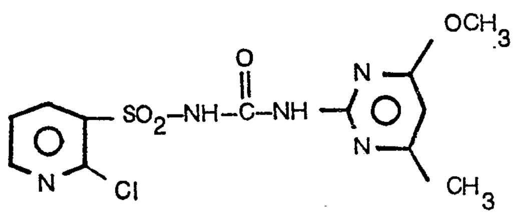 | |
|---|---|---|
| *Rate, kg/ha* | 0.06 | 0.25 |
| CRABGRASS | 9G, 6C | 10C |
| BARNYARDGRASS | 10C | 10C |
| SORGHUM | 10C | 10E |
| WILD OATS | 7G, 3C | 10C |
| JOHNSONGRASS | 9G, 9C | 10C |
| DALLISGRASS | 9G, 5C | 10C |
| GIANT FOXTAIL | 10C | 10C |
| KY. BLUEGRASS | 10E | 10E |
| CHEATGRASS | 10E | 10E |
| SUGARBEETS | 10C | 10C |
| CORN | 9G, 8C | 10E |
| MUSTARD | 10C | 10C |
| COCKLEBUR | 8G, 5H | 8G, 8H |
| PIGWEED | 10E | 10E |
| NUTSEDGE | 8G | 10E |
| COTTON | 8G | 8G |
| MORNINGGLORY | 8G, 8C | 8G, 8C |
| CASSIA | 8G, 8C | 8G, 8C |
| TEAWEED | 10C | 10C |
| VELVETLEAF | 10C | 10C |
| JIMSONWEED | 8G, 8C | 8G, 9C |
| SOYBEAN | 8G, 8C | 8G, 9C |
| RICE | 10E | 10E |
| WHEAT | 10C | 10C |

Test C

Two compounds, 2 - chloro - N - [(4 - methoxy - 6 - methyl - 1,3,5 - triazin - 2 - yl)aminocarbonyl - 3 - pyridinesulfonamide, (Compound A) and 2 - chloro - N - [(4 - methoxy - 6 - methylpyridin - 2 - yl)aminocarbonyl], (Compound B), were applied pre- and post-emergence to a number of crops and weeds. The post-emergence treatment was applied 20 days after planting the crops. The following data show that one or the other of the two compounds exhibits pre- and/or post-emergence selectivity on cucumber, squash, flax, wheat, oats and tomatoe.

TABLE XX

| | | Pre-Emergence % Control Overall | | | |
|---|---|---|---|---|---|
| | | Grasses | | Broadleaves | |
| Materials | Rate, Active kg/ha | 5 Weeks | 11 Weeks | 5 Weeks | 11 Weeks |
| Comp. A | .015 | 50 | 0 | 65 | 50 |
| | .031 | 70 | 20 | 92 | 80 |
| | .063 | 85 | 30 | 98 | 80 |
| Comp. B | .015 | 98 | 80 | 100 | 99 |
| | .031 | 100 | 80 | 100 | 98 |

| | | Pre-Emergence % Control — 5 Weeks | | | | | |
|---|---|---|---|---|---|---|---|
| Materials | Rate, Active kg/ha | Flax | Tomato | Cucumber | Squash | Oats | Wheat |
| Comp. A | .015 | 0 | 20 | 0 | 20 | 0 | 0 |
| | .031 | 0 | 30 | 30 | 30 | 0 | 0 |
| | .063 | 20 | 20 | 25 | 70 | 15 | 10 |
| Comp. B | .015 | 0 | 30 | 0 | 20 | 70 | 30 |
| | .031 | 15 | 50 | 25 | 20 | 72 | 55 |

| | | Post-Emergence % Control Overall | | | |
|---|---|---|---|---|---|
| | | Grasses | | Broadleaves | |
| Materials | Rate, Active kg/ha | 5 Weeks | 11 Weeks | 5 Weeks | 11 Weeks |
| Comp. A | .015 | 80 | 50 | 90 | 50 |
| | .031 | 85 | 90 | 94 | 70 |
| | .063 | 98 | 98 | 98 | 95 |
| Comp. B | .015 | 92 | 95 | 98 | 40 |
| | 031 | 98 | 98 | 98 | 75 |
| | .063 | 99 | 99 | 99 | 90 |

TABLE XX (continued)

| Materials | Rate, Active kg/ha | Post-Emergence | | | | | |
|---|---|---|---|---|---|---|---|
| | | % Control — 5 Weeks | | | | | |
| | | Flax | Tomato | Cucumber | Squash | Oats | Wheat |
| Comp. A | .015 | 0 | 80 | 0 | 70 | 0 | 0 |
| | .031 | 0 | 80 | 30 | 95 | 0 | 0 |
| | .063 | 0 | 98 | 70 | 95 | 0 | 0 |
| Comp. B | .015 | 30 | 0 | 0 | 55 | 60 | 20 |
| | .031 | 40 | 0 | 0 | 40 | 70 | 50 |
| | .063 | 60 | 0 | 30 | 60 | 90 | 70 |

**Claims**

1. A compound of the formula:

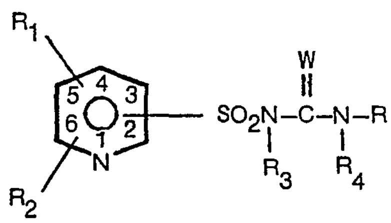

wherein

R is 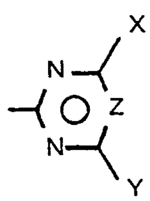 , 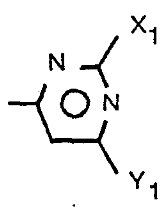 , or 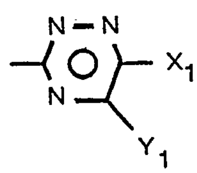

(a) (b) (c)

$R_1$ is H, Cl, Br, F, $C_1$—$C_4$ alkyl, $C_1$—$C_4$ alkoxy, $C_1$—$C_4$ alkylthio, $NO_2$ or $\overset{O}{\overset{\|}{C}}OR_5$;
$R_2$ is H, Cl, Br or $CH_3$;
$R_3$ and $R_4$ are independently H or $CH_3$;
$R_5$ is $C_1$—$C_6$ alkyl, $C_3$—$C_6$ alkenyl, $CH_2CH_2OCH_3$, $CH_2CH_2OCH_2CH_3$, $CH_2CH_2CH_2OCH_3$ or $CH_2CH_2Cl$;
W is oxygen or sulfur;
X is $CH_3$, $CH_3O$— or $CH_3CH_2O$—;
Y is H, Cl, $CH_3$, $CF_3$, $NHCH_3$, —$N(CH_3)_2$, —$CH_2OR_{10}$, —$CH_2CH_2OR_{10}$, —$OCH_2CF_3$ or $VR_9$;
Z is CH or N;
V is oxygen or sulfur;
$R_9$ is $CH_3$, —$CH_2CH_3$, —$CH_2CH_2OR_{10}$, —$CH_2CO_2R_{10}$, —$\underset{CH_3}{\underset{|}{C}}HCO_2R_{10}$, or —$CH_2CH_2CO_2R_{10}$;
$R_{10}$ is $CH_3$ or $CH_3CH_2$;
$Y_1$ is H, $OCH_3$ or $CH_3$;
$X_1$ is H, Cl, $OCH_3$, $OCH_2CH_3$ or $CH_3$
and agriculturally suitable salts thereof; providing that 1) both $X_1$ and $Y_1$ are not simultaneously H and 2) when R is (b) or (c), then $R_3$ and $R_4$ are both H.

2. A compound of Claim 1 wherein $R_3$ and $R_4$ are hydrogen and W is oxygen.

3. A compound of Claim 2 wherein $R_2$ is hydrogen.

4. A compound of Claim 3 wherein $R_1$ is chlorine.

5. A compound of Claim 3 that is a 2- or 4-chloro-3-pyridyl sulfonyl compound.

6. A compound of Claims 1—5 wherein R is

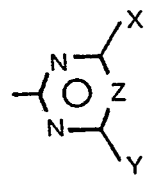 .

7. A compound of Claim 6 wherein X is $CH_3$ or $—OCH_3$ and Y is $CH_3$, $—OCH_3$ or $—OCH_2CH_3$.

8. A compound of Claim 6 wherein Z is CH.

9. A compound of Claim 1 which is 2-chloro-N-[(4-methoxy-6-methyl-1,3,5-triazin-2-yl)aminocarbonyl]-3-pyridinesulfonamide, and agriculturally suitable salts thereof.

10. A compound of Claim 1 which is 2-chloro-N-[(4-methoxy-6-methylpyrimidin-2-yl)aminocarbonyl]-3-pyridinesulfonamide, and agriculturally suitable salts thereof.

11. A compound of Claim 1 which is 2-chloro-N-[4,6-dimethylpyrimidin-2-yl)aminocarbonyl]-3-pyridinesulfonamide, and agriculturally suitable salts thereof.

12. A compound of Claim 1 which is 2-chloro-N-[(4,6-dimethoxypyrimidin-2-yl)aminocarbonyl]-3-pyridinesulfonamide, and agriculturally suitable salts thereof.

13. A compound of Claim 1 which is 2-chloro-N-[(4,6-dimethyl-1,3,5-triazin-2-yl)aminocarbonyl]-3-pyridinesulfonamide, and agriculturally suitable salts thereof.

14. A compound of Claim 1 which is 2-chloro-N-[4,6-dimethoxy-1,3,5-triazin-2-yl)aminocarbonyl]-3-pyridinesulfonamide, and agriculturally suitable salts.

15. A composition for the control of undesirable vegetation comprising a herbicidal compound and at least one of (a) a surface-active agent, and (b) a solid or liquid diluent, characterised in that said herbicidal compound is a compound of any of Claims 1—14.

16. A method for the control of undesirable vegetation by applying to the locus of such undesirable vegetation an effective amount of a herbicidal compound, characterised in that said herbicidal compound comprises a compound of any of Claims 1—14.

17. A method for modifying the growth of plants by applying to the locus of such plants an effective amount of a plant growth regulant, characterised in that said plant growth regulant comprises a compound of any of Claims 1—14.

18. A process for making a compound of Claim 1 which comprises reacting together compounds of formulae

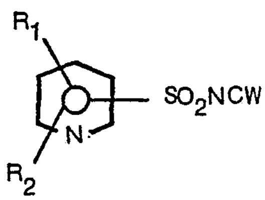 and 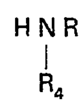

wherein

R, $R_1$, $R_2$, $R_4$ and W are as defined in Claim 1, to obtain a corresponding compound of Claim 1 wherein $R_3$ is H.

19. A process for making a compound of Claim 1 which comprises reacting together compounds of formulae

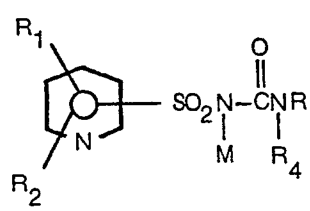 and $(R_3)_nX$

wherein

R, $R_1$, $R_2$ and $R_4$ are as defined in Claim 1, $R_3$ is methyl, M is an alkali metal cation, X is an incipient anion and n is an integer corresponding to the valence of X, to obtain a corresponding compound of claim 1 wherein $R_3$ is methyl and W is O.

20. A process for making a compound of Claim 1 which comprises reacting together compounds of formulae

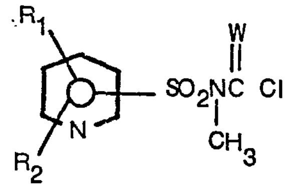 and 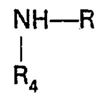

wherein

R, $R_1$, $R_2$, $R_4$ and W are as defined in Claim 1, to obtain a compound of Claim 1 wherein $R_3$ is $CH_3$.

21. A process for making a compound of Claim 1 which comprises oxidising e.g. with mercuric oxide/boron trifluoride etherate or mercuric chloride/calcium carbonate, a compound of general formula

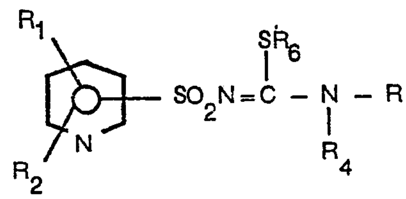

wherein

R, $R_1$, $R_2$ and $R_4$ are as defined in Claim 1 and $R_6$ is $C_1$—$C_5$ alkyl, to obtain a corresponding compound of Claim 1 wherein $R_3$ is H and W is O.

22. A process for making a compound of Claim 1 which comprises reacting together compounds general formula

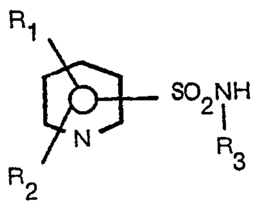 and S C N—R

wherein

R, $R_1$, $R_2$ and $R_3$ are as defined in Claim 1 to obtain a compound of Claim 1 wherein $R_4$ is H and W is S.

23. A process for making a compound of Claim 1 wherein W is O which comprises reacting a compound of Claim 1 wherein W is S with mercuric oxide.

**Revendications**

1. Un composé de la formule:

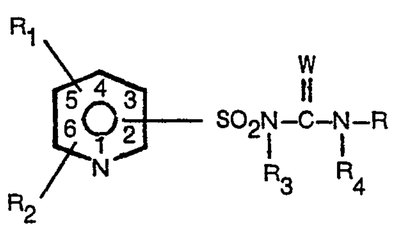 dans laquelle

R est 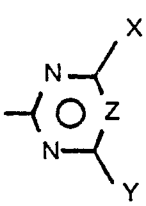, 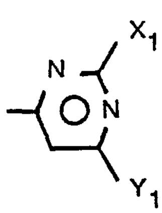, ou 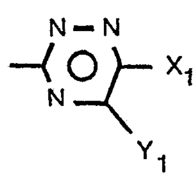

(a) (b) (c)

$R_1$ est H, Cl, Br, F, un groupe alcoyle en $C_1$—$C_4$, alcoxy en $C_1$—$C_4$, alcoylthio en $C_1$—$C_4$, $NO_2$ ou

$$\overset{O}{\overset{\|}{C}}OR_5;$$

$R_2$ est H, Cl, Br ou $CH_3$;
$R_3$ et $R_4$ sont indépendamment H ou $CH_3$;
$R_5$ est un groupe alcoyle en $C_1$—$C_6$, alcényle en $C_3$—$C_6$, $CH_2CH_2OCH_3$, $CH_2CH_2OCH_2CH_3$, $CH_2CH_2CH_2OCH_3$ ou $CH_2CH_2Cl$;
W est de l'oxygène ou du soufre;
X est $CH_3$, $CH_3O$— ou $CH_3CH_2O$;
Y est H, Cl, $CH_3$, $CF_3$, $NHCH_3$, —$N(CH_3)_2$, —$CH_2OR_{10}$, —$CH_2CH_2OR_{10}$, —$OCH_2CH_3$ ou $VR_9$;
Z est CH ou N;
V est de l'oxygène ou du soufre;
$R_9$ est $CH_3$, —$CH_2CH_3$, —$CH_2CH_2OR_{10}$, —$CH_2CO_2R_{10}$, $-\underset{CH_3}{\underset{|}{C}}HCO_2R_{10}$ ou —$CH_2CH_2CO_2R_{10}$,
$R_{10}$ est $CH_3$ ou $CH_3CH_2$;
$Y_1$ est H, $OCH_3$ ou $CH_3$;
$X_1$ est H, Cl, $OCH_3$, $OCH_2CH_3$ ou $CH_3$
et ses sels utilisables en agriculture; avec les conditions que 1) $X_1$ et $Y_1$ ne sont pas tous deux simultanément H et 2) quand R est (b) ou (c), alors $R_3$ et $R_4$ sont tous deux H.

2. Un composé selon la revendication 1, dans lequel $R_3$ et $R_4$ sont de l'hydrogène et W est de l'oxygène.

3. Un composé selon la revendication 2, dans lequel $R_2$ est de l'hydrogène.

4. Un composé selon la revendication 3, dans lequel $R_1$ est du chlore.

5. Un composé selon la revendication 3, qui est un composé 2- ou 4-chloro-3-pyridyl sulfonyle.

6. Un composé selon l'une des revendications 1 à 5, dans lequel R est

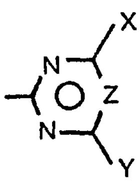

7. Un composé selon la revendication 6, dans lequel X est $CH_3$ ou —$OCH_3$ et Y est $CH_3$, —$OCH_3$ ou —$OCH_2CH_3$.

8. Un composé selon la revendication 6, dans lequel Z est H.

9. Un composé selon la revendication 1, qui est le 2-chloro-N-[(4-méthoxy-6-méthyl-1,3,5-triazin-2-yl)aminocarbonyl]-3-pyridinesulfonamide, et ses sels utilisables en agriculture.

10. Un composé selon la revendication 1, qui est le 2-chloro-N-[(4-méthoxy-6-méthylpyrimidin-2-yl)aminocarbonyl]-3-pyridinesulfonamide, et ses sels utilisables en agriculture.

11. Un composé selon la revendication 1, qui est le 2-chloro-N-[4,6-diméthylpyrimidin-2-yl)aminocarbonyl]-3-pyridinesulfonamide, et ses sels utilisables en agriculture.

12. Un composé selon la revendication 1, qui est le 2-chloro-N-[(4,6-diméthoxypyrimidine-2-yl)aminocarbonyl]-3-pyridinesulfonamide, et ses sels utilisables en agriculture.

13. Un composé selon la revendication 1, qui est le 2-chloro-N-[(4,6-diméthyl-1,3,5-triazin-2-yl)aminocarbonyl]-3-pyridinesulfonamide, et ses sels utilisables en agriculture.

14. Un composé selon la revendication 1, qui est le 2-chloro-N-[4,6-diméthoxy-1,3,5-triazin-2-yl)aminocarbonyl]-3-pyridinesulfonamide, et ses sels utilisables en agriculture.

15. Une composition pour lutter contre la végétation indésirable comprenant un composé herbicide et au moins un ingrédient choisi parmi (a) un agent tensio-actif et (b) un diluant solide ou liquide, caractérisé en ce que le composé herbicide est un composé selon l'une quelconque des revendications 1 à 14.

16. Un procédé de lutte contre la végétation indésirable en appliquant à l'endroit où se trouve cette végétation une quantité efficace d'un composé herbicide, caractérisé en ce que le composé herbicide comprend un composé selon l'une quelconque des revendications 1 à 14.

17. Un procédé pour modifier la croissance de plantes en appliquant à l'endroit où se trouve ces plantes une quantité efficace d'une agent de régulation de la croissance des plantes, caractérisé en ce que cet agent de régulation de la croissance des plantes comprend un composé selon l'une quelconque des revendications 1 à 14.

18. Un procédé de préparation d'un composé de la revendication 1, selon lequel on fait réagir ensemble des composés des formules

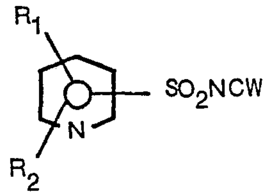 et 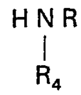

dans lesquelles R, $R_1$, $R_2$, $R_4$ et W sont tels que définis dans la revendication 1, pour obtenir un composé correspondant de la revendication 1 dans lequel $R_3$ est H.

19. Un procédé de préparation d'une composé de la revendication 1, selon lequel on fait réagir ensemble des composés de formules

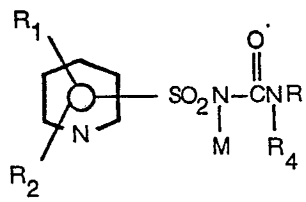 et $(R_3)_nX$

dans lesquelles R, $R_1$, $R_2$ et $R_3$ sont tels que définis dans la revendication 1, $R_3$ est un groupe méthyle, M est un cation de métal alcalin, X est un anion naissant et n est un nombre entier correspondant à la valence de X, pour obtenir un composé correspondant de la revendication 1 dans lequel $R_3$ est un groupe méthyle et W est O.

20. Un procédé de préparation d'un composé de la revendication 1, selon lequel on fait réagir ensemble des composés des formules

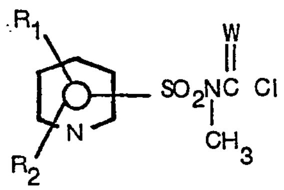 et 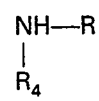

dans lesquelles R, $R_1$, $R_2$, $R_4$ et W sont tels que définis dans la revendication 1, pour obtenir un composé de la revendication 1 dans lequel $R_3$ est $CH_3$.

21. Un procédé de préparation d'une composé de la revendication 1, selon lequel on oxyde, par exemple avec une combinaison oxyde mercurique éthérate de trifluorure de bore ou chlorure mercurique/carbonate de calcium, un composé de formule générale

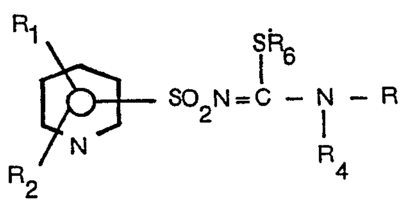

dans laquelle R, $R_1$, $R_2$ et $R_4$ sont tels que définis dans la revendication 1 et $R_6$ est un groupe alcoyle en $C_1$—$C_5$, pour obtenir un composé correspondant de la revendication 1 dans lequel $R_3$ est H et W est O.

22. Un procédé de préparation d'un composé de la revendication 1, selon lequel on fait réagir ensemble des composés des formules générales

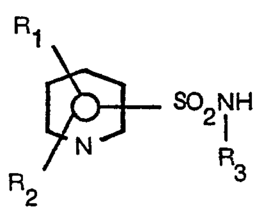 et $SCN-R$

dans lesquelles R, $R_1$, $R_2$ et $R_3$ sont tels que définis dans la revendication 1, pour obtenir un composé de la revendication 1 dans lequel $R_4$ est H et W est S.

23. Un procédé de préparation d'un composé de la revendication 1 dans lequel W est O, selon lequel on fait réagir un composé de la revendication 1 dans lequel W est S avec de l'oxyde mercurique.

**Patentansprüche**

1. Verbindung der Formel

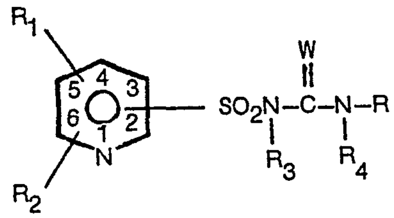

in der

R 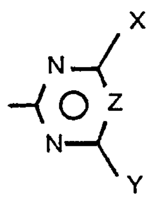 , 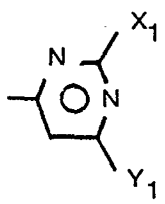 , oder 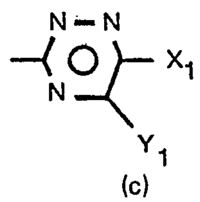

(a) (b) (c)

bedeutet,

$R_1$ H, Cl, Br, F, $C_1$—$C_4$-Alkyl, $C_1$—$C_4$-Alkoxy, $C_1$—$C_4$-Alkylthio, $NO_2$ oder $\overset{\displaystyle O}{\overset{\|}{C}}OR_5$ bedeutet,

$R_2$ H, Cl, Br oder $CH_3$ bedeutet,

$R_3$ und $R_4$ unabhängig voneinander H oder $CH_3$ bedeuten,

$R_5$ $C_1$—$C_6$-Alkyl, $C_3$—$C_6$-Alkenyl, $CH_2CH_2OCH_3$, $CH_2CH_2OCH_2CH_3$, $CH_2CH_2CH_2OCH_3$ oder $CH_2CH_2Cl$ bedeuten,

W Sauerstoff oder Schwefel bedeutet,

X $CH_3$, $CH_3O$— oder $CH_3CH_2O$— bedeutet,

Y H, Cl, $CH_3CF_3$, $NHCH_3$, —$N(CH_3)_2$, —$CH_2OR_{10}$, —$CH_2CH_2OR_{10}$, —$OCH_2CF_3$ oder $VR_9$ bedeutet,

Z CH oder N bedeutet,

V Sauerstoff oder Schwefel bedeutet,

$R_9$ $CH_3$, —$CH_2CH_3$, —$CH_2CH_2OR_{10}$, —$CH_2CO_2R_{10}$, —$\underset{CH_3}{\underset{|}{C}H}CO_2R_{10}$ oder $CH_2CH_2CO_2R_{10}$ bedeutet,

$R_{10}$ $CH_3$ oder $CH_3CH_2$ bedeutet,

$Y_1$ H, $OCH_3$ oder $CH_3$ bedeutet,

$X_1$ H, Cl, $OCH_3$, $OCH_2CH_3$ oder $CH_3$ bedeutet,

sowie für landwirtschaftliche Zwecke geeignete Salze davon, mit der Massgabe, dass (1) $X_1$ und $Y_1$ nicht gleichzeitig H bedeuten und, (2) wenn R (b) oder (c) ist, $R_3$ und $R_4$ beide H sind.

2. Verbindung nach Anspruch 1, dadurch gekennzeichnet, dass $R_3$ und $R_4$ Wasserstoff und W Sauerstoff bedeuten.

3. Verbindungen nach Anspruch 2, dadurch gekennzeichnet, dass $R_2$ Wasserstoff bedeutet.

4. Verbindungen nach Anspruch 3, dadurch gekennzeichnet, dass $R_1$ Chlor bedeutet.

5. Verbindung nach Anspruch 3, nämlich 2- oder 4-Chlor-3-pyridylsulfonylverbindungen.

6. Verbindungen nach Anspruch 1 bis 5, dadurch gekennzeichnet, dass R

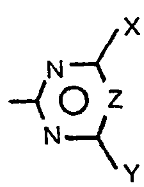

bedeutet.

7. Verbindungen nach Anspruch 6, dadurch gekennzeichnet, dass X $CH_3$ oder —$OCH_3$ und Y $CH_3$, —$OCH_3$ oder —$OCH_2CH_3$ bedeutet.

8. Verbindungen nach Anspruch 6, dadurch gekennzeichnet, dass Z CH bedeutet.

9. Verbindung nach Anspruch 1, nämlich 2-Chlor-N-[(4-methoxy-6-methyl-1,3,5-triazin-2-yl)-aminocarbonyl]-3-pyridinsulfonamid sowie für landwirtschaftliche Zwecke geeignete Salze davon.

10. Verbindung nach Anspruch 1, nämlich 2-Chlor-N-[(4-methoxy-6-methylpyrimidin-2-yl)-aminocarbonyl]-3-pyridinsulfonamid sowie für landwirtschaftliche Zwecke geeignete Salze davon.

11. Verbindung nach Anspruch 1, nämlich 2-Chlor-N-[(4,6-dimethylpyrimidin-2-yl)-aminocarbonyl]-3-pyridinsulfonamid sowie für landwirtschaftliche Zwecke geeignete Salze davon.

12. Verbindung nach Anspruch 1, nämlich 2-Chlor-N-[(4,6-dimethoxypyrimidin-2-yl)-aminocarbonyl]-3-pyridinsulfonamid sowie für landwirtschaftliche Zwecke geeignete Salze davon.

13. Verbindung nach Anspruch 1, nämlich 2-Chlor-N-[(4,6-dimethyl-1,3,5-triazin-2-yl)-aminocarbonyl]-3-pyridinsulfonamid sowie für landwirtschaftliche Zwecke geeignete Salze davon.

14. Verbindung nach Anspruch 1, nämlich 2-Chlor-N-[(4,6-dimethoxy-1,3,5-triazin-2-yl)-aminocarbonyl]-3-pyridinsulfonamid sowie für landwirtschaftliche Zwecke geeignete Salze davon.

15. Zusammensetzung zur Kontrolle von unerwünschter Vegetation, enthaltend eine herbizide Verbindung und mindestens (a) ein oberflächenaktives Mittel und (b) ein festes oder flüssige Verdünnungsmittel, dadurch gekennzeichnet, dass es sich bei der herbiziden Verbindung um eine Verbindung nach einem der Ansprüche 1 bis 14 handelt.

16. Verfahren zur Kontrolle von unerwünschter Vegetation durch Aufbringen einer wirksamen Menge einer herbiziden Verbindung auf die Stelle der unerwünschten Vegetation, dadurch gekennzeichnet, dass man als herbizide Verbindung eine Verbindung nach einem der Ansprüche 1 bis 14 verwendet.

17. Verfahren zum Modifizieren des Wachstums von Pflanzen durch Aufbringen einer wirksamen Menge eines Pflanzenwuchsreglers auf den Ort des Pflanzenwachstums, dadurch gekennzeichnet, dass man als Pflanzenwuchsregler eine Verbindung nach einem der Ansprüche 1 bis 14 verwendet.

18. Verfahren zur Herstellung von Verbindungen nach Anspruch 1, dadurch gekennzeichnet, dass man Verbindungen der folgenden Formeln miteinander umsetzt

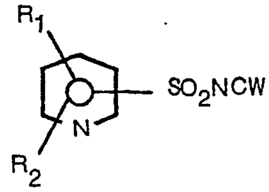 und 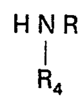

wobei R, $R_1$, $R_2$, $R_4$ und W die in Anspruch 1 angegebenen Bedeutungen haben, wodurch man eine entsprechende Verbindung nach Anspruch 1 erhält, in der $R_3$ H bedeutet.

19. Verfahren zur Herstellung von Verbindungen nach Anspruch 1, dadurch gekennzeichnet, dass man Verbindungen der folgenden Formeln miteinander umsetzt

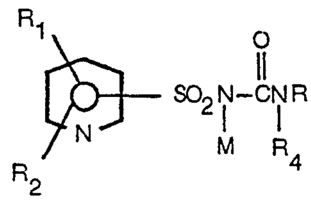 und $(R_3)_nX$

wobei R, $R_1$, $R_2$ und $R_4$ die in Anspruch 1 angegebene Bedeutung haben, $R_3$ Methyl bedeutet, M ein Alkalimetallkation bedeutet, X ein anfängliches Anion bedeutet und n eine ganze Zahl entsprechend der Wertigkeit von X ist, wodurch man eine entsprechende Verbindung nach Anspruch 1 erhält, in der $R_3$ Methyl bedeutet und W O ist.

20. Verfahren zur Herstelung von Verbindungen nach Anspruch 1, dadurch gekennzeichnet, dass man Verbindungen der folgenden Formeln miteinander umsetzt

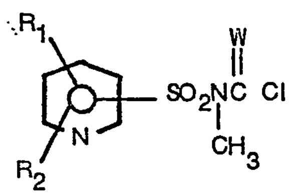 und 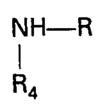

wobei R, $R_1$, $R_2$, $R_4$ und W die in Anspruch 1 angegebene Bedeutung haben, wodurch man eine Verbindung nach Anspruch 1 erhält, in der $R_3$ $CH_3$ bedeutet.

21. Verfahren zur Herstellung von Verbindungen nach Anspruch 1, dadurch gekennzeichnet, dass

man eine Verbindung der folgenden allgemeinen Formel oxidiert, beispielsweise mit Quecksilber(II)-oxid/Bortrifluoridätherat oder Quecksilber(II)-chlorid/Calciumcarbonat,

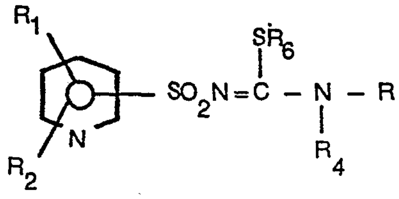

wobei R, $R_1$, $R_2$ und $R_4$ die in Anspruch 1 angegebene Bedeutung haben und $R_6$ $C_1$—$C_5$-Alkyl bedeutet, wodurch man eine entsprechende Verbindung nach Anspruch 1 erhält, in der $R_3$ H bedeutet und W O bedeutet.

22. Verfahren zur Herstellung von Verbindungen nach Anspruch 1, dadurch gekennzeichnet, dass man Verbindungen der folgenden allgemeinen Formeln miteinander umsetzt.

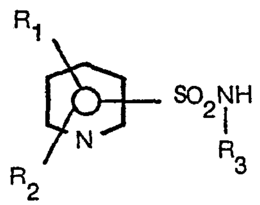 und S C N—R

wobei R, $R_1$, $R_2$ und $R_3$ die im Anspruch 1 angegebene Bedeutung haben, wodurch man eine Verbindung nach Anspruch 1 erhält, in der $R_4$ H bedeutet und W S bedeutet.

23. Verfahren zur Herstellung von Verbindungen nach Anspruch 1, wobei W O bedeutet, dadurch gekennzeichnet, dass man eine Verbindung nach Anspruch 1, in der W S bedeutet, mit Quecksilber(II)-oxid umsetzt.